# EUROPEAN PATENT APPLICATION

(11) **EP 1 837 357 A1**
(43) Date of publication of application: **26.09.2007**
(21) Application number: 05809629.8
(22) Date of filing: 21.11.2005
(51) Int. Cl.: C08G 59/24, B41J 2/01, B41M 5/00, C08G 65/18, C09D 11/00

(54) **ACTINIC RAY CURABLE COMPOSITION, ACTINIC RAY CURABLE INK, METHOD OF IMAGE FORMING THEREWITH, INKJET RECORDING APPARATUS AND EPOXY COMPOUND**

(30) Priority: 22.12.2004 JP 2004370907; 13.06.2005 JP 2005172127
(71) Applicant: Konica Minolta Medical & Graphic, Inc., Tokyo 163-0512 (JP)
(72) Inventor: OKUBO, Kimihiko, Konica Minolta Tech. Center, Inc., Hachioji-shi, Tokyo 1928505 (JP); MIURA, Norio, Konica Minolta Technology Center,Inc, Hachioji-shi, Tokyo 1928505 (JP); KURATA, Takeshi, Konica Minolta Tech. Center, Inc., Hachioji-shi, Tokyo 1928505 (JP)
(74) Representative: McCluskie, Gail Wilson
(86) International application number: PCT/JP2005/021351
(87) International publication number: WO 2006/067927

(57) **Abstract**

This invention provides an actinic ray curable composition, which exhibits excellent storage stability, low viscosity, high sensitivity and can form cured film having high hardness and superior weather-proofing under various environments particularly even under a high humidity atmosphere, and actinic ray curable ink utilizing the same, an image forming method utilizing said actinic ray curable ink as ink-jet ink, an ink-jet recording apparatus and a new epoxy compound. An actinic ray curable composition of this invention is **characterized by** containing at least one type of an epoxy compound represented by following Formula (X) and at least one type of an oxetane compound as a photo polymerizable compound and having a viscosity at 25 °C of 1 - 500 mPa·s.

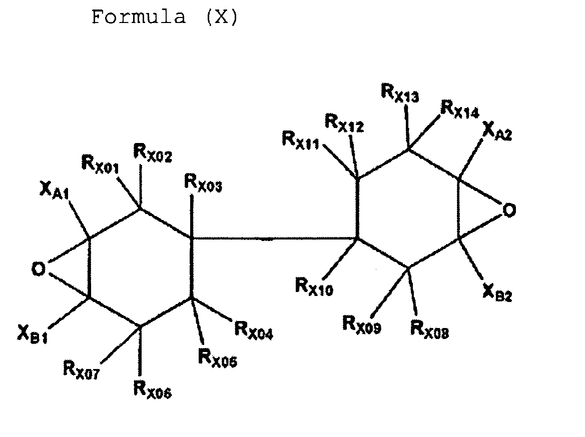

## Description

### FIELD OF THE INVENTION

The present invention relates to an actinic ray curable composition, actinic ray curable ink, an image forming method utilizing the same, an ink-jet recording apparatus, and an epoxy compound.

### BACKGROUND

Heretofore, a curable composition, which is cured by actinic rays such as ultraviolet rays and electron rays or by heat, has been utilized in practice for various applications such as paints, adhesives and printing inks on such as plastics, paper, wood and inorganic materials, a printed circuit board and electrical insulation related materials. In recent years, as for printing inks, paints and adhesives among them, further improvement of weather-proofing and adhesive capabilities has been sought. Yet further, as ink-jet inks utilizing them, ultraviolet curable ink-jet ink, which cures by ultraviolet rays, is known. An ink-jet method utilizing this ultraviolet curable ink has attracted attention in recent years with respect to relatively low odor, rapid drying and the capability of recording on non-process recording medium, and ultraviolet curable ink-jet ink has been offered (for example, refer to Patent Documents 1 and 2). In this field, it is essential that formed film exhibits higher strength and flexibility in addition to a low viscosity of the ink. It has been proposed to provide ink-jet ink with plasticity by addition of a plastisizer (for example, refer to Patent Document 3). However, it is for melt-type ink and there is no description with respect to ultraviolet curable ink-jet ink utilizing solvent-type ink.

Further, when utilizing this ink, there is a problem that curing sensitivity tends to vary depending on the type of a recording material and application environment.

Since ink utilizing a radical polymerizing compound suffers from an oxygen inhibiting effect, curing inhibition tends to be caused in the case of a low ink droplet volume. Further, ink utilizing a cationic polymerizing compound does not suffer from the above oxygen inhibiting effect, however, there is another problem of being easily affected by water content (moisture) of at the molecular level (for example, refer to Patent Documents 4 - 7).
[Patent Document 1] Unexamined Japanese Patent Application Publication No. (hereinafter, referred to as JP-A) 6-200204
[Patent Document 2] Japanese Translation of PCT International Application Publication No. 2000-504778
[Patent Document 3] JP-A 8-3493
[Patent Document 4] JP-A 2001-220526
[Patent Document 5] JP-A 2002-188025
[Patent Document 6] JP-A 2002-317139
[Patent Document 7] JP-A 2003-55449

### SUMMARY OF THE INVENTION

### PROBLEMS TO BE SOLVED

This invention is presented in view of the above problems, and an object is to provide an actinic ray curable composition, which exhibits excellent storage stability, low viscosity, high sensitivity and formation of cured film exhibiting excellent hardness and superior weather-resistance under various environments, either under high humidity ambience, and an actinic ray curable ink utilizing the same, an image forming method utilizing said actinic ray curable ink as an ink-jet ink, an ink-jet recording apparatus and a new epoxy compound.

### MEANS TO SOLVE THE PROBLEMS

The above-described object of this invention is achieved by the following constitutions.

Item 1. An actinic ray curable composition characterized by containing at least one type of an epoxy compound represented following Formula (X) and at least one type of an oxetane compound, as a photo polymerizable compound, and having a viscosity of 1 - 500 mPa·s at 25 °C.

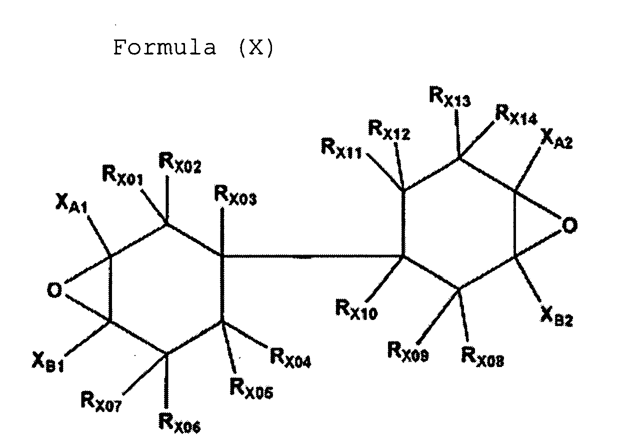

In the formula, X_{A1}, X_{A2}, X_{B1} and X_{B2} are each a hydrogen atom or an alkyl group, and R_{X01}- R_{X14} are each a hydrogen atom or a substituent.

Item 2. The actinic ray curable composition described in aforesaid Item 1, wherein, in the aforesaid epoxy compound represented by Formula (X), at least one of X_{A1} or X_{A2} is an alkyl group and at least one of X_{B1} or X_{B2} is an alkyl group.

Item 3. The actinic ray curable composition described in above Item 1 or 2, wherein the aforesaid oxetane compound is an oxetane compound having no substituent at the 2-position of an oxetane ring.

Item 4. The actinic ray curable composition described in any one of above Items 1 - 3, wherein at least one type of an epoxy compound represented by following Formula (A) is further incorporated as a photo polymerizable compound.

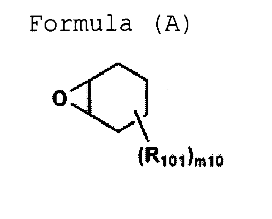

In the formula, R₁₀₁ is a substituent containing no functional group which is cationic polymerizing or radical polymerizing reactive, and m10 is 1, 2, 3 or 4.

Item 5. The actinic ray curable composition described in above Item 4, wherein the aforesaid epoxy compound represented by Formula (A) is a compound represented by following Formula (A-I).

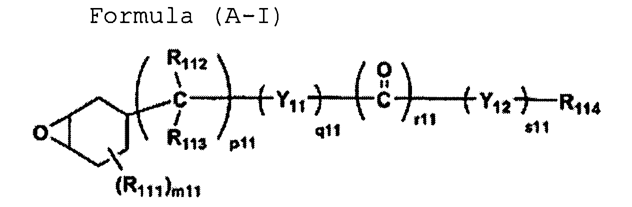

In the formula, R₁₁₁ is a substituent, and m11 is 0, 1, 2 or 3. R₁₁₂, R₁₁₃ and R₁₁₄ are each independently a hydrogen atom or a substituted or unsubstituted alkyl group. Y₁₁ and Y₁₂ are each independently O or S; p11 is 0, 1 or 2; q11 is 0 or 1; r11 is 0 or 1; and s11 is 0 or 1.

Item 6. The actinic ray curable composition described in above Item 4, wherein the aforesaid epoxy compound represented by Formula (A) is a compound represented by following Formula (A-II).

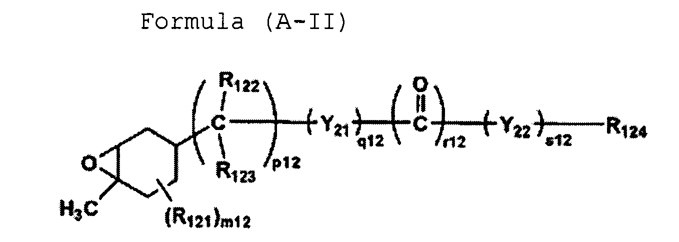

In the formula, R₁₂₁ is a substituent, and m12 is 0, 1 or 2. R₁₂₂, R₁₂₃ and R₁₂₄ are each independently a hydrogen atom or a substituted or unsubstituted alkyl group. Y₂₁ and Y₂₂ are each independently O or S; p12 is 0, 1 or 2; q12 is 0 or 1; r12 is 0 or 1; and s12 is 0 or 1.

Item 7. The actinic ray curable composition described in above Item 4, wherein the aforesaid epoxy compound represented by Formula (A) is a compound represented by following Formula (A-III), (A-IV), or (A-V).

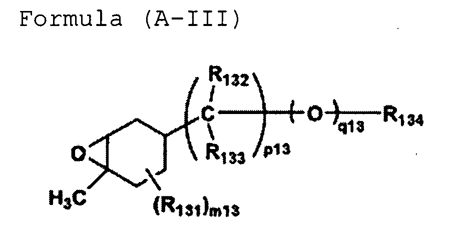

In the formula, R₁₃₁ is a substituent, and m13 is 0, 1 or 2, R₁₃₂, R₁₃₃ and R₁₃₄ are each independently a hydrogen atom or a substituted or unsubstituted alkyl group, p13 is 0, 1 or 2, and q13 is 0 or 1.

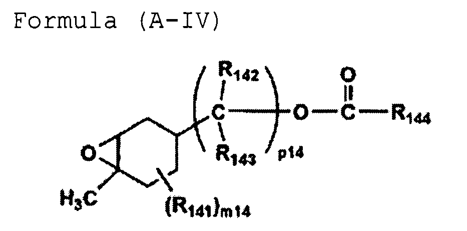

In the formula, R₁₄₁ is a substituent, and m14 is 0, 1 or 2. R₁₄₂, R₁₄₃ and R₁₄₉ are each independently a hydrogen atom or a substituted or unsubstituted alkyl group. p14 is 0, 1 or 2.

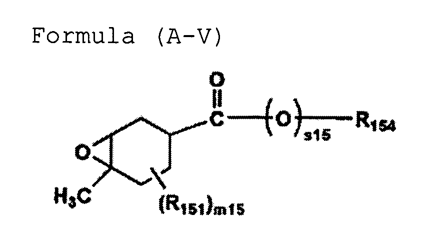

In the formula, R₁₅₁ is a substituent, and m15 is 0, 1 or 2. R₁₅₄ is a hydrogen atom or a substituted or unsubstituted alkyl group, and s15 is 0 or 1.

Item 8. The actinic ray curable composition described in above Item 4, wherein the aforesaid epoxy compound represented by Formula (A) is a compound represented by following Formula (A-VI).

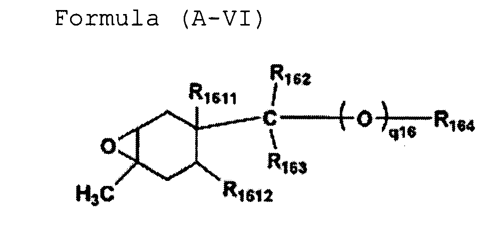

In the formula, R₁₆₁₁ and R₁₆₁₂ each independently are a hydrogen atom or an alkyl group having a carbon number of 1 - 6. R₁₆₂, R₁₅₃ and R₁₆₄ are each independently a hydrogen atom or a substituted or unsubstituted alkyl group, and q16 is 0 or 1.

Item 9. The actinic ray curable composition described in any one of above Items 3 - 8, wherein the aforesaid oxetane compound, having no substituent at the 2-position of an oxetane ring, is a poly-functional oxetane compound having at least two oxetane rings.

Item 10. The actinic ray curable composition described in any one of above Items 1 - 9, wherein the aforesaid actinic ray curable composition contains a compound which generates acid by actinic ray irradiation.

Item 11. The actinic ray curable composition described in above Item 10, wherein the aforesaid compound, which generates acid by actinic ray irradiation, is an onium compound.

Item 12. The actinic ray curable composition described in above Item 11, wherein the aforesaid onium compound is a sulfonium compound.

Item 13. The actinic ray curable composition described in above Item 12 is characterized by that the aforesaid onium compound being a compound represented by following Formula (I-1), (I-2), or (I-3).

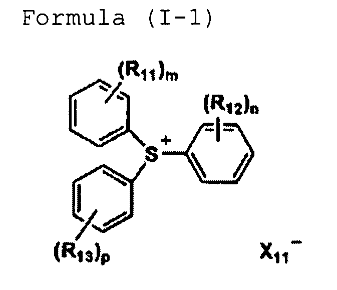

In the formula, R₁₁, R₁₂ and R₁₃ are a substituent; and m, n and p are an integer of 0 - 2, while X₁₁⁻ is a counter ion.

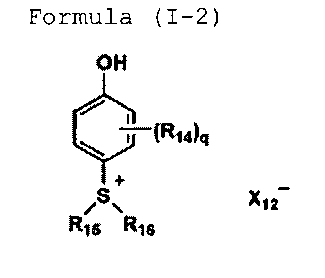

In the formula, R₁₄ is a substituent, and q is an integer of 0 - 2. R₁₅ and R₁₆ are a substituted or unsubstituted alkyl group, a substituted or unsubstituted alkenyl group, a substituted or unsubstituted alkynyl group, or a substituted or unsubstituted aryl group, while X₁₂⁻ is a counter ion.

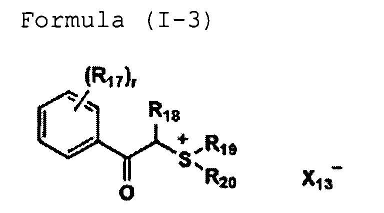

In the formula, R₁₇ is a substituent, and r is an integer of 0 - 3. R₁₉ and R₂₀ are a substituted or unsubstituted alkyl group, a substituted or unsubstituted alkenyl group, a substituted or unsubstituted alkynyl group, or a substituted or unsubstituted aryl group, while X₁₃⁻ is a counter ion.

Item 14. An actinic ray curable ink characterized by containing an actinic ray curable composition described in any one of Items 1 - 13.

Item 15. The actinic ray curable ink described in above Item 14, wherein viscosity at 25 °C is 7 - 40 mPa·s.

Item 16. The actinic ray curable ink described in above Item 14 or 15 wherein the aforesaid actinic ray curable ink contains a pigment.

Item 17. An image forming method in which the actinic ray curable ink described in any one of Items 14 - 16 is image-wise ejected onto a recording material from an ink-jet recording head and printing of an image is performed on said recording material, wherein the actinic ray curable ink is cured by irradiation of actinic rays in 0.001 - 1.0 second after ink deposition.

Item 18. An image forming method in which the actinic ray curable ink described in any one of Items 14 - 16 is image-wise ejected onto a recording material from an ink-jet recording head and printing is performed on said recording material, wherein the minimum ink liquid quantity ejected from each nozzle of said ink-jet recording head is 2 - 15 pl.

Item 19. An ink-jet recording apparatus which is utilized for the image forming method described in above Item 17 or 18, wherein ink ejection is performed after actinic ray curable ink and a recording head are heated at 35 - 100 °C.

Item 20. An ink-jet recording apparatus which is utilized for the image forming method described in aforesaid Item 17 or 18, wherein ink ejection is performed onto a recording material being heated at 35 - 60 °C.

Item 21. An epoxy compound characterized by being represented by following Formula (X-a).

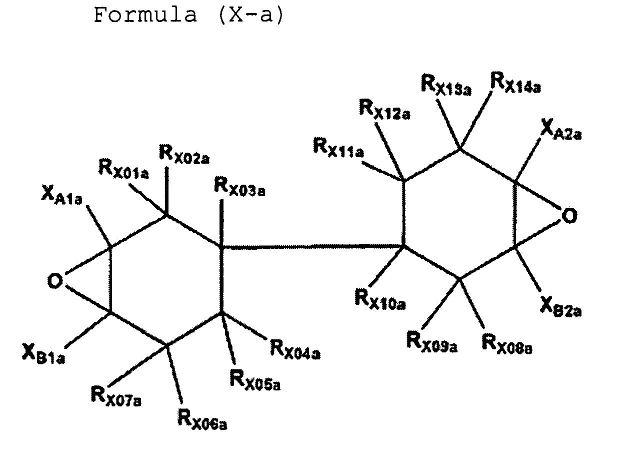

In the formula, X_{A1a}, X_{A2a}, X_{B1a}, and X_{B2a} are each a hydrogen atom or an alkyl group, R_{X01a} - R_{X14a} are each a hydrogen atom or a substituent, at least one of X_{A1a} or X_{B1a} is an alkyl group, and at least one of X_{A2a} or X_{B2a} is an alkyl group.

Item 22. An epoxy compound characterized by being represented by following Formula (X-b).

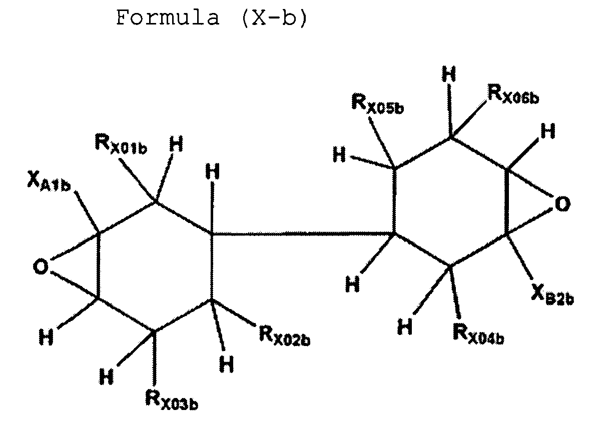

In the formula, X_{A1b} and X_{B2b} are each an alkyl group, and R_{X01b} - R_{X06b} are each a hydrogen atom or an alkyl group.

### EFFECTS OF THE INVENTION

This invention can provide an actinic ray curable composition, exhibiting excellent storage stability, low viscosity, high sensitivity and can form cured film of excellent hardness and superior weather-resistance under varying ambience either under high humidity, and an actinic ray curable ink utilizing the same, an image forming method utilizing the actinic ray curable ink as ink-jet ink, an ink-jet recording apparatus, and a new epoxy compound.

### BRIEF DESCRIPTION OF THE DRAWINGS

Fig. 1 shows a constitution of the primary portion of a recording apparatus utilized in this invention.
Fig. 2 shows a constitution of the primary portion of another recording apparatus utilized in this invention.

### DESCRIPTION OF THE SYMBOLS

1: Recording apparatus
2: Head carriage
3: Recording head
4: Irradiation means
5: Platen portion
6: Guide member
7: Bellows structure
P: Recording material

### DETAILED DESCRIPTION OF THE INVENTION

The inventors of this invention, as a result of extensive study in view of the above problems, have found that an actinic ray curable composition which is superior in storage stability and can provide strong cured film of superior weather resistance without being affected by humidity even under a light source of low illuminance, when an actinic ray curable composition containing both of a bisalicyclic epoxy compound, in which two cyclohexane skeletons are directly bonded, and an oxetane compound is utilized, and that a high quality image can be formed when this actinic ray curable composition is utilized in an actinic ray curable ink with which an image is formed utilizing an ink-jet recording apparatus.

It was also found that marked improvement of storage stability of a curable composition in addition to further improvement of curing capability can be achieved when a bisalicyclic epoxy compound, in which two cyclohexane skeletons are directly bonded, having a structure in which an alkyl group is further substituted to either one of two carbon atoms constituting an oxirane ring of a bisalicyclic epoxy compound, according to this invention, is utilized together with an oxetane compound, and prepared can be a curable composition which exhibits a high activity, no deterioration of the capabilities even after long-term storage and excellent storage stability.

By employing a poly-functional, being not less than bi-functional, compound as an oxetane compound of this invention, ink of higher sensitivity and cured film of greater hardness can be realized.

According to this invention, in an actinic ray curable composition of this invention, by employing a bisalicyclic epoxy compound, in which two cyclohexane skeletons are directly bonded, and a specific mono-functional epoxy compound as a cationic polymerizing compound utilized together with an oxetane compound, it has become possible to improve reactivity, and to enhance strength of the cured film, and attain other various physical properties of the cured film.

In the following, this invention will be further detailed.

In above Formula (X), X_{A1}, X_{A2}, X_{B1} and X_{B2} are each a hydrogen atom or an alkyl group. Examples of an alkyl group include alkyl groups of a carbon number of 1 - 20, such as a methyl group, an ethyl group, a propyl group, an isopropyl group, an n-butyl group, an isobutyl group, a t-butyl group, a pentyl group, a hexyl group, a cyclopentyl group, a cyclohexyl group, a heptyl group, an octyl group, a 2-ethylhexyl group, a decyl group and a dodecyl group. These may be further provided with a substituent, and examples of a substituent of an alkyl group having a substituent include a halogen atom, (such as a chlorine atom, a bromine atom and a fluorine atom); an alkoxy group having a carbon number of 1 - 20 (such as a methoxy group, an ethoxy group, an n-propoxy group, an iso-propoxy group, an n-butoxy group and a tert-butoxy group); an acyl group (such as an acetyl group, a propionyl group and a trifluoroacetyl group); an acyloxy group having a carbon number of 1 - 20 (such as an acetyl group, a propionyl group and a trifluoroactyl group); an alkoxycarbonyl group having a carbon number of 1 - 20 (such as a methoxycarbonyl, an ethoxycarbonyl and a tert-butoxycarbonyl group); and an alkylthiocarbonyl group having a carbon number of 2 - 20 (such as a methylthiocarbonyl, an ethylthiocarbonyl and a tert-butylthiocarbonyl group); an aryloxycarbonyl group; an alkylsulfonyl group; an arylsulfonyl group; a cyano group; and a nitro group.

X_{A1} X_{A2}, X_{B1} and X_{B2} are each independently preferably a hydrogen atom or an alkyl group having a carbon number of 1 - 6 (such as a methyl group, an ethyl group, a propyl group, an isopropyl group, a n-butyl group, an isobutyl group, a t-butyl group, a pentyl group and a hexyl group), but are each independently more preferably a hydrogen atom, a methyl group, an ethyl group, a propyl group, an isopropyl group, a n-butyl group, an isobutyl group, or a t-butyl group.

Among X_{A1}, X_{A2}, X_{B1} and X_{B2} which bond to the two carbon atoms constituting an oxirane ring, the case that either of X_{A1} and X_{B1} is an alkyl group and either of X_{A2} and X_{B2} is an alkyl group is preferable with respect to enhanced curing capability as well as improved storage stability. In this case, as an alkyl group of either X_{A1} or X_{B1}, and an alkyl group as either X_{A2} or X_{B2}, preferable are a methyl group, an ethyl group, a propyl group, an isopropyl group, an n-butyl group, an isobutyl group and a t-butyl group; and either X_{A1} or X_{B1}, and either X_{A2} or X_{B2} is an alkyl group are most preferably a methyl group.

R_{X01} - R_{X14} are each a hydrogen atom or a substituent. Examples of a substituent include a halogen atom (such as chlorine, bromine and fluorine), an alkoxy group having a carbon number of 1 - 20 (such as a methoxy group, an ethoxy group, an n-propoxy group, an iso-propoxy group, an n-butoxy group and a tert-butoxy group), an acyl group (such as an acetyl group, a propionyl group and a trifluoroacetyl group), an acyloxy group having a carbon number of 1 - 20 (such as an acetoxy group, a propionyloxy group and a trifluoroacetoxy group), an alkoxycarbonyl group having a carbon number of 1 - 20 (such as a methoxycarbonyl, an ethoxycarbonyl and a tert-butoxycarbonyl group), and an alkylthiocarbonyl group having a carbon number of 1 - 20 (such as a methylthiocarbonyl, an ethylthiocarbonyl and a tert-butylthiocarbonyl group), an aryloxycarbonyl group, an alkylsulfonyl group, an arylsulfonyl group, a cyano group and a nitro group; and an alkoxy group and an alkoxycarbonyl group are preferable as a substituent. R_{X01} - R_{X14} may be further provided with a substituent, and examples of said substituent include groups identical to the above substituents. R_{X01} - R_{X14} may bond at any position to form a ring.

In above formula (X-a), X_{A1a}, X_{A2a}, X_{B1a} and X_{B2a} are each groups identical to X_{A1}, X_{A2}, X_{B1} and X_{B2} described above; R_{X01a} - R_{X14a} are each groups identical to R_{X01} - R_{X14} described above; and either one of X_{A1a} and X_{B1a} is an alkyl group and either X_{A2a} or X_{B2a} is an alkyl group. It is preferable that X_{A1a}, of X_{A1a} or X_{B1a} is an alkyl group, and X_{B2a} of X_{A2a} or X_{B2a} is an alkyl group.

An epoxy compound represented by aforesaid Formula (X-a) is more preferably a compound represented by above Formula (X-b).

In the formula, X_{A1b} and X_{B1b} are each an alkyl group, examples of which group include an alkyl group having a carbon number of 1 - 20 (such as a methyl group, an ethyl group, a propyl group, an isopropyl group, an n-butyl group, an isobutyl group, a t-butyl group, a pentyl group, a hexyl group, a cyclopentyl group, a cyclohexyl group, a heptyl group, an octyl group, a 2-ethylhexyl group, a decyl group and a dodecyl group), of which preferable is the methyl group, ethyl group, propyl group, isopropyl group, n-butyl group, isobutyl group or t-butyl group, and of which most preferable is the methyl group. R_{X01b}- R_{X14b} are each a hydrogen atom or an alkyl group, examples of which group include an alkyl group having a carbon number of 1 - 20 (such as a methyl group, an ethyl group, a propyl group, an isopropyl group, a n-butyl group, an isobutyl group, a t-butyl group, a pentyl group, a hexyl group, a cyclopentyl group, a cyclohexyl group, a heptyl group, an octyl group, a 2-ethylhexyl group, a decyl group and a dodecyl group). These may be further provided with a substituent, examples of which group having a substituent include an alkyl group having a substituent identical to X_{A1}, X_{A2}, X_{B1} and X_{B2} described above. An alkyl group represented by R_{X01b} - R_{X14b} is preferably a methyl group, an ethyl group, an isopropyl group, an n-butyl group, an isobutyl group or a t-butyl group and most preferably a methyl group.

In the following, specific examples of a compound represented by Formula (X), (X-a), or (X-b) according to this invention will be shown, however, this invention is not limited thereto.

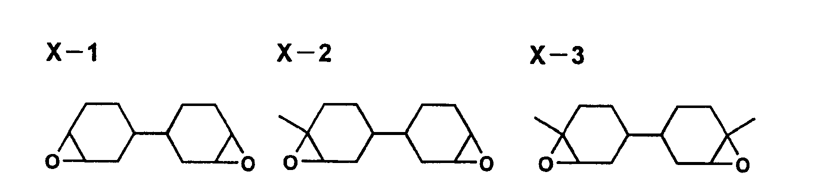

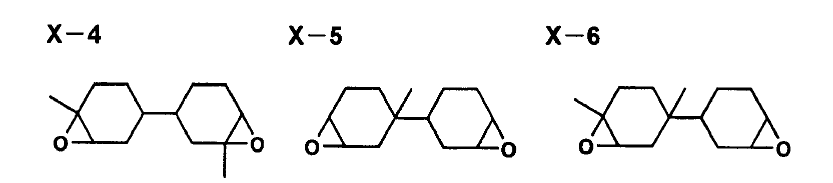

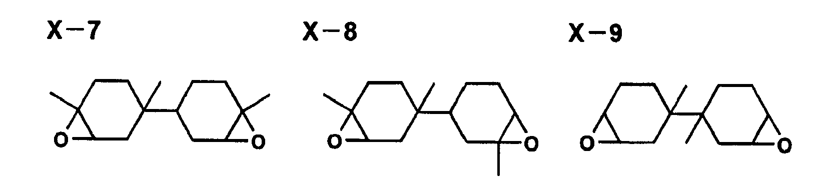

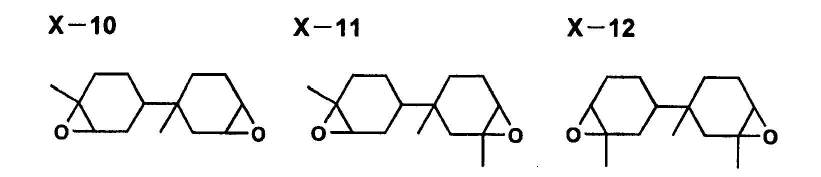

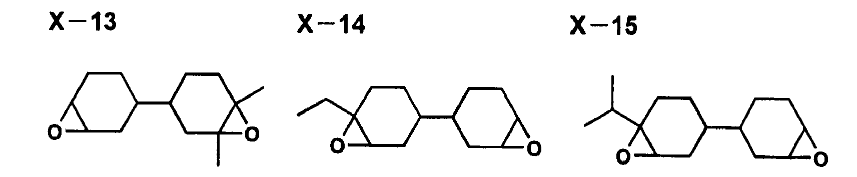

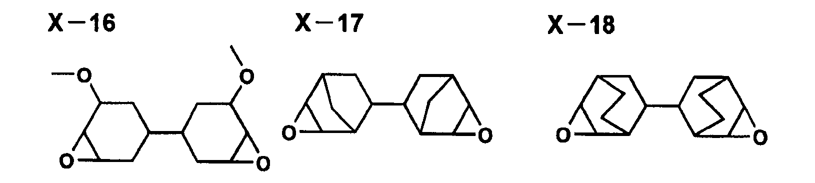

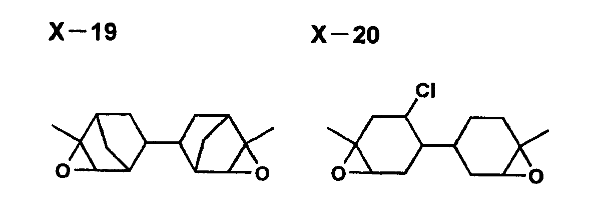

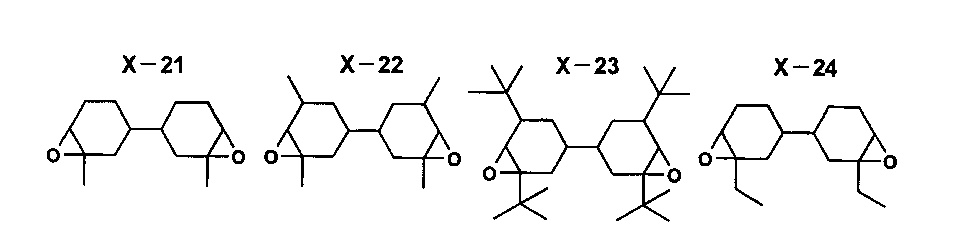

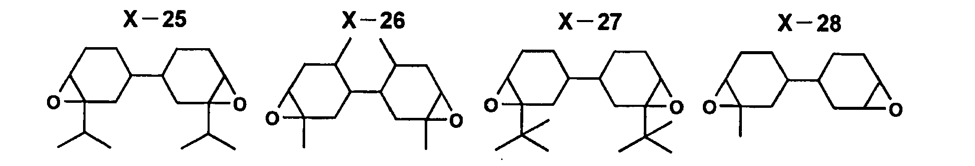

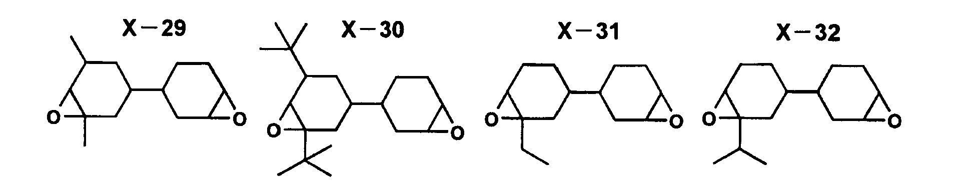

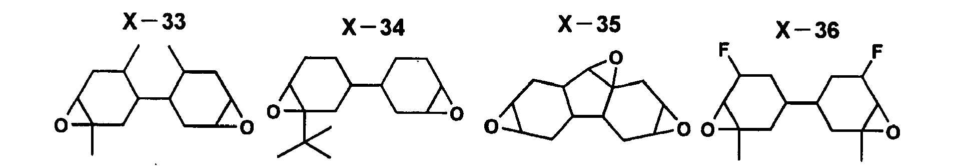

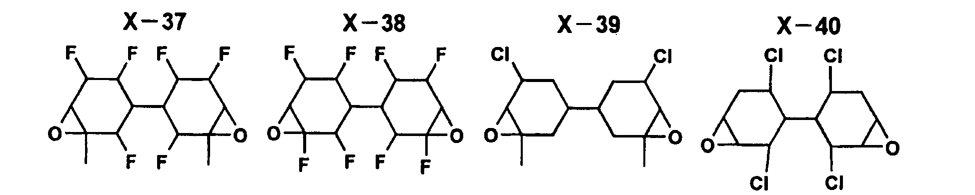

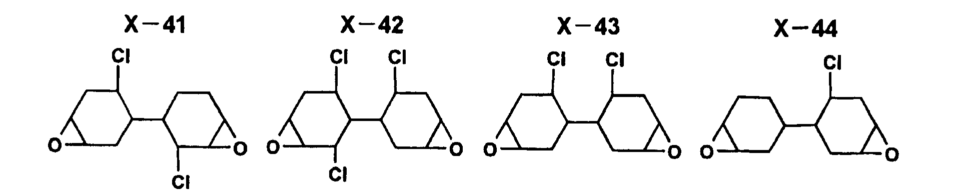

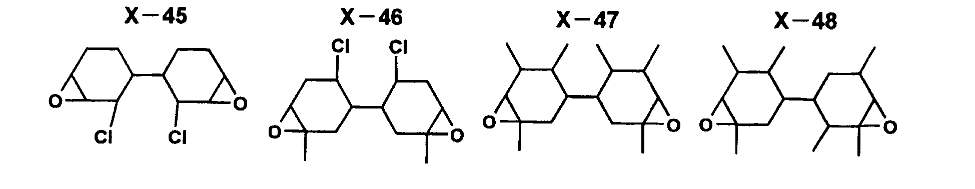

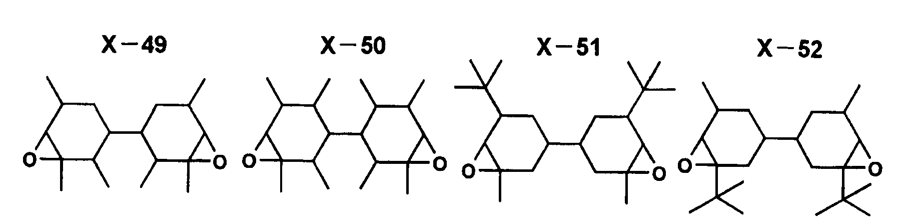

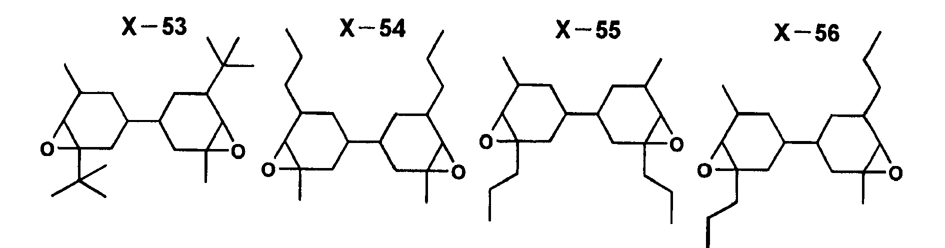

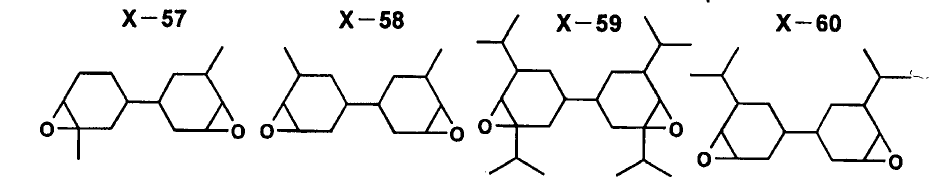

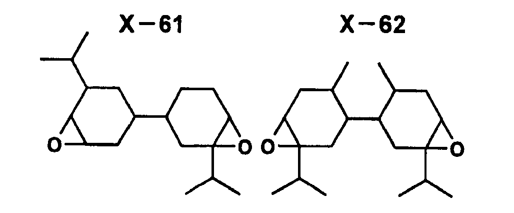

A compound represented by Formula (X), (X-a) or (X-b) of this invention, a manufacturing method of which is not limited, can be prepared by epoxydation of an appropriate olefin compound as a starting material via an appropriate oxidation reaction. A synthesis method of an olefin compound as a starting material can be referred to methods described in such as J. Chem. Soc., 1726 (1951), Chem. Lett., 9, 845 (1995), J. Chem. Soc., 940 (1956), J. Chem. Soc., 935 (1963), Japanese Patent Nos. 2520759 and 3189392, Monatsh. Chem., 89, 135 (1958), USP No. 2,118,954, German Patent No. 2,530,122, J. Am. Chem. Soc., 38, 2518 (1916), J. Am. Chem. Soc., 40, 842 (1918), J. Am. Chem. Soc., 74, 4872 (1952) and J. Org. Chem., 35, 1646 (1970). An oxidation reaction can be referred to documents described in a manufacturing method of an epoxy compound represented by Formula (A) or (B) of this invention, which will be described later.

Synthesis examples of a compound represented by Formula (X), (X-a) or (X-b) will be described, however, this invention is not limited thereto.

### Synthesis Example 1

### Synthesis of Exemplary Compound X-21

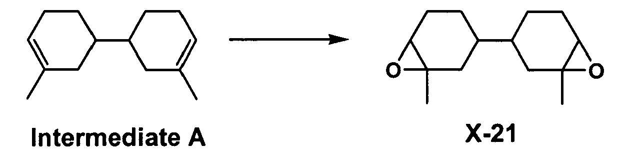

Intermediate A of 9.5 g was dissolved in 95 ml of methylene chloride. Methylene chloride of 100 ml, in which 29.2 g of m-chloroperbenzoic acid had been dissolved, was titrated into the above solution of intermediate A over 2 hours. After 2 hours of a reaction under room temperature, disappearance of a starting material was confirmed by gas chromatography. After finishing the reaction, a suitable amount of sodium sulfite aqueous solution was added to inactivate excess m-chloroperbenzoic acid. An organic layer was separated to be washed by a sodium bicarbonate aqueous solution for several times, and the solvent in the organic layer was evaporated under reduced pressure. The residue was purified by reduced pressure evaporation to prepare X-21. The prepared amount was 7.5 g (yield of 68%). The aimed product was confirmed by NMR and mass spectrum.

(¹H-NMR) (CDCl₃) δ (ppm): 1.2 - 2.3 (m, 20H, hydrogen substituted to carbon atom at following a), 2.9 - 3.1 (m, 2H, hydrogen substituted to carbon atom at following b).

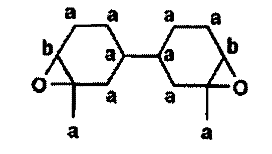

### Synthesis Example 2

### Synthesis of Exemplary Compound X-22

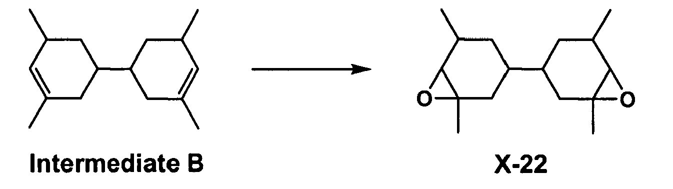

Intermediate B of 11.0 g was dissolved in 80 ml of methylene chloride. Methylene chloride of 100 ml, in which 29.2 g of m-chloroperbenzoic acid had been dissolved, was titrated into the above solution of intermediate B over 3 hours. After 3 hours of a reaction under room temperature, disappearance of a starting material was confirmed by gas chromatography. After finishing the reaction, a suitable amount of sodium sulfite aqueous solution was added to inactivate excess m-chloroperbenzoic acid. An organic layer was separated to be washed by a sodium bicarbonate aqueous solution for several times, and the solvent in the organic layer was evaporated under reduced pressure. The residue was purified by reduced pressure evaporation to prepare X-22. The prepared amount was 10.1 g (yield of 80%). The aimed product was confirmed by NMR and mass spectrum.

(¹H-NMR) (CDCl₃) δ (ppm): 1.0 - 2.3 (m, 24H, hydrogen substituted to carbon atom at following a), 2.9 - 3.1 (m, 2H, hydrogen substituted to carbon atom at following b).

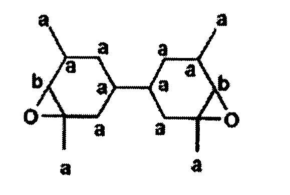

### Synthesis Example 3

### Synthesis of Exemplary Compound X-24

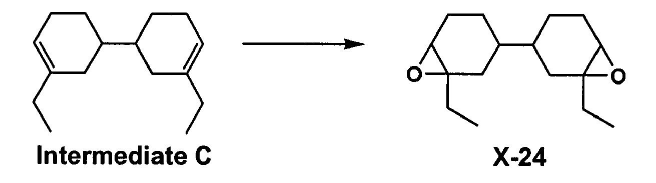

Intermediate C of 11.1 g was dissolved in 110 ml of methylene chloride. Methylene chloride of 100 ml, in which 29.2 g of m-chloroperbenzoic acid had been dissolved, was titrated into the above solution of intermediate C over 2 hours. After 2 hours of a reaction under room temperature, disappearance of a starting material was confirmed by gas chromatography. After finishing the reaction, a suitable amount of sodium sulfite aqueous solution was added to inactivate excess m-chloroperbenzoic acid. An organic layer was separated to be washed by a sodium bicarbonate aqueous solution for several times, and the solvent in the organic layer was evaporated under reduced pressure. The residue was purified by reduced pressure evaporation to prepare X-24. The prepared amount was 10.4 g (yield of 82%). The aimed product was confirmed by NMR and mass spectrum.

(¹H-NMR) (CDCl₃) δ (ppm): 0.9 - 2.4 (m, 24H, hydrogen substituted to carbon atom at following a), 2.9 - 3.1 (m, 2H, hydrogen substituted to carbon atom at following b).

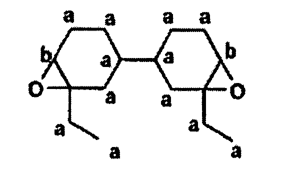

### Synthesis Example 4

### Synthesis of Exemplary Compound X-26

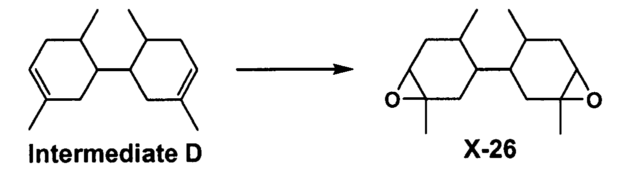

Intermediate D of 11.0 g was dissolved in 110 ml of methylene chloride. Methylene chloride of 100 ml, in which 29.2 g of m-chloroperbenzoic acid had been dissolved, was titrated into the above solution of intermediate D over 2 hours. After 3 hours of a reaction under room temperature, disappearance of a starting material was confirmed by gas chromatography. After finishing the reaction, a suitable amount of sodium sulfite aqueous solution was added to inactivate excess m-chloroperbenzoic acid. An organic layer was separated to be washed by a sodium bicarbonate aqueous solution for several times, and the solvent in the organic layer was evaporated under reduced pressure. The residue was purified by reduced pressure evaporation to prepare X-26. The prepared amount was 9.6 g (yield of 77%). The aimed product was confirmed by NMR and mass spectrum.

(¹H-NMR) (CDCl₃) δ (ppm): 1.0 - 2.3 (m, 24H, hydrogen substituted to carbon atom at following a), 2.9 - 3.1 (m, 2H, hydrogen substituted to carbon atom at following b).

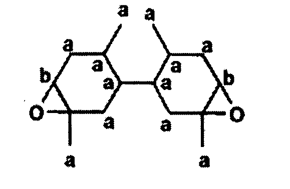

### Synthesis Example 5

### Synthesis of Exemplary Compound X-27

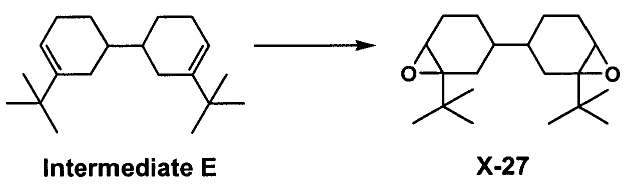

Intermediate E of 20.6 g was dissolved in 200 ml of methylene chloride. Methylene chloride of 150 ml, in which 43.8 g of m-chloroperbenzoic acid had been dissolved, was titrated into the above solution of intermediate E over 4 hours. After 6 hours of a reaction under room temperature, disappearance of a starting material was confirmed by gas chromatography. After finishing the reaction, a suitable amount of sodium sulfite aqueous solution was added to inactivate excess m-chloroperbenzoic acid. An organic layer was separated to be washed by a sodium bicarbonate aqueous solution for several times, and the solvent in the organic layer was evaporated under reduced pressure. The residue was purified by reduced pressure evaporation to prepare X-27. The prepared amount was 11.2 g (yield of 49%). The aimed product was confirmed by NMR and mass spectrum.

(¹H-NMR) (CDCl₃) δ (ppm) : 0.9 - 2.3 (m, 32H, hydrogen substituted to carbon atom at following a), 2.9 - 3.1 (m, 2H, hydrogen substituted to carbon atom at following b).

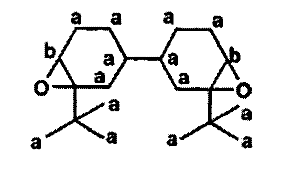

In an actinic ray curable composition of this invention, an oxetane compound, the 2-position of an oxetane ring of which is not substituted, is preferably utilized together with a compound represented by Formula (X).

In the following, an oxetane compound the 2-position of which is not substituted will be explained. An example of an oxetane compound the 2-position of which is not substituted includes a compound represented by following Formula (101).

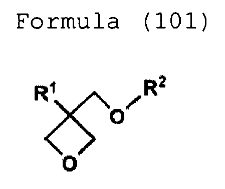

In Formula (101), R¹ is a hydrogen atom, an alkyl group having a carbon number of 1 - 6 such as a methyl group, an ethyl group, a propyl group and a butyl group; a fluoroalkyl group having a carbon number of 1 - 6, an allyl group, an aryl group, a furyl group or a thienyl group. R² is an alkyl group having a carbon number of 1 - 6 such as a methyl group, an ethyl group, a propyl group and a butyl group; an alkenyl group having a carbon number of 2 - 6 such as a 1-propenyl group, a 2-propenyl group, a 2-methyl-1-propenyl group, a 2-methyl-2-propenyl group, a 1-butenyl group, a 2-butenyl group and a 3-butenyl group; a group having an aromatic ring such as a phenyl group, a benzyl group, a fluorobenzyl group, a methoxybenzyl group and a phenoxybenzyl group; an alkylcarbonyl group having a carbon number of 2 - 6 such as an ethylcarbonyl group, a propylcarbonyl group and a butylcarbonyl group; an alkoxycarbonyl group having a carbon number of 2 - 6 such as an ethoxycarbonyl group, a propoxycarbonyl group and a butoxylcarbonyl group; or an N-alkylcarbamoyl group having a carbon number of 2 - 6 such as an ethylcarbamoyl group, a propylcarbamoyl group, a bytylcarbamoyl group and a pentylcarbamoyl group.

As an oxetane compound utilized in this invention, a compound having two oxetane rings is specifically preferable because the prepared composition exhibits superior adhesiveness and excellent working properties due to low viscosity.

Examples of a compound having two oxetane rings include such as compounds represented by following Formula (102).

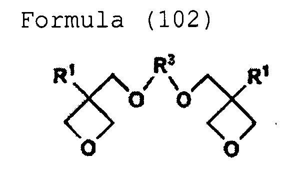

In Formula (102), R¹ is a group identical with those in above-described Formula (101). R³ is, for example, a linear or branched alkylene group such as an ethylene group, a propylene group and a butylenes group; a linear or branched poly(alkyleneoxy) group such as a poly(ethyleneoxy) group and a poly(propyleneoxy) group; a linear or branched unsaturated hydrocarbon group such as a propenylene group, a methylpropenylene group and a butenylene group; a carbonyl group or an alkylene group containing a carbonyl group; an alkylene group containing a carboxyl group and an alkylene group containing a carbamoyl group.

Furhther, R³ includes a polyvalent group selected from groups represented by following Formulas (103), (104) and (105).

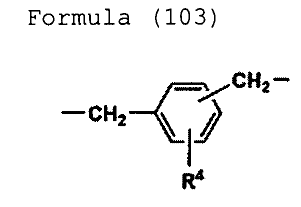

In Formula (103), R⁴ is a hydrogen atom or an alkyl group having a carbon number of 1 - 4 such as a methyl group, an ethyl group, a propyl group and a butyl group; an alkoxy group having a carbon number of 1 - 4 such as a methoxy group, an ethoxy group, a propoxy group and a butoxy group; a halogen atom such as a chlorine atom and a bromine atom; a nitro group, a cyano group, a mercapto group, a lower alkylcarboxyl group, a carboxyl group or a carbamoyl group.

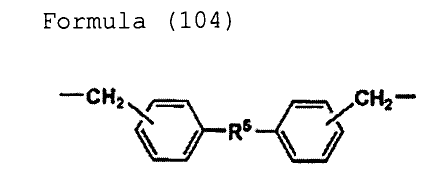

In Formula (104), R⁵ is an oxygen atom, a sulfur atom, a methylene group, NH, SO, SO₂, C(CF₃)₂ or C(CH₃)₂.

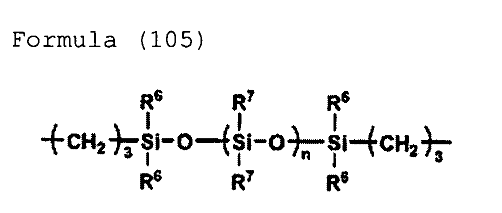

In Formula (105), R⁶ is an alkyl group having a carbon number of 1 - 4 such as a methyl group, an ethyl group, a propyl group and a butyl group; or an aryl group. n is an integer of 0 - 2,000. R⁷ is an alkyl group having a carbon number of 1 - 4 such as a methyl group, an ethyl group, a propyl group and a butyl group; or an aryl group. R⁷ also includes a group selected from groups represented by following Formula (106).

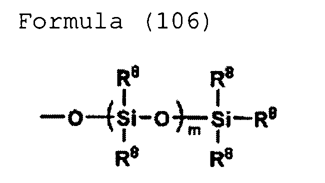

In Formula (106), R⁸ is an alkyl group having a carbon number of 1 - 4 such as a methyl group, an ethyl group, a propyl group and a butyl group; or an aryl group. m is an integer of 0 - 100. Specific examples of a compound having two oxetane rings include the following compounds.

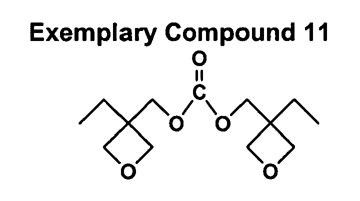

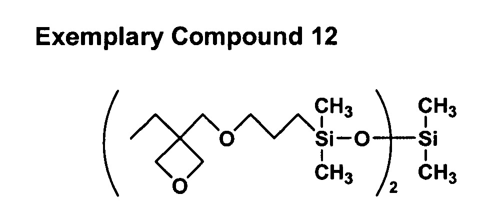

Exemplary compound 11 is a compound of aforesaid Formula (102) in which R¹ is an ethyl group and R³ is a carboxyl group. Further, exemplary compound 12 is a compound of aforesaid Formula (102) in which R¹ is an ethyl group, R³ is aforesaid Formula (105), in which R⁶ and R⁷ are a methyl group, and n is 1.

In a compound having two oxetane rings, preferable examples other than the above-described compounds include a compound represented by following Formula (107). In Formula (107), R¹ is identical with R¹ of aforesaid Formula (101) .

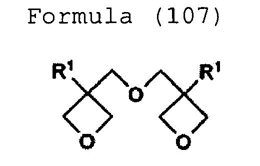

Further, examples of a compound having 3 - 4 oxetane rings include a compound represented by following Formula (108).

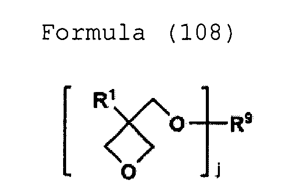

In Formula (108), R¹ is identical with R¹ of aforesaid Formula (101). R⁹ includes a branched alkylene group having a carbon number of 1 - 12 such as groups represented by following A - C, a branched poly(alkyleneoxy) group such as groups represented by following D, and a branched polysiloxy group such as groups represented by following E. j is 3 or 4.

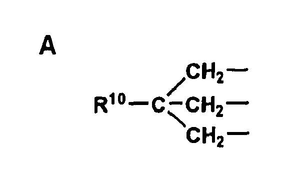

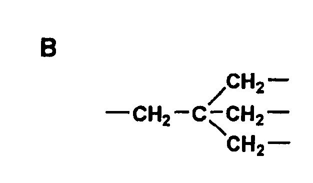

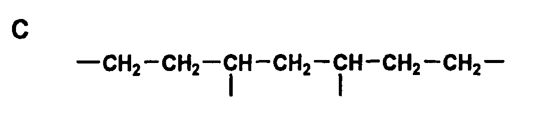

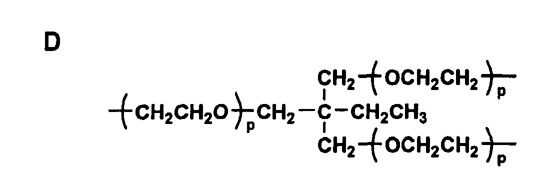

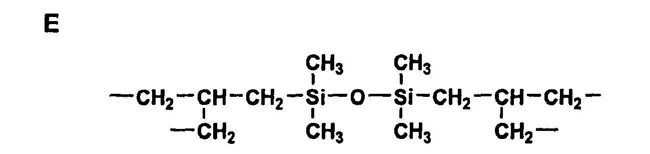

In above-described A, R¹⁰ is a lower alkyl group such as a methyl group, an ethyl group and a propyl group. Further, in above-described D, p is an integer of 1 - 10.

An example of a compound having 3 - 4 oxetane rings includes exemplary compound 13.

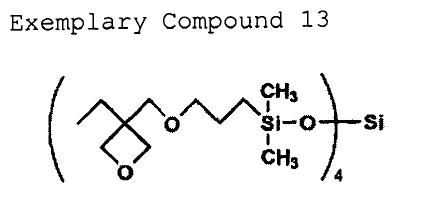

Further, examples of a compound having 1 - 4 oxetane rings other than those explained above include a compound represented by following Formula (109).

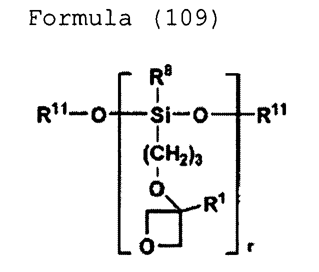

In Formula (109), R⁸ is identical with R⁸ of aforesaid Formula (106). R¹¹ is an alkyl group having a carbon number of 1 - 4 such as a methyl group, an ethyl group, a propyl group and a butyl group; or a trialkylsilyl group, and r is 1 - 4.

Specific examples of an oxetane compound preferably utilized in this invention include the following compounds.

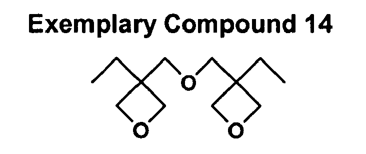

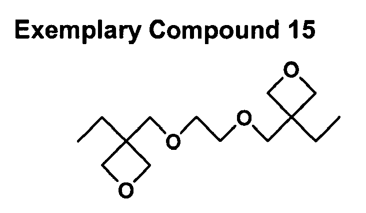

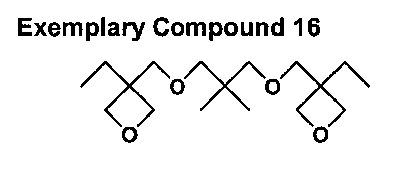

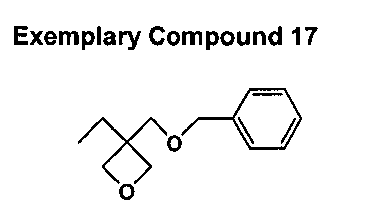

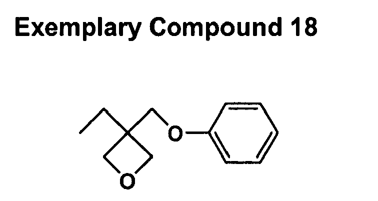

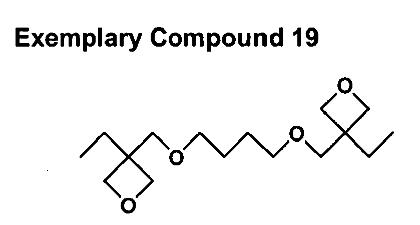

A manufacturing method of each compound having an oxetane ring described above is not specifically limited, and it can be performed according to a conventionally well known method such as a synthesis method of an oxetane ring from diol which is disclosed by D. B. Pattison, J. Am. Chem. Soc., 3455, 79 (1957). Further, in addition to these, a compound having 1 - 4 oxetane rings and a molecular weight of approximately 1,000 - 5,000 is listed. As specific example compounds thereof, the following compounds will be listed.

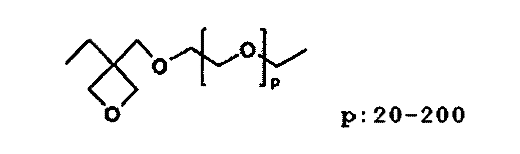

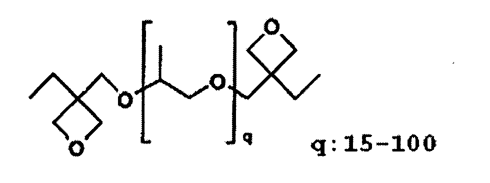

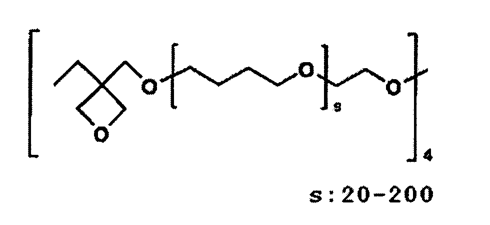

In aforesaid Formula (A), R₁₀₁ is a substituent containing no functional group, which is cationic polymerizing or radical polymerizing. Examples of the substituent include a halogen atom (such as a chlorine atom, a bromine atom and a fluorine atom), an alkyl group having a carbon number of 1 - 20 (such as a methyl group, an ethyl group, a propyl group, an isopropyl group and a butyl group), a cycloalkyl group having a carbon number of 3 - 6 (such as cyclopropyl group, a cyclobutyl group, a cyclopentyl group and a cyclohexyl group), an alkoxy group having a carbon number of 1 - 20 (such as a methoxy group, an ethoxy group, a propoxy group, an iso-propoxy group, a n-butoxy group and a tert-butoxy group), an acyl group having a carbon number of 2 - 20 (such as an acetyl group, a propionyl group and a trifluoroacetyl group), an acyloxy group having a carbon number of 2 - 20 (such as an acetoxy group, a propionyloxy group and a trifluoroacetoxy group), an acylthio group having a carbon number of 2 - 20 (such as an acetylthio group, a propionylthio group and a trifluoroacetylthio group), an alkoxycarbonyl group having a carbon number of 2 - 20 (such as a methoxycarbonyl group, an ethoxycarbonyl group and a tert-butoxycarbonyl group) and an alkylthiocarbonyl group having a carbon number of 2 - 20 (such as a methylthiocarbonyl group, an ethylthiocarbonyl group and a tert-butylthiocarbonyl group).

These groups may be further provided with a substituent. The substituents include a halogen atom (such as a chlorine atom, a bromine atom and a fluorine atom), an alkoxy group having a carbon number of 1 - 20 (such as a methoxy group, an ethoxy group, a n-propoxy group, an iso-propoxy group, a n-butoxy group and a tert-butoxy group), an acyl group having a carbon number of 2 - 20 (such as an acetyl group, a propionyl group and a trifluoroacetyl group), an acyloxy group having a carbon number of 2 - 20 (such as an acetoxy group, a propionyloxy group and a trifluoroacetoxy group), an alkoxycarbonyl group having a carbon number of 2 - 20 (such as a methoxycarbonyl group, an ethoxycarbonyl group and a tert-butoxycarbonyl group) and an alkylthiocarbonyl group having a carbon number of 2 - 20 (such as a methylthiocarbonyl group, an ethylthiocarbonyl group and a tert-butylthiocarbonyl group), an aryloxycarbonyl group, an alkylsulfonyl group, an arylsulfonyl group, a cyano group and a nitro group. Preferable substituents are a halogen atom, an alkoxy group, an acyloxy group and an alkoxycarbonyl group.

More preferable alicyclic epoxide, with respect to forming cured film having a high hardness and improving adhesion of cured film with a substrate, is a compound represented by aforesaid Formula (A-I).

In above-described formula, examples of a substituent represented by R₁₁₁ include a halogen atom (such as a chlorine atom, a bromine atom and a fluorine atom), an alkyl group having a carbon number of 1 - 20 (such as a methyl group, an ethyl group, a propyl group, an isopropyl group and a butyl group), an alkoxy group having a carbon number of 1 - 20 (such as a methoxy group, an ethoxy group, a propoxy group, an iso-propoxy group, a n-butoxy group and a tert-butoxy group), an acyl group (such as an acetyl group, a propionyl group and a trifluoroacetyl group), an acyloxy group having a carbon number of 1 - 20 (such as an acetoxy group, a propionyloxy group and a trifluoroacetoxy group), an alkoxycarbonyl group having a carbon number of 1 - 20 (such as a methoxycarbonyl group, an ethoxycarbonyl group and a tert-butoxycarbonyl group) and an alkylthiocarbonyl group having a carbon number of 2 - 20 (such as a methylthiocarbonyl group, an ethylthiocarbonyl group and a tert-butylthiocarbonyl group), an aryloxycarbonyl group, an alkylsulfonyl group, an arylsulfonyl group, a cyano group and a nitro group. Preferable substituents are an alkyl group, an alkoxy group and an alkoxycarbonyl group.

R₁₁₂, R₁₁₃ and R₁₁₄ are a hydrogen atom or a substituted or non-substituted alkyl group. Examples of an alkyl group include groups identical with examples of an alkyl group of R₁₁₁ described above. Examples of a substituent of an alkyl group having a substituent include a halogen atom (such as a chlorine atom, a bromine atom and a fluorine atom), an alkoxy group having a carbon number of 1 - 20 (such as a methoxy group, an ethoxy group, a n-propoxy group, an iso-propoxy group, a n-butoxy group and a tert-butoxy group), an acyl group (such as an acetyl group, a propionyl group and a trifluoroacetyl group), an acyloxy group having a carbon number of 1 - 20 (such as an acetoxy group, a propionyloxy group and a trifluoroacetoxy group), an alkoxycarbonyl group having a carbon number of 1 - 20 (such as a methoxycarbonyl group, an ethoxycarbonyl group and a tert-butoxycarbonyl group) and an alkylthiocarbonyl group having a carbon number of 2 - 20 (such as a methylthiocarbonyl group, an ethylthiocarbonyl group and a tert-butylthiocarbonyl group), an aryloxycarbonyl group, an alkylsulfonyl group, an arylsulfonyl group, a cyano group and a nitro group. Preferable substituents are an alkoxy group and an alkoxycarbonyl group.

Y₁₁ and Y₁₂ are 0 or S and preferably 0. m11 is 0 - 3 and preferably 1 or 2. p11 is 0, 1 or 2; q11, r11 and s11 are 0 or 1.

Specifically preferable alicyclic epoxide, with respect to forming cured film having a high hardness and improving adhesion of cured film with a substrate, is a compound represented by aforesaid Formula (A-II).

In the above formula, R₁₂₁ is identical with aforesaid R₁₁₁. Y₂₁ and Y₂₂ are O or S and preferably O. m12 is 0 - 2 and preferably 0 or 1. p12 is 0, 1 or 2; q12, r12 and s12 are 0 or 1. R₁₂₂, R₁₂₃ and R₁₂₄ are identical with R₁₁₂, R₁₁₃ and R₁₁₄·

Furthermore preferable alicyclic epoxide, with respect to forming cured film having a high hardness, improving adhesion of cured film with a substrate, and curing sensitivity hardly being affected by variation of printing environment, are compounds represented by aforesaid Formula (A-III), (A-IV) or (A-V).

In the above formula, R₁₃₁, R₁₄₁ and R₁₅₁ are identical with aforesaid R₁₁₁. m13, m14 and m15 are 0 - 2 and preferably 0 or 1. p13 and p14 are 0, 1 or 2; q13 and s15 are 0 or 1. R₁₃₂, R₁₃₃, R₁₃₄, R₁₄₂, R₁₄₃, R₁₄₄ and R₁₅₄ are identical with R₁₁₂, R₁₁₃ and R₁₁₄.

Specifically preferable alicyclic epoxide, with respect to forming cured film having a high hardness, improving adhesion of cured film with a substrate, and curing sensitivity hardly being affected by variation of printing environment, is a compound represented by aforesaid Formula (A-VI).

In the above formula, R₁₆₁₁ and R₁₆₁₂ are a hydrogen atom or an alkyl group having a carbon number of 1 - 6 (such as a methyl group, an ethyl group, a propyl group, an isopropyl group, a butyl group, a sec-butyl group, t-butyl group, a pentyl group and a hexyl group), and preferable alkyl groups are a methyl group, an ethyl group and a propyl group. R₁₆₂, R₁₆₃ and R₁₆₄ are identical with R₁₁₂, R₁₁₃ and R₁₁₄. q16 is 0 or 1.

In the following, specific examples of a mono-functional alicyclic epoxy compound (Formula (A)) of this invention will be shown; however, this invention is not limited thereto.

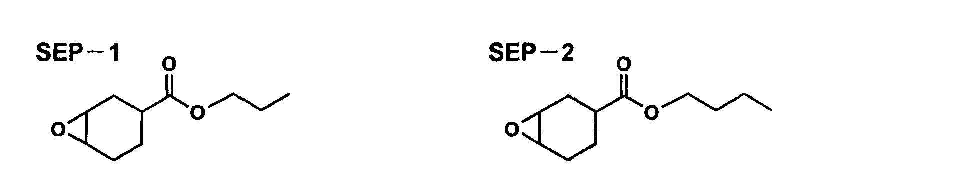

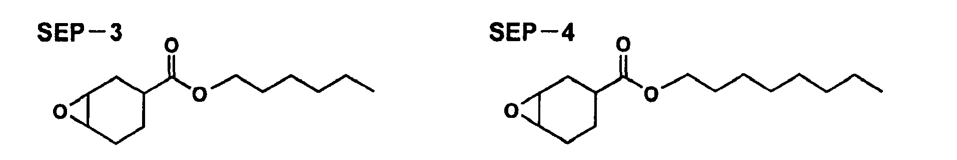

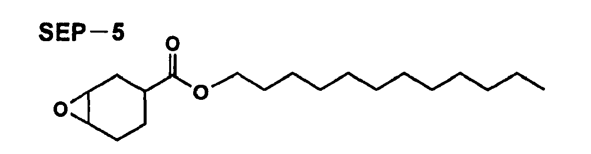

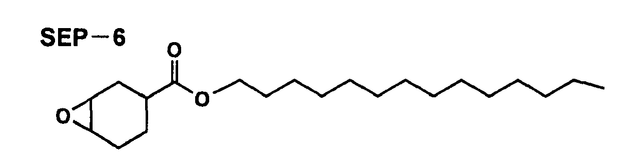

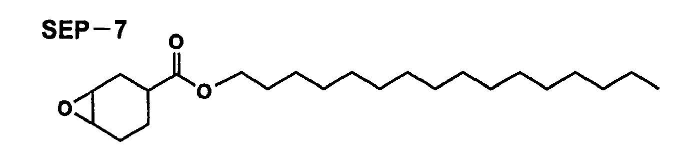

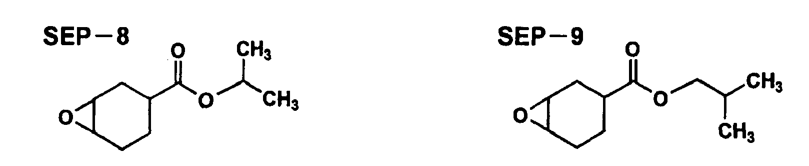

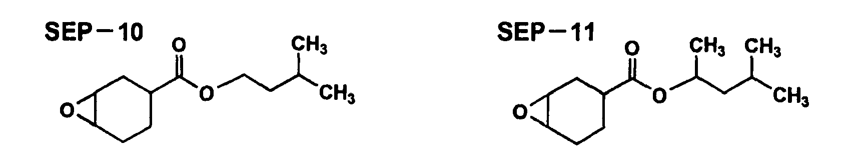

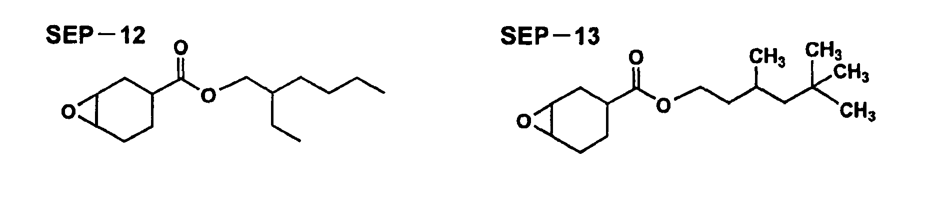

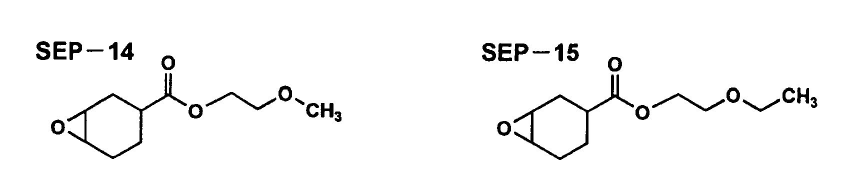

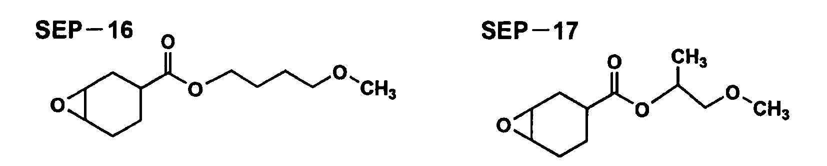

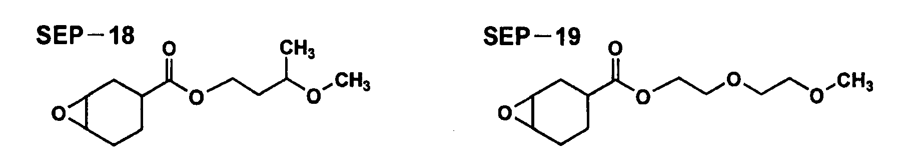

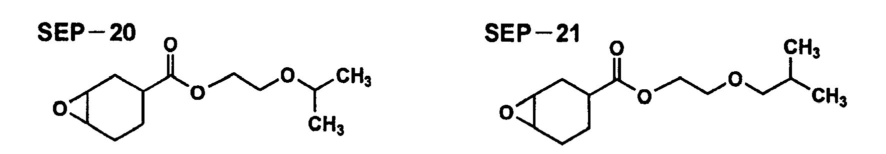

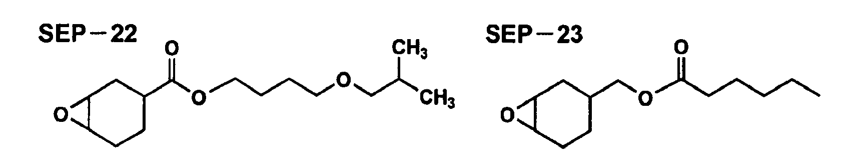

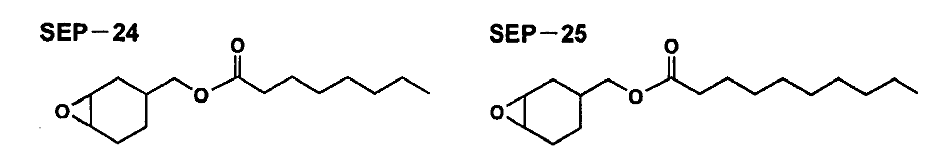

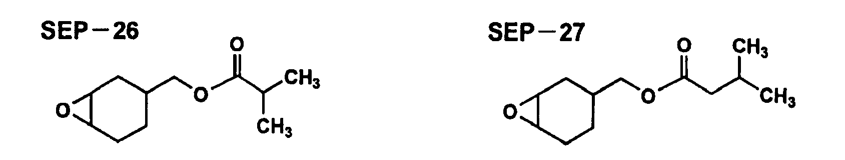

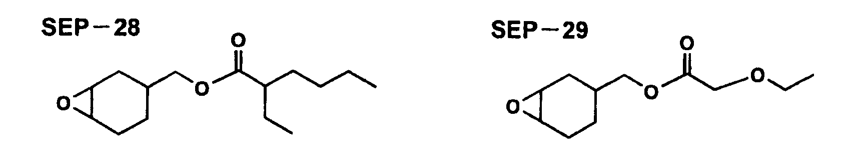

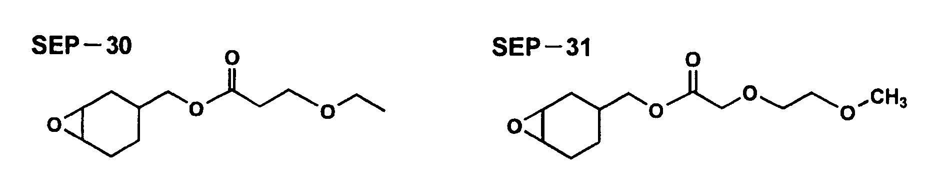

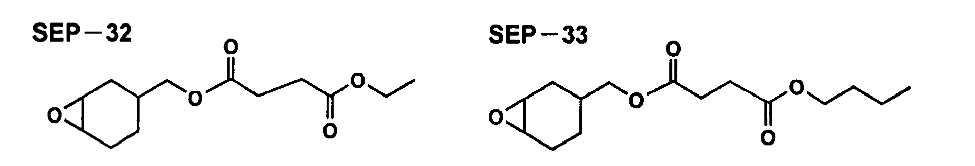

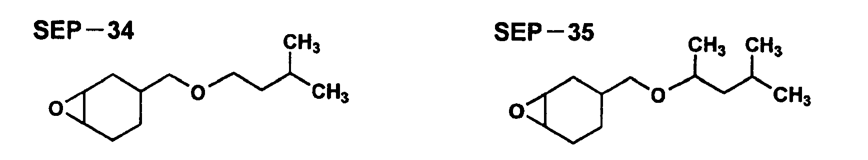

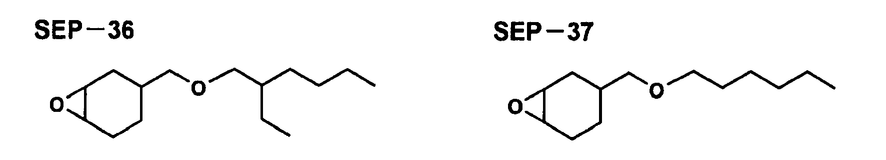

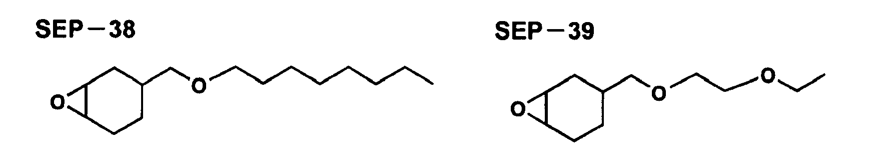

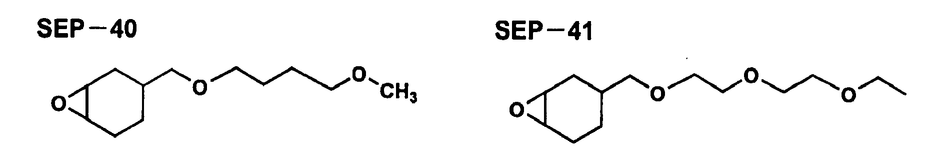

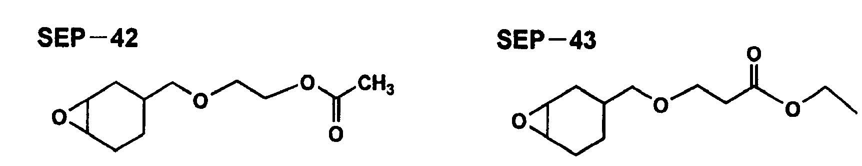

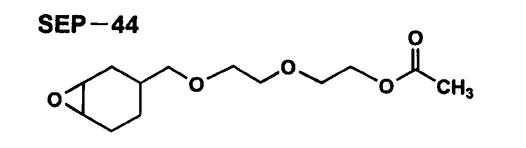

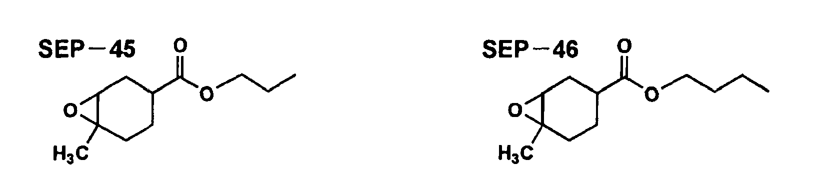

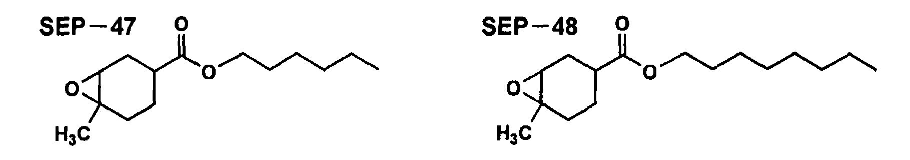

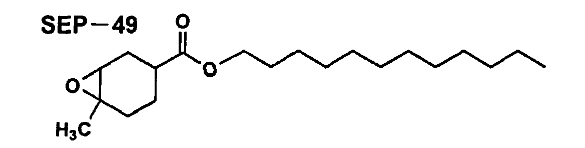

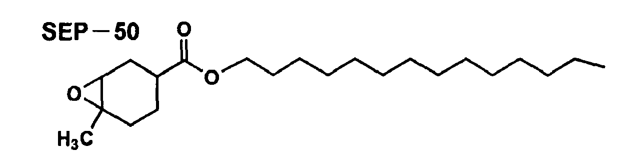

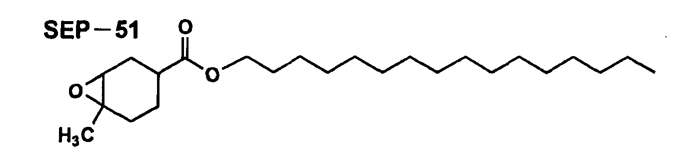

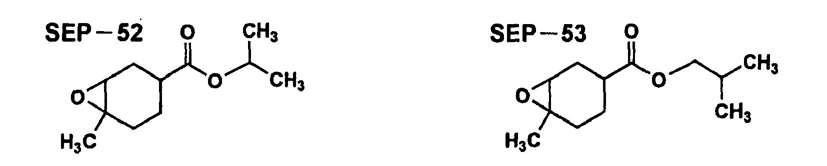

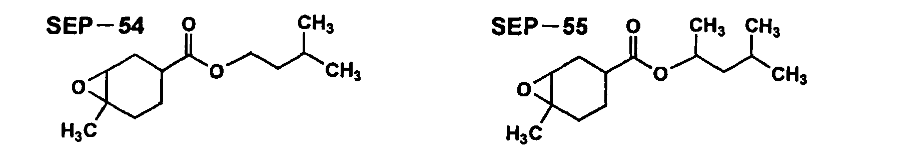

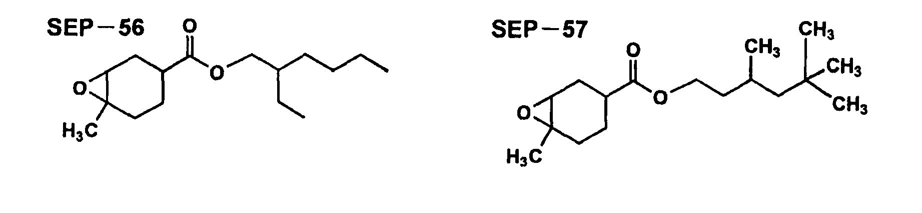

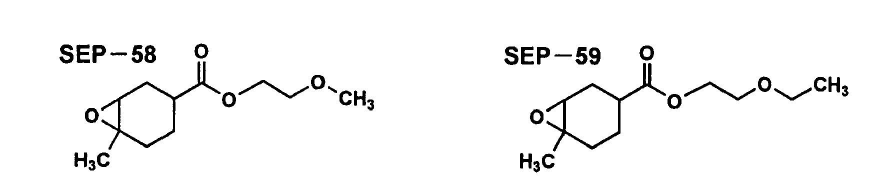

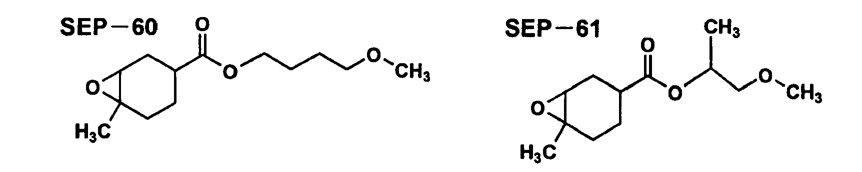

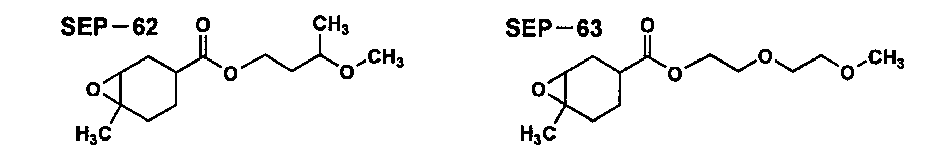

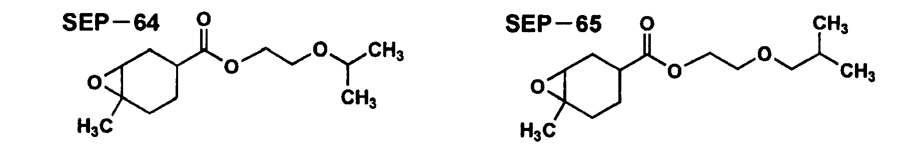

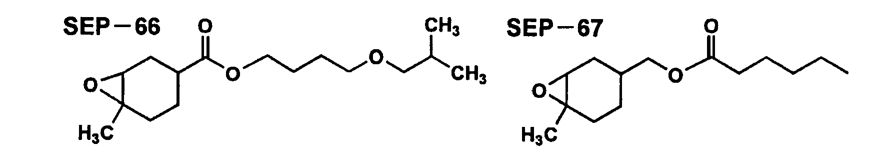

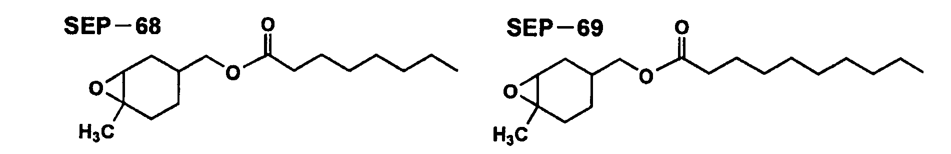

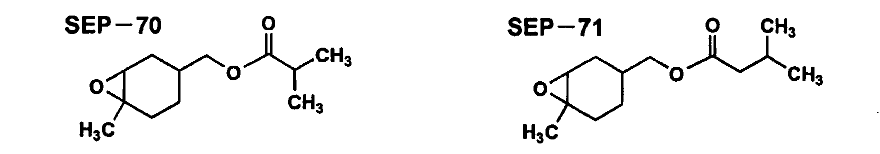

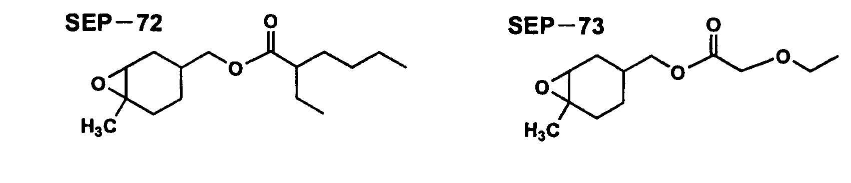

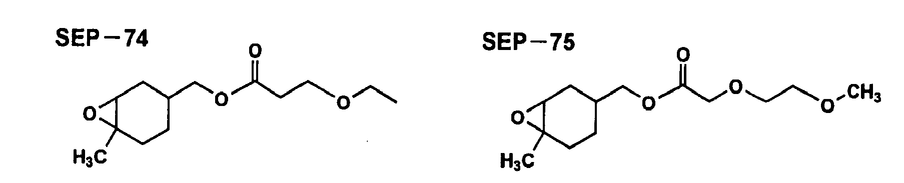

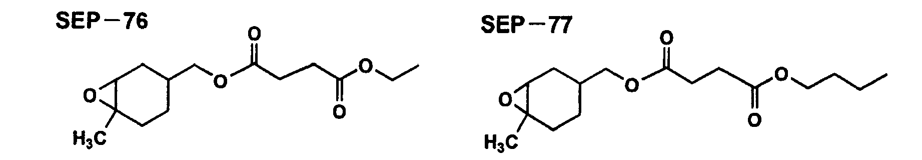

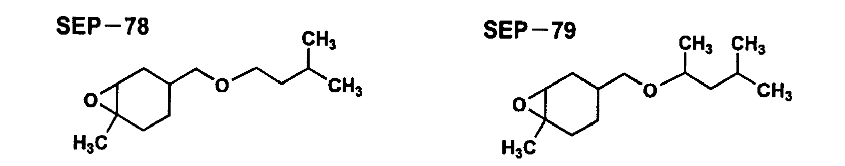

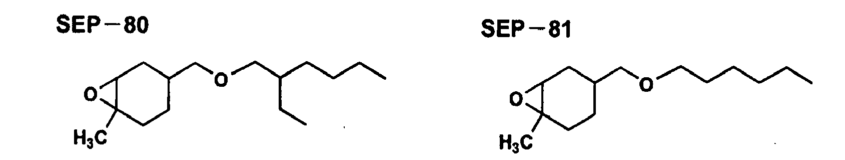

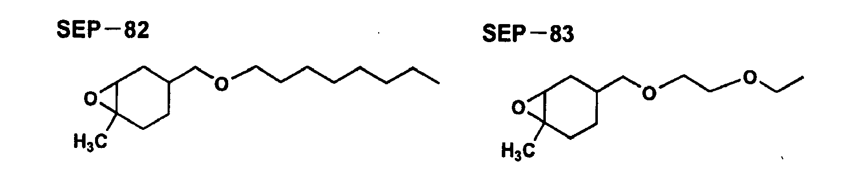

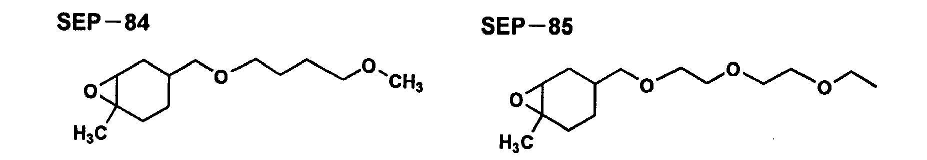

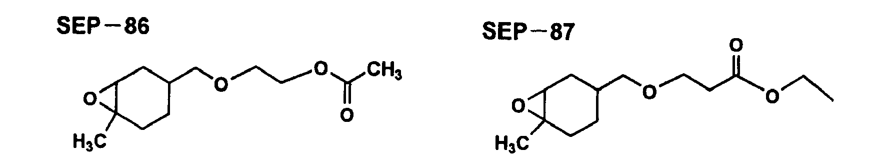

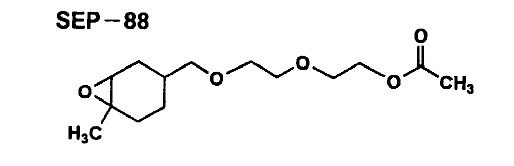

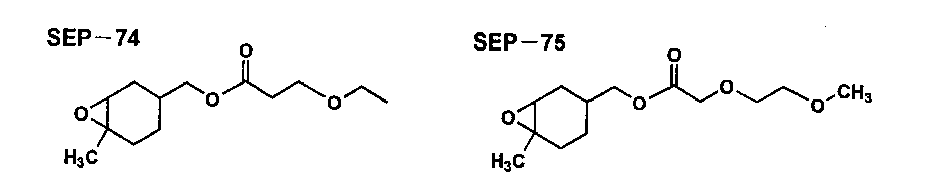

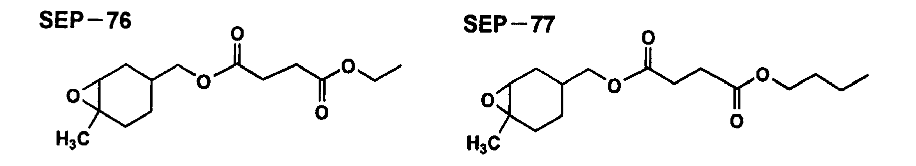

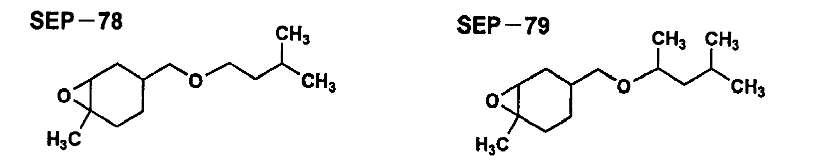

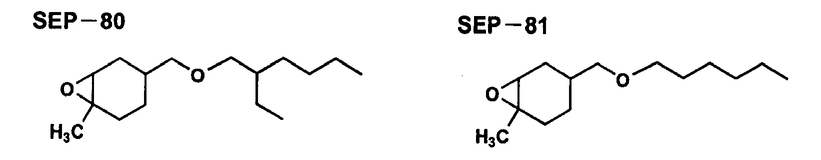

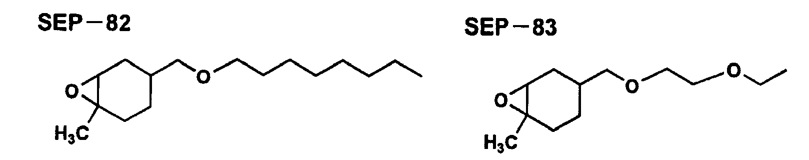

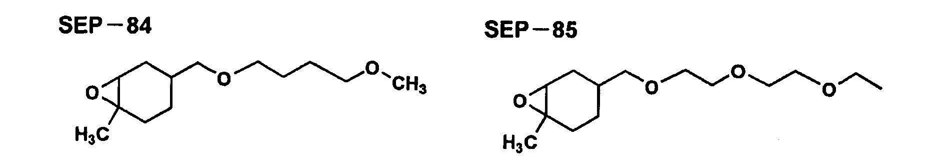

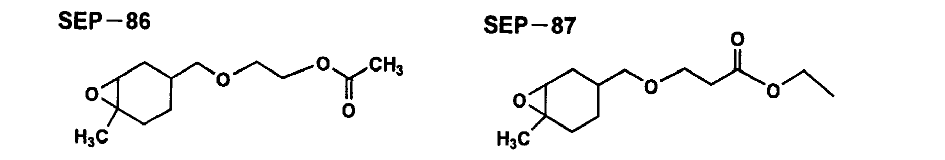

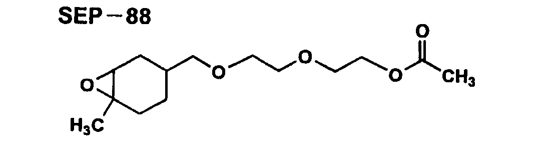

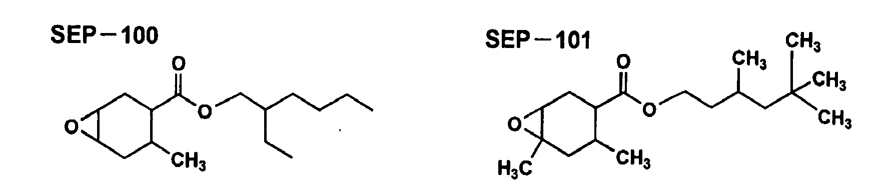

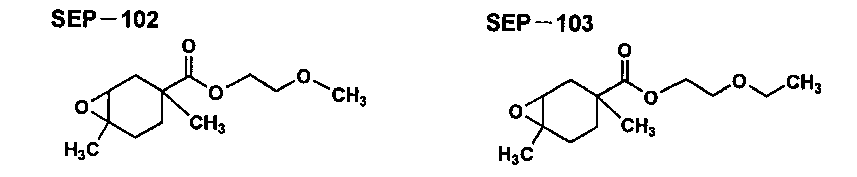

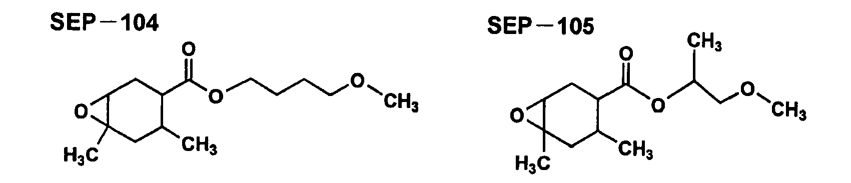

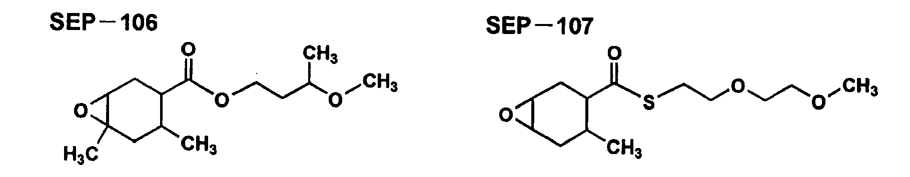

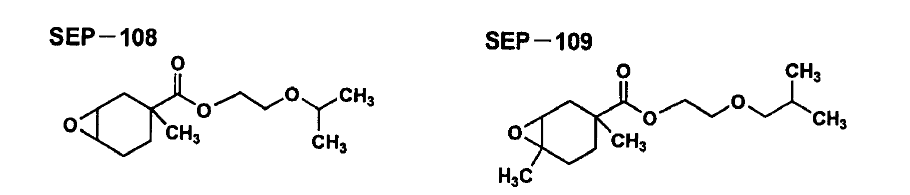

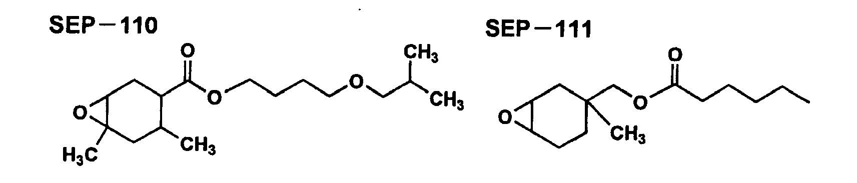

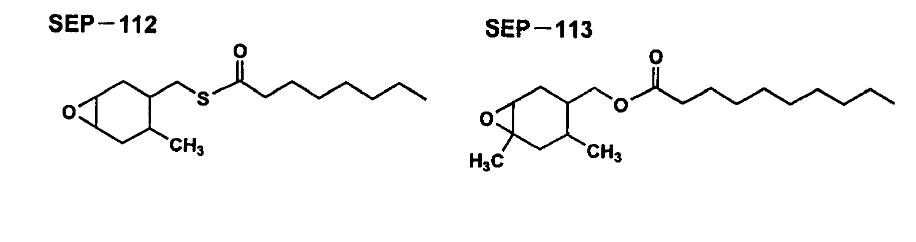

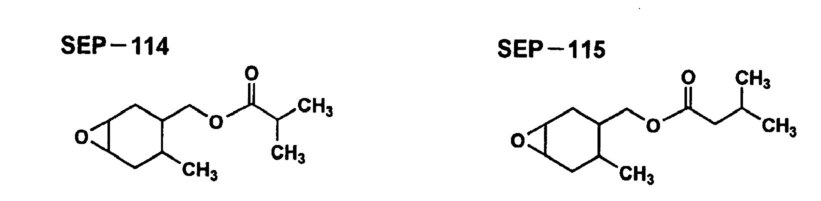

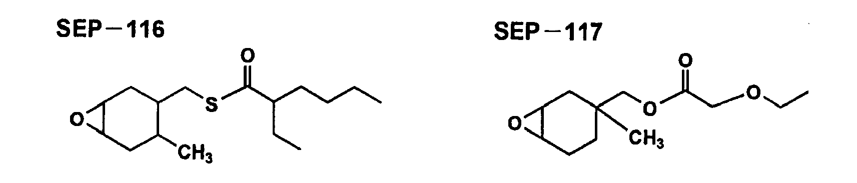

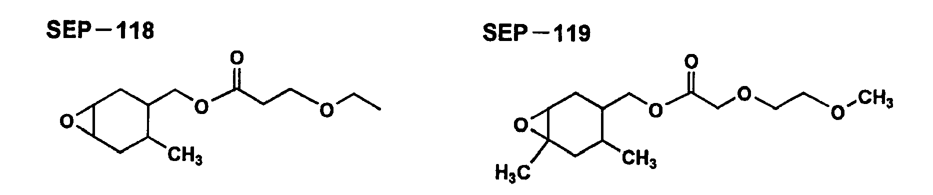

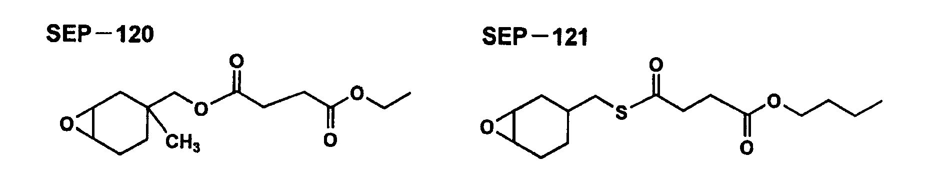

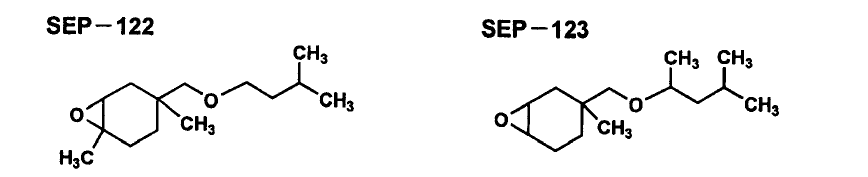

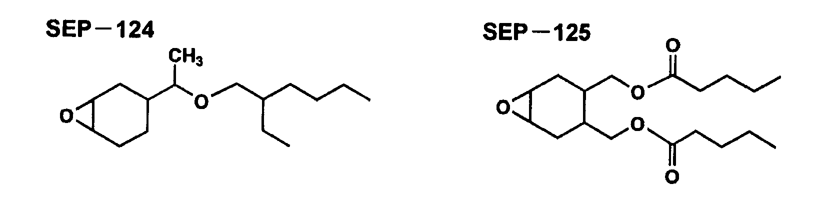

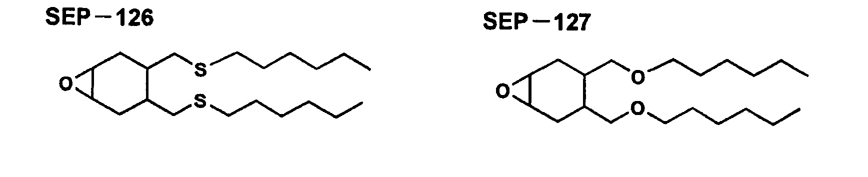

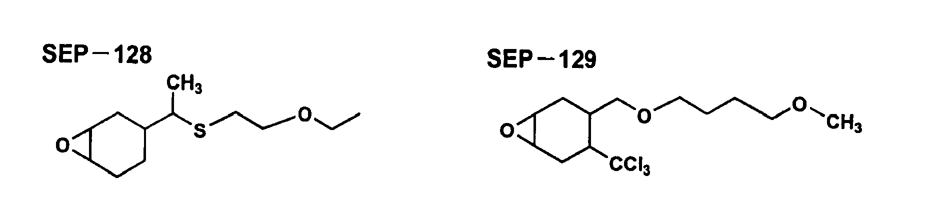

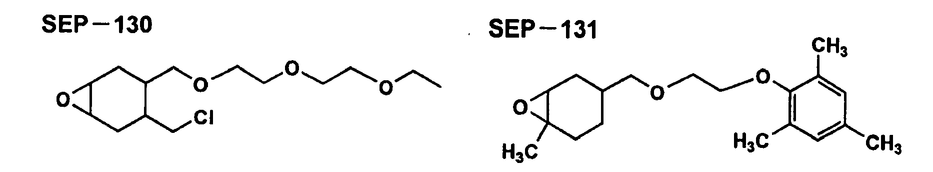

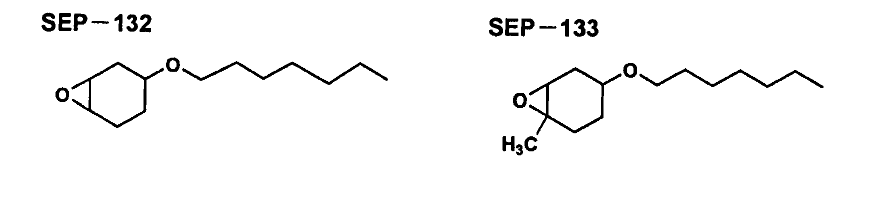

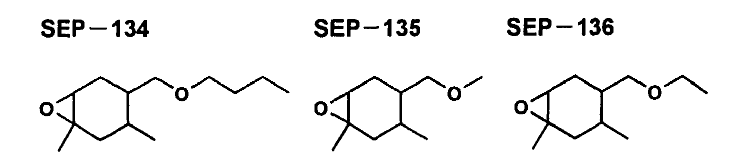

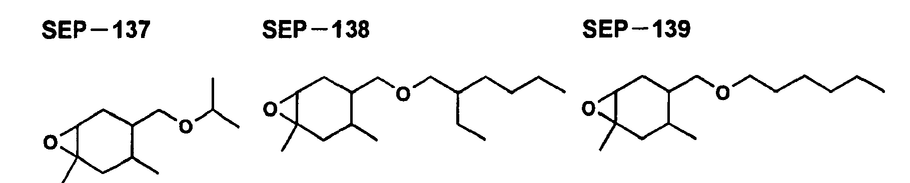

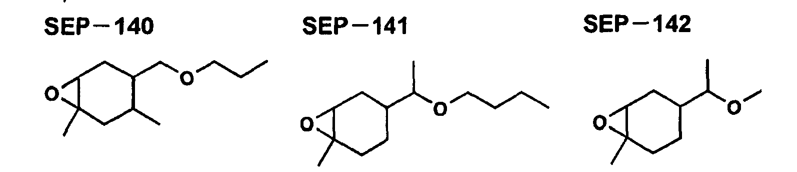

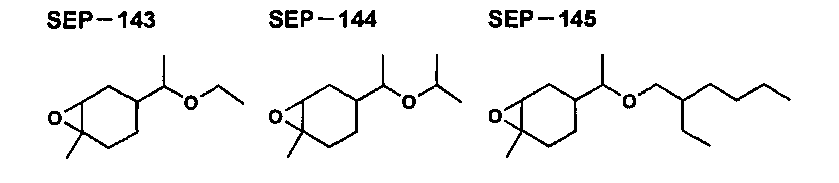

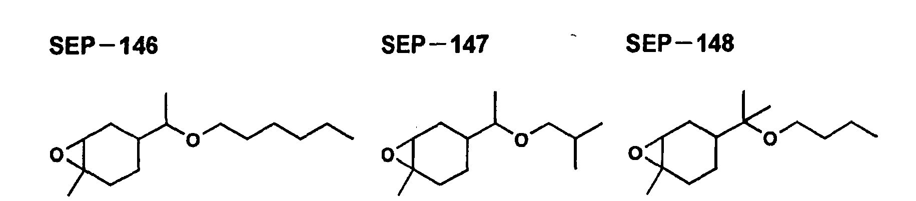

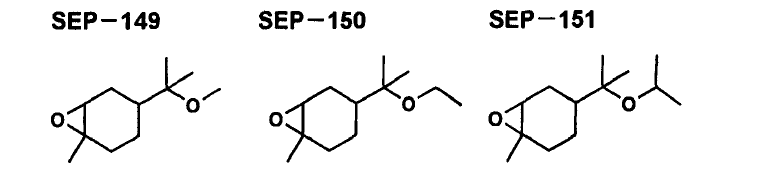

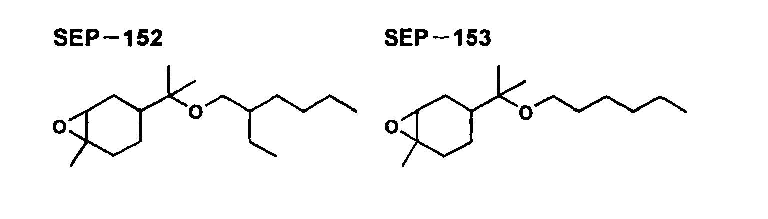

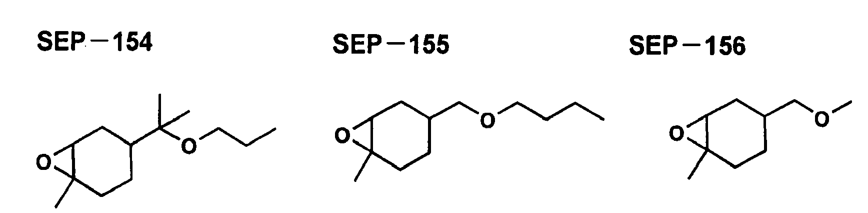

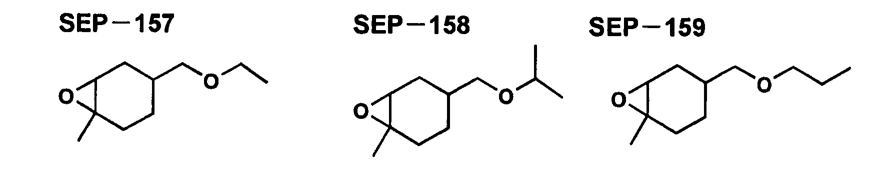

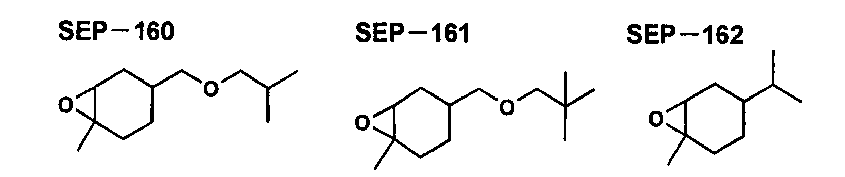

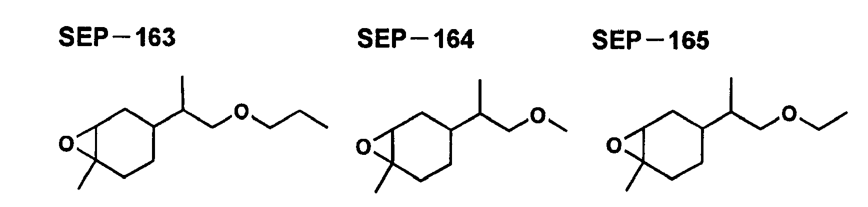

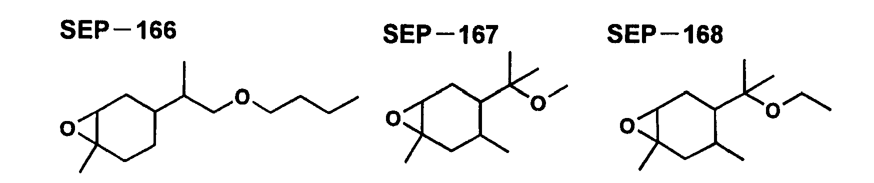

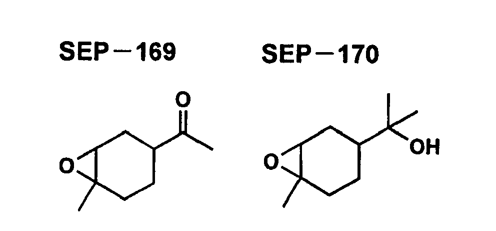

An addition amount of a mono-functional epoxy compound according to this invention is preferably 10 - 20 weight%. When the addition amount is less than 10 weight%, the cured film is not provided with sufficient flexibility while when it is less than 10 weight%, physical properties after being cured is too weak to be utilized. In this invention, one type of a mono-functional epoxy compound may be utilized alone or at least two types may be utilized appropriately in combination.

These alicyclic epoxy compounds, although the manufacturing method is not specifically limited, can be synthesized referring to, for example, the forth edition, Experimental Chemistry Course 20, Organic Synthesis II, p. 213 -, published by Maruzen KK Shuppan (1992); "The Chemistry of Heterocyclic Compounds-Small Ring Heterocycles part 3, Oxiranes", edited by Alfred Hasfner, John & Willey and Sons, An Interscience Publication, New York (1985); Yoshimura, Adhesion vol. 29, No. 12, 32 (1985), Yoshimura, Adhesion vol. 30, No. 5, 42 (1986), Yoshimura, Adhesion vol. 30, No. 7, 42 (1986), JP-A Nos. 11-100378, 4-36263 and 4-69360.

In an actinic ray curable composition of this invention, a poly-functional alicyclic epoxy compound can be utilized in combination together with a compound represented by Formula (X), an oxetane compound and a mono-functional epoxy compound represented by Formula (A). Thereby, an effect of sensitivity improvement or an improvement effect of cured film physical properties can be obtained.

As bi-functional alicyclic epoxide, preferable is a poly-functional epoxide compound represented by following Formula (B).

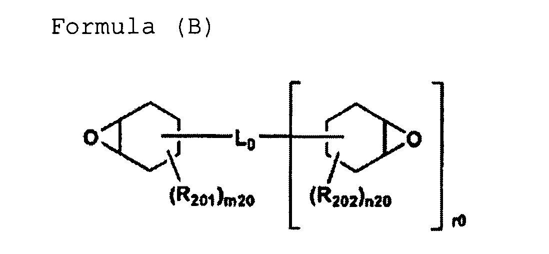

In the formula, R₂₀₁ and R₂₀₂ are a substituent; and m20 and n20 are 0, 1 or 2, and preferably 0 or 1. r0 is 1 - 3. L₀ is an r0 + 1 valent connecting group, which may contain an oxygen atom or a sulfur atom in the main chain and has a carbon number of 1 - 15, or a single bond.

In the above formula, examples of a substituent represented by R₂₀₁ and R₂₀₂ are a halogen atom (such as a chlorine atom, a bromine atom and a fluorine atom), an alkyl group having a carbon number of 1 - 6 (such as a methyl group, an ethyl group, a propyl group, an isopropyl group and a butyl group), an alkoxy group having a carbon number of 1 - 6 (such as a methoxy group, an ethoxy group, a n-propoxy group, an iso-propoxy group and n-butoxy group and a tert-butoxy group), an acyl group (such as an acetyl group, a propionyl group and a trifluoroacetyl group), an acyloxy group (such as an acetoxy group, a propionyloxy group and a trifluoroacetoxy group), an alkoxycarbonyl group (such as a methoxycarbonyl group, an ethoxycarbonyl group and a tert-butoxycarbonyl group). Preferable substituents are an alkyl group, an alkoxy group and an alkoxycarbonyl group.

An example of a divalent connecting group, which may contain an oxygen atom or a sulfur atom in the main chain and has a carbon number of 1 - 15, includes the following groups and groups formed by combining these groups with a plural number of -O- group, -S- group, -CO- group and -CS- group.
Methylene group [-CH₂-]
Ethylidene group [>CHCH₃]
Isopropylidene group [>C(CH₃)₂]
1,2-Ethylene group [-CH₂CH₂-]
1,2-Propylene group [-CH (CH₃) CH₂-]
1,3-Propanediyl group [-CH₂CH₂CH₂-]
2,2-Dimethyl-1,3-propanediyl group [-CH₂C(CH₃)₂CH₂-]
2,2-Dimethoxy-1,3-propanediyl group
[-CH₂C (OCH₃)₂CH₂-1]
2,2-Dimethoxymethyl-1,3-propanediyl group
[-CH₂C(CH₂OCH₃)₂CH₂- ]
1-Methyl-1,3-propanediyl group [-CH(CH₃)CH₂CH₂-]
1,4-Butanediyl group [-CH₂CH₂CH₂CH₂-]
1,5-Pentanediyl group [-CH₂CH₂CH₂CH₂CH₂-]
Oxydiethylene group [-CH₂CH₂OCH₂CH₂-]
Thiodiethylene group [-CH₂CH₂SCH₂CH₂-]
3-Oxthothiodiethylene group [-CH₂CH₂SOCH₂CH₂-]
3,3-Dioxthothiodiethylene group [-CH₂CH₂SO₂CH₂CH₂-]
1,4-Dimethyl-3-oxa-1,5-pentadiyl group
[-CH(CH₃)CH₂OCH(CH₃)CH₂-]
3-Oxopentanediyl group [-CH₂CH₂COCH₂CH₂-]
1,5-Dioxo-3-oxapentanediyl group [-COCH₂OCH₂CO-]
4-Oxa-1, 7-heptanediyl group [-CH₂CH₂CH₂OCH₂CH₂CH₂-]
3,6-Dioxa-1,8-octanediyl group
[-CH₂CH₂OCH₂CH₂OCH₂CH₂- ]
1,4,7-Trimethyl-3,6-dioxa-1,8-octanediyl group
[-CH(CH₃)CH₂O-CH (CH₃) CH₂OCH (CH₃) CH₂-]
5,5-Dimethyl-3,7-dioxa-1,9-nonanediyl group
[-CH₂CH₂OCH₂C(CH₃)₂CH₂OCH₂CH₂-]
5,5-Dimethoxy-3,7-dioxa-1,9-nonanediyl group [-CH₂CH₂OCH₂C(OCH₃)₂CH₂OCH₂CH₂-]
5,5-Dimethoxymethyl-3,7-dioxa-1,9-nonanediyl group [-CH₂CH₂OCH₂C (CH₂OCH₃)₂CH₂OCH₂CH₂-]
4,7-Dioxo-3,8-dioxa-1,10-decanediyl group
[-CH₂CH₂OCOCH₂CH₂COOCH₂CH₂-]
3,8-Dioxo-4,7-dioxa-1,10-decanediyl group
[-CH₂CH₂COOCH₂CH₂OCOCH₂CH₂-]
1,3-Cyclopentanediyl group [-1,3-C₅H₈-]
1,2-Cyclohexanediyl group [-1,2-C₆H₁₀-]
1,3-Cyclohexanediyl group [-1,3-C₆H₁₀-]
1,4-Cyclohexanediyl group [-1,4-C₆H₁₀-]
2,5-Tetrahydrofurandiyl group [2,5-C₄H₆O-]
p-Phenylene group [-p-C₆H₄-]
m-Phenylene group [-m-C₆H₄-]
α,α'-o-Xylylene group [-O-CH₂-C₆H₄-CH₂-]
α,α'-m-Xylylene group [-m-CH₂-C₆H₄-CH₂-]
α,α' -p-Xylylene group [-p-CH₂-C₆H₉-CH₂-]
Furan-2,5-diyl-bismethylene group [2,5-CH₂-C₄H₂O-CH₂-]
Thiophen-2, 5-diyl-bismethylene [2,5-CH₂-C₄H₂S-CH₂-] i-Propylidenbis-p-phenylene [-p-C₆H₄-C(CH₃)₂-p-C₆H₄-]

A not less than tri-valent connecting group includes groups which are formed by removing necessary hydrogen atoms at arbitrary positions from the divalent groups listed above, and groups formed by combining them with a plural number of - O- group, -S- group, -CO- group and -CS- group.

L₀ may be provided with a substituent. Examples of a substituent include a halogen atom (such as a chlorine atom, a bromine atom and a fluorine atom), an alkyl group having a carbon number of 1 - 6 (such as a methyl group, an ethyl group, a propyl group, an isopropyl group and a butyl group), an alkoxy group having a carbon number of 1 - 6 (such as a methoxy group, an ethoxy group, a n-propoxy group, an iso-propoxy group, a n-butoxy group and a tert-butoxy group), an acyl group (such as an acetyl group, a propionyl group and a trifluoroacetyl group), an acyloxy group (such as an acetoxy group, a propionyloxy group and a trifluoroacetoxy group) and an alkoxycarbonyl group (such as a methoxycarbonyl group, an ethoxycarbonyl group and a tert-butoxycarbonyl group). A preferable substituent is an alkyl group, an alkoxy group or an alkoxycarbonyl group.

L₀ is preferably a divalent connecting group which may contain an oxygen atom or a sulfur atom in the main chain and having a carbon number of 1 - 8, and more preferable is a divalent connecting group the main chain of which is comprised of only carbon atoms and having a carbon number of 1 - 5.

Specifically preferable alicyclic epoxide, with respect to forming cured film having a high hardness and improving adhesion of cured film with a substrate, is a compound represented by following Formula (B-I) or (B-II).

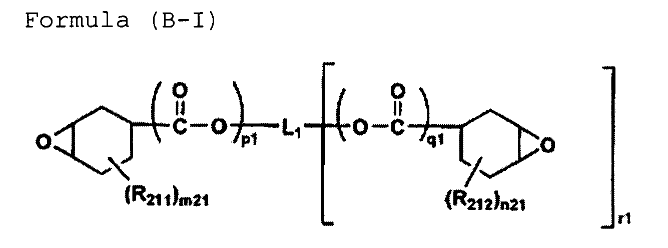

In the formula, R₂₁₁ and R₂₁₂ are a substituent; m21 and n21 are 0, 1 or 2, and preferably 0 or 1. p1 and q1 are each 0 or 1. r1 is 1 - 3. L₁ is an r1 + 1 valent connecting group, which may contain an oxygen atom or a sulfur atom in the main chain and has a carbon number of 1 - 15, or a single bond.

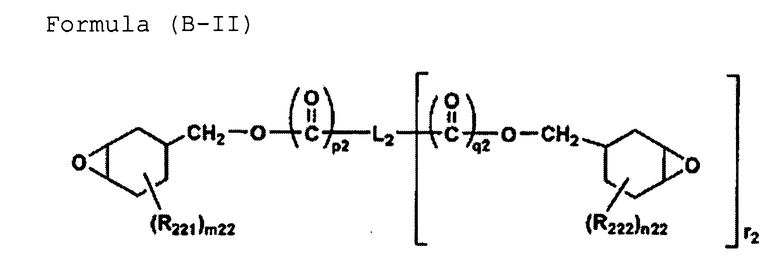

In the formula, R₂₂₁ and R₂₂₂ are a substituent; m22 and n22 are 0, 1 or 2, and preferably 0 or 1. p2 and q2 are each 0 or 1. r2 is 1 - 3. L₂ is an r2 + 1 valent connecting group, which may contain an oxygen atom or a sulfur atom in the main chain and has a carbon number of 1 - 15, or a single bond.

In the above formula, R₂₁₁, R₂₁₂, R₂₂₁ and R₂₂₂ are identical with R₁₁₁ in aforesaid Formula (A-1).

Examples of a divalent connecting group, represented by L₁ and L₂, which may contain an oxygen atom or a sulfur atom in the main chain and has a carbon number of 1 - 15, include those described in explanation of L₀. L₁ and L₂ are preferably a divalent connecting group which may contain an oxygen atom or a sulfur atom in the main chain and having a carbon number of 1 - 8, and more preferable is a divalent connecting group the main chain of which is comprised of only carbon atoms and having a carbon number of 1 - 5.

Specifically preferable alicyclic epoxide, with respect to forming cured film having a high hardness and curing sensitivity hardly being affected by variation of printing environment, are compounds represented by following Formula (B-III) or (B-IV).

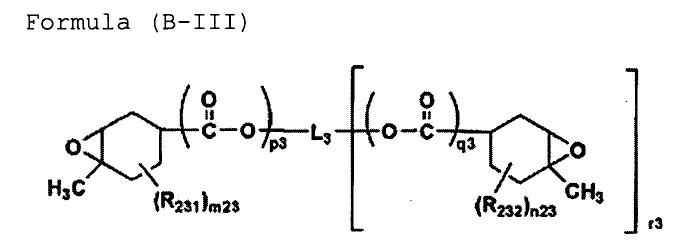

In the formula, R₂₃₁ and R₂₃₂ are a substituent; m23 and n23 are 0 or 1. p3 and q3 are each 0 or 1. r3 is 1 - 3. L₃ is an r3 + 1 valent connecting group provided with a branched structure, which may contain an oxygen atom or a sulfur atom in the main chain and has a carbon number of 1 - 15, or a single bond.

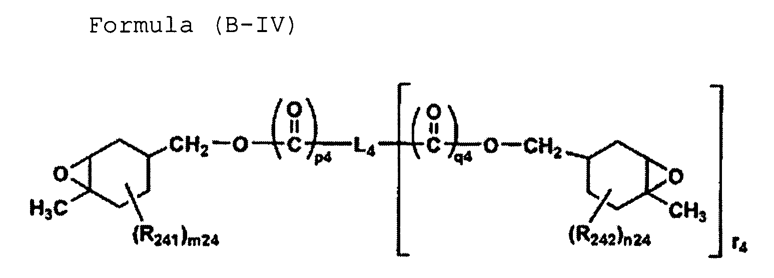

In the formula, R₂₄₁ and R₂₄₂ are a substituent; m24 and n24 are 0 or 1. p4 and q4 are each 0 or 1. r4 is 1 - 3. L₄ is an r4 + 1 valent connecting group provided with a branched structure, which may contain an oxygen atom or a sulfur atom in the main chain and has a carbon number of 1 - 15, or a single bond.

In the above formula, R₂₃₁, R₂₃₂, R₂₄₁ and R₂₄₂ are identical with R₁₁₁ in aforesaid Formula (A-1). Examples of a divalent connecting group, represented by L₃ and L₄, which may contain an oxygen atom or a sulfur atom in the main chain and has a carbon number of 1 - 15, include those described in explanation of L₀.

In the following, specific examples of a preferable alicyclic epoxide will be shown; however, this invention is not limited thereto.

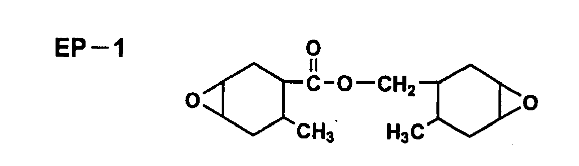

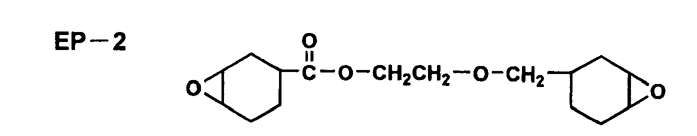

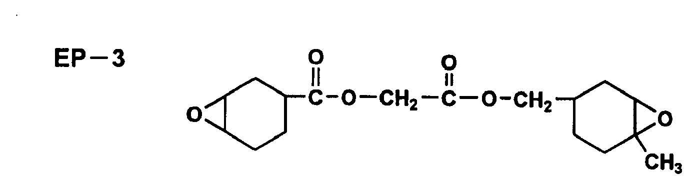

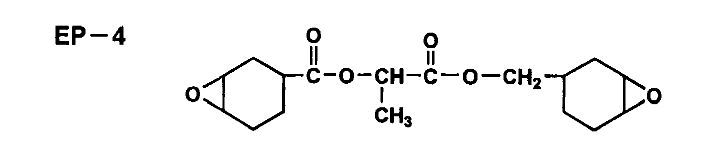

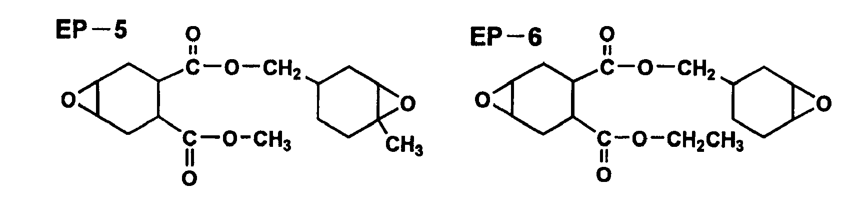

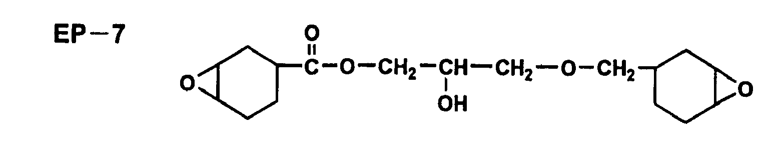

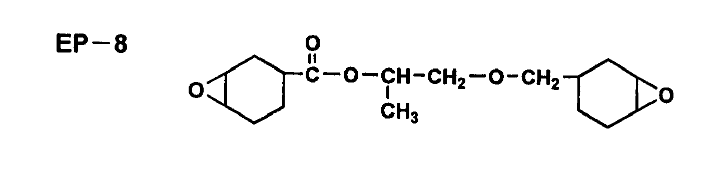

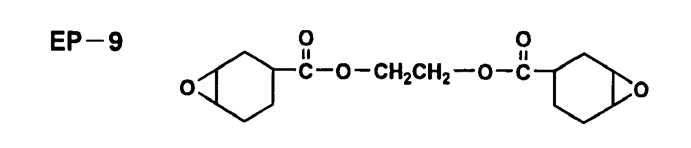

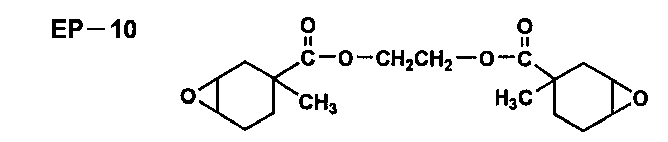

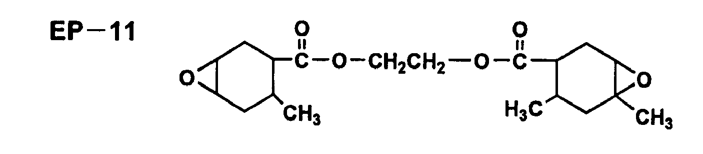

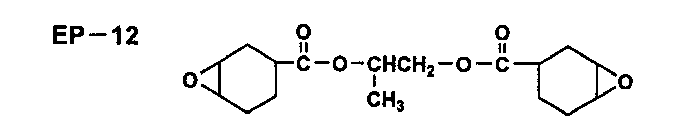

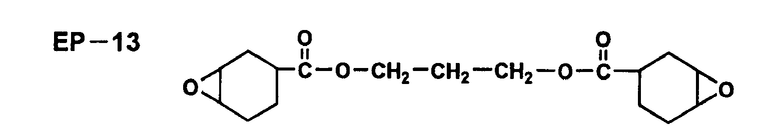

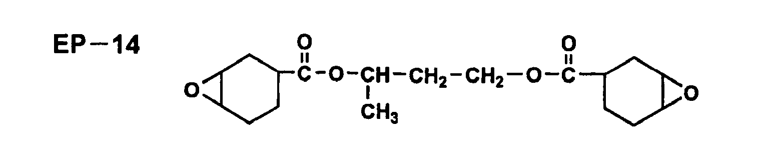

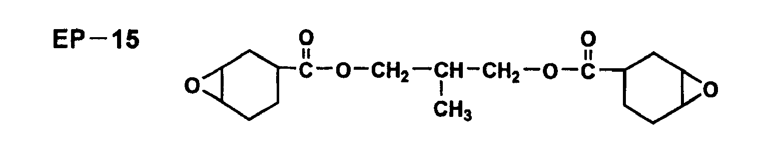

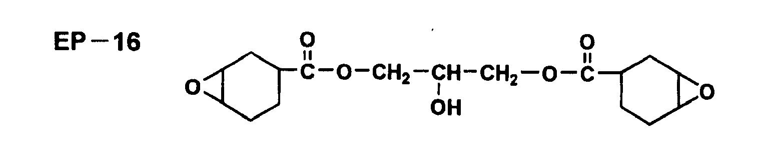

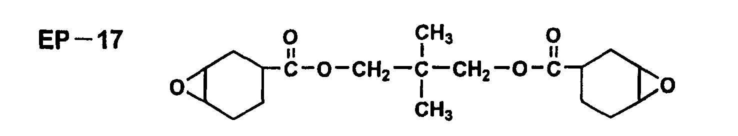

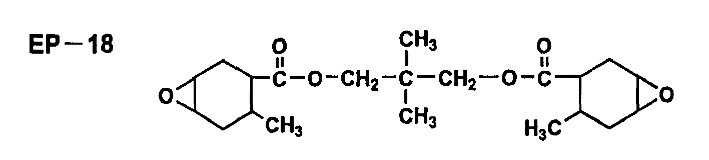

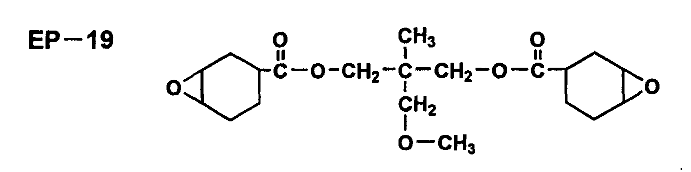

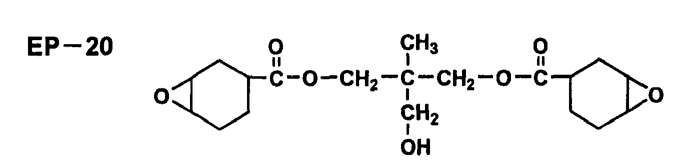

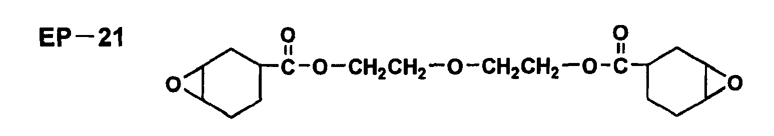

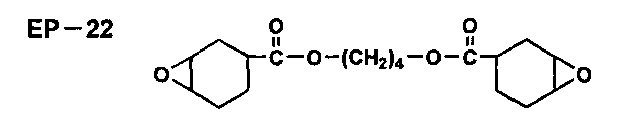

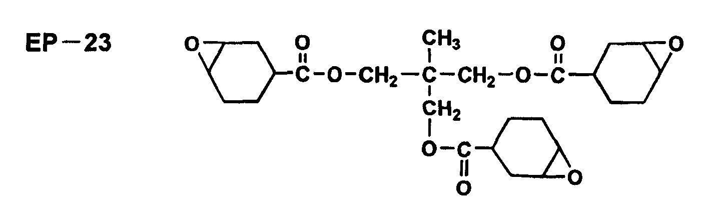

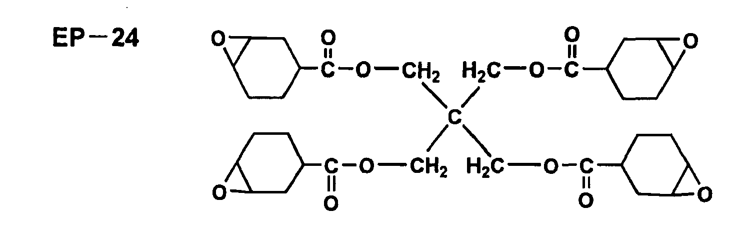

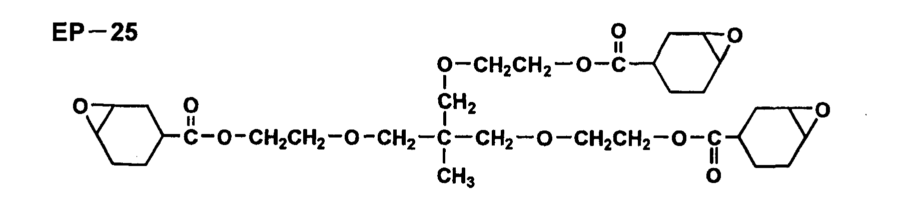

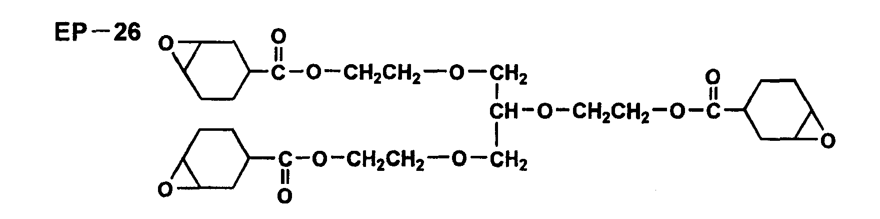

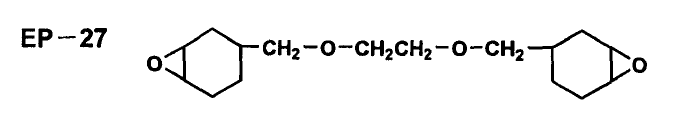

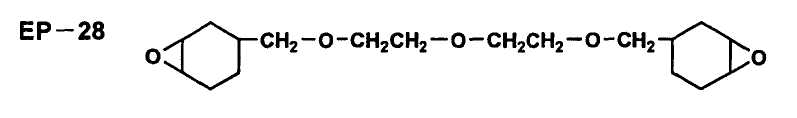

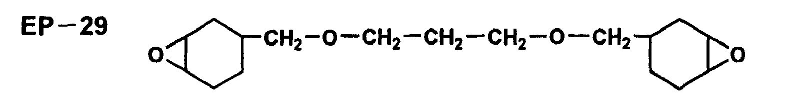

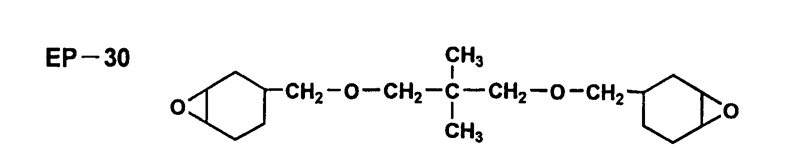

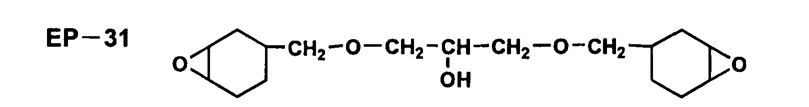

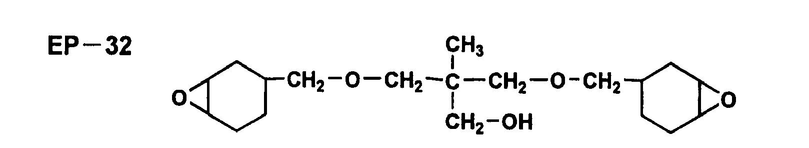

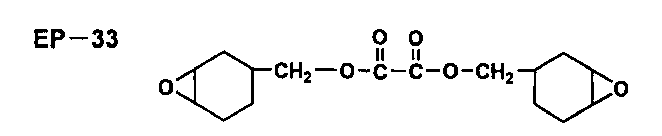

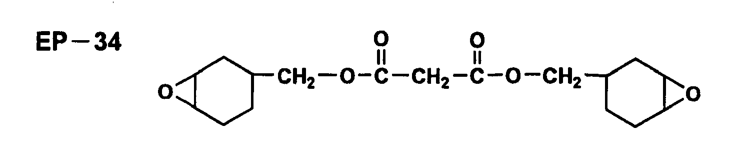

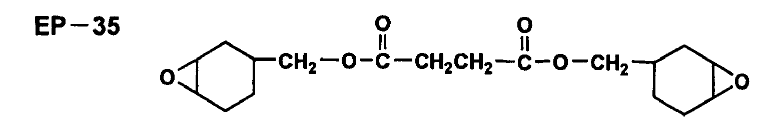

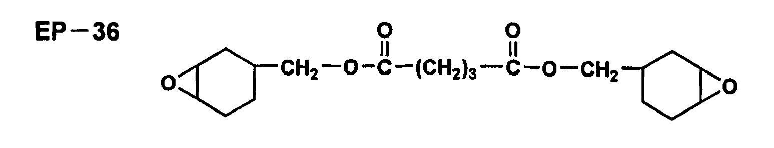

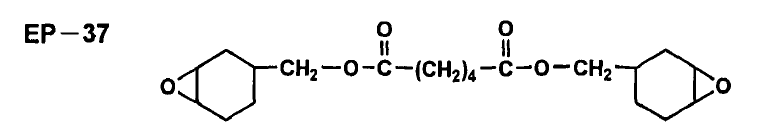

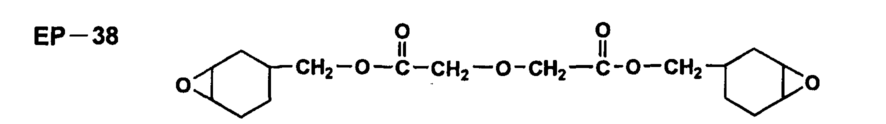

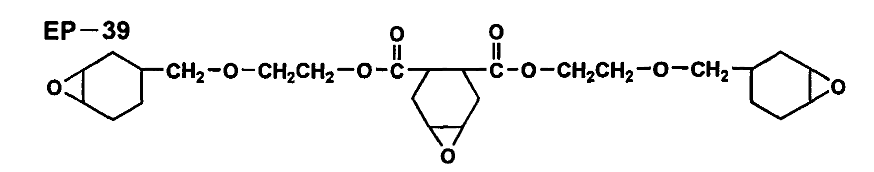

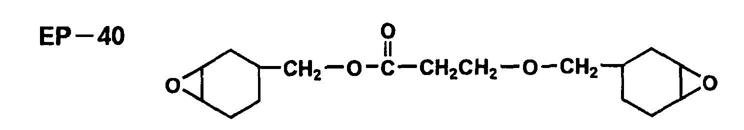

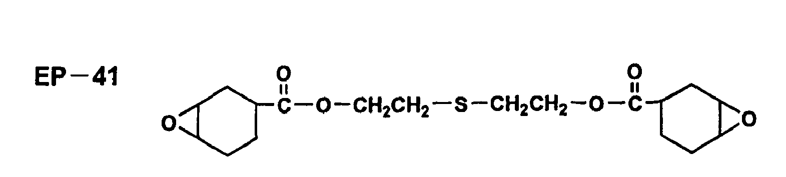

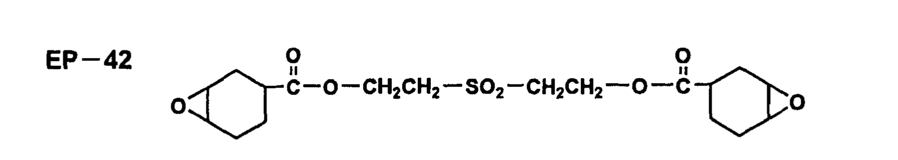

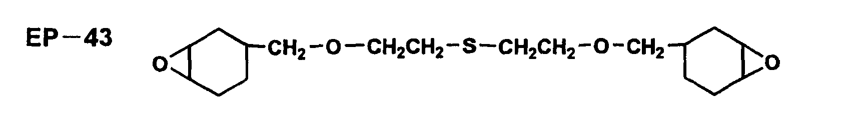

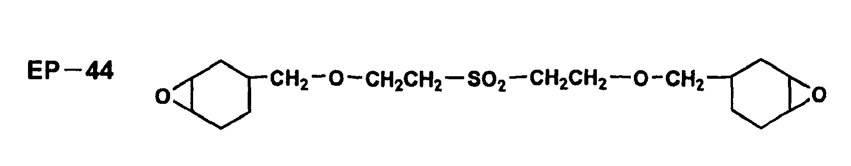

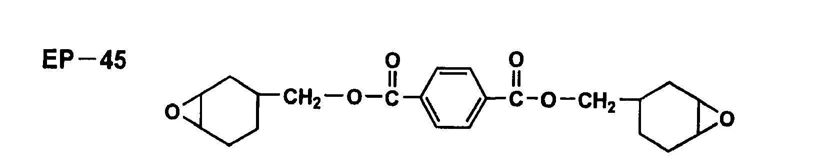

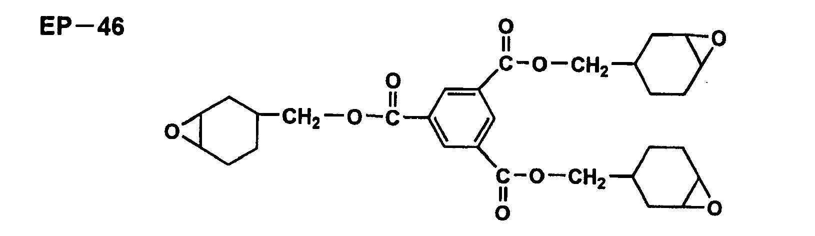

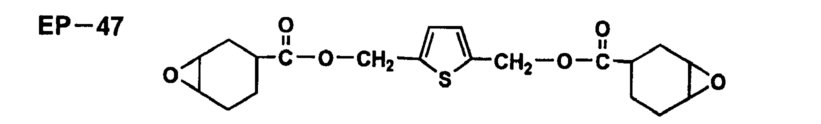

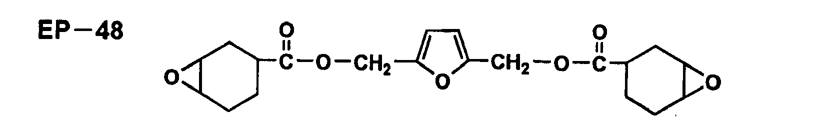

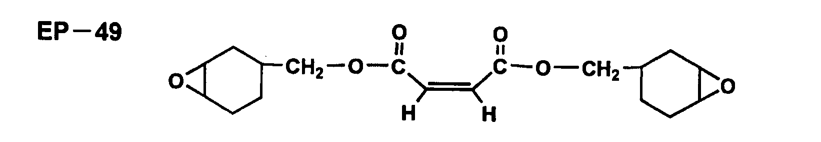

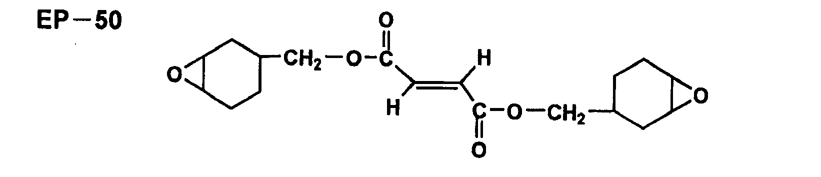

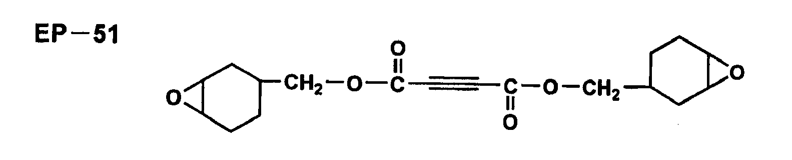

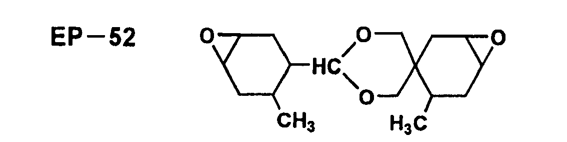

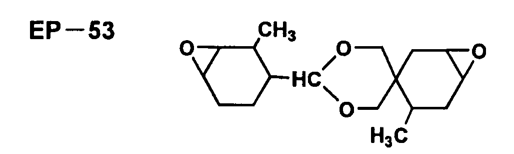

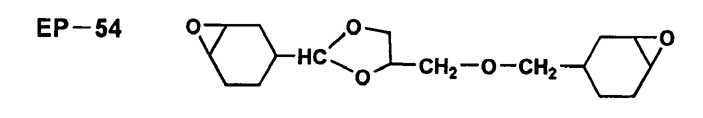

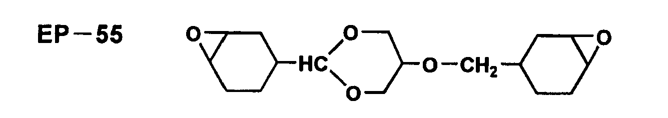

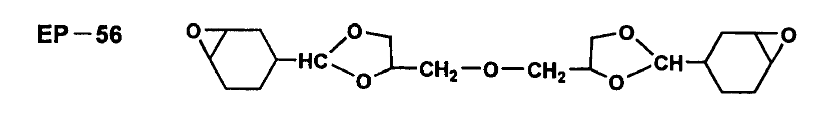

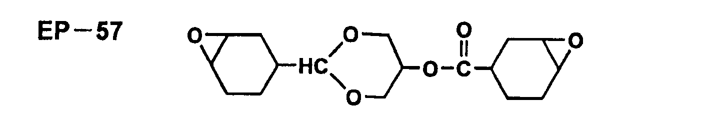

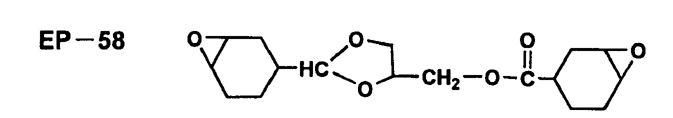

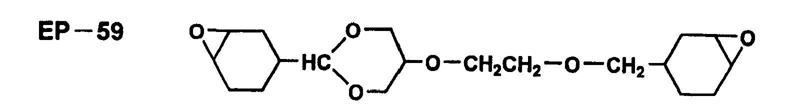

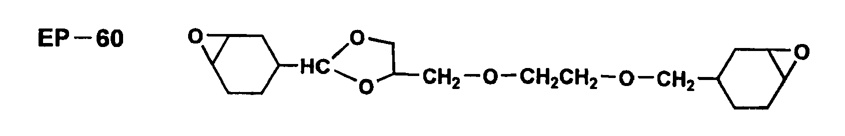

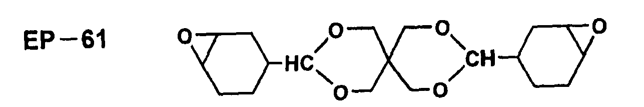

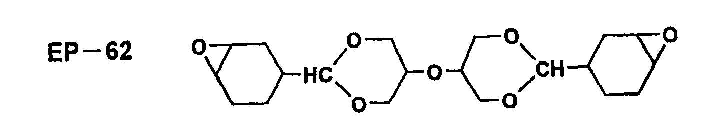

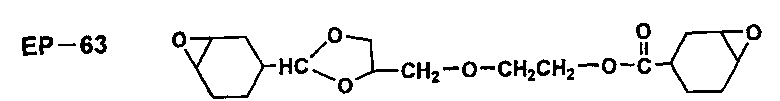

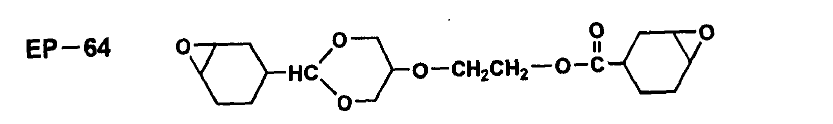

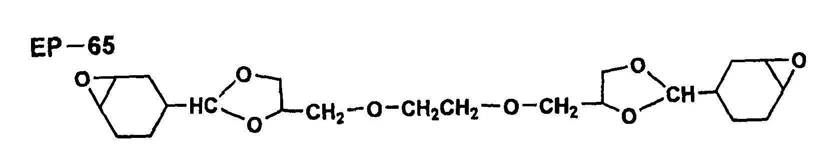

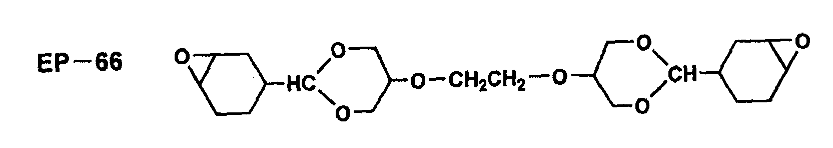

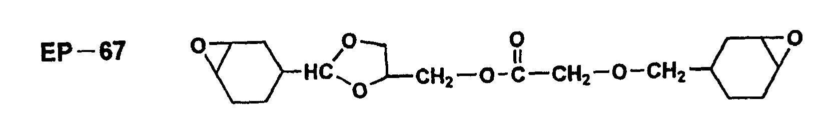

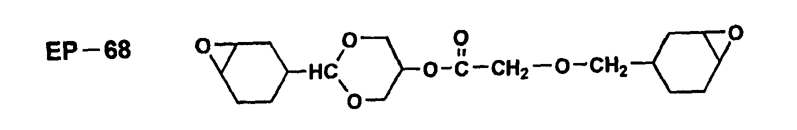

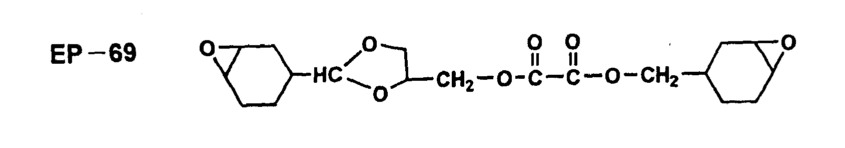

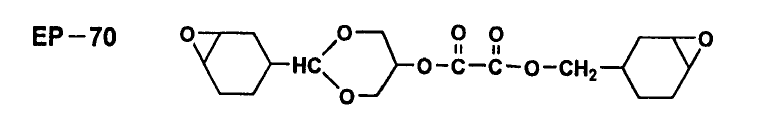

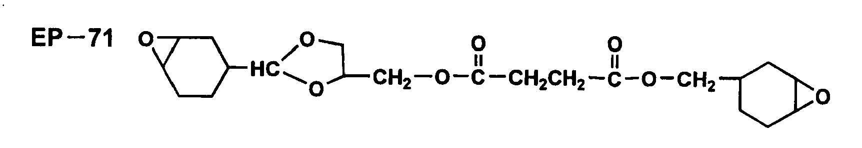

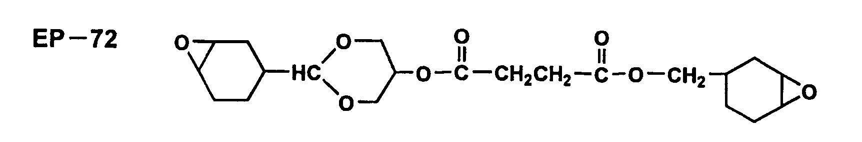

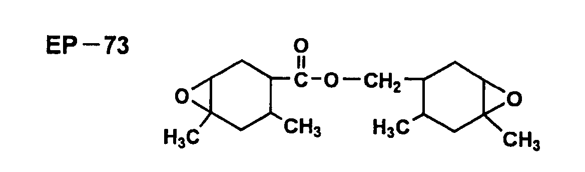

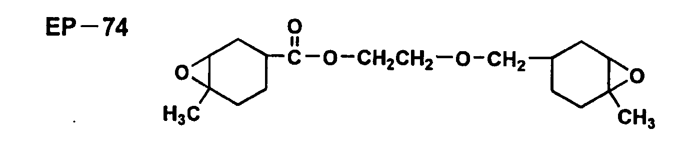

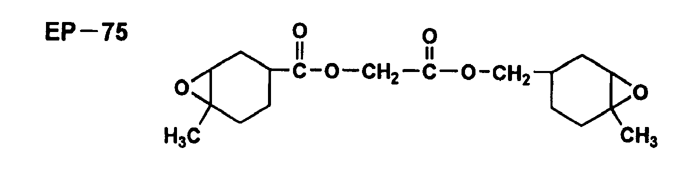

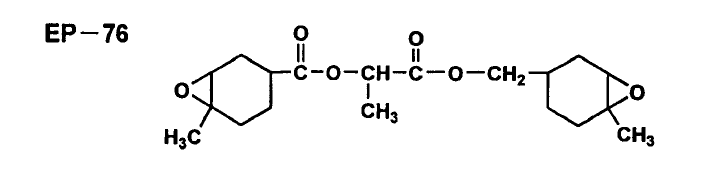

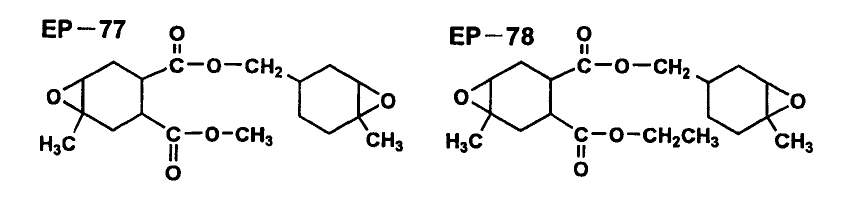

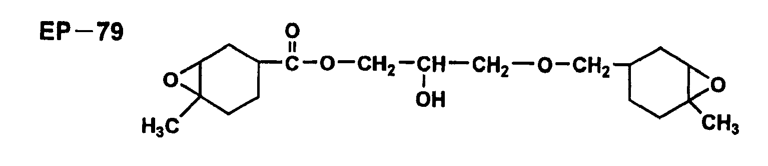

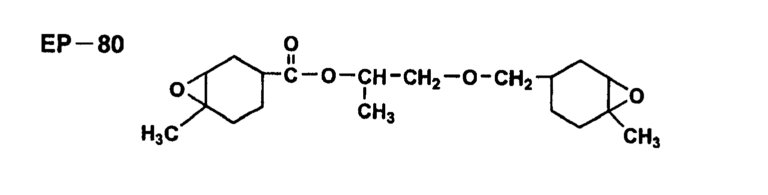

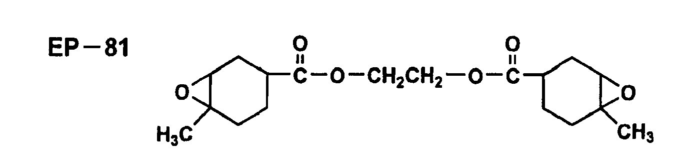

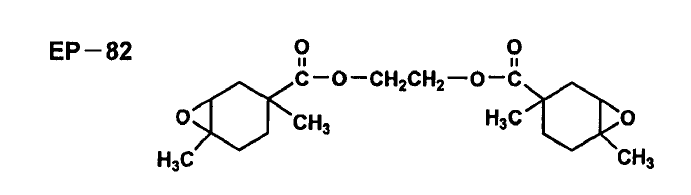

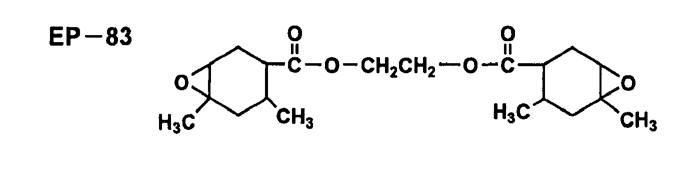

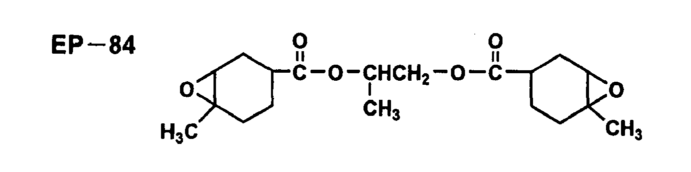

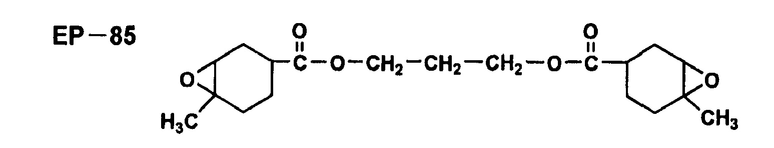

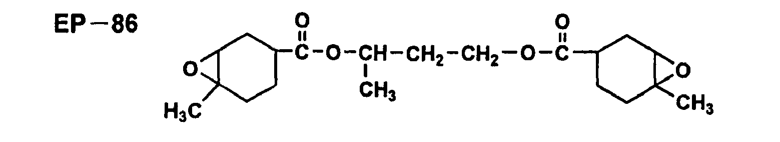

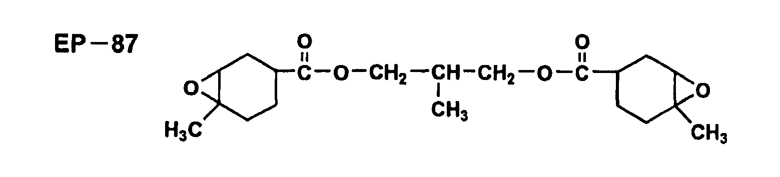

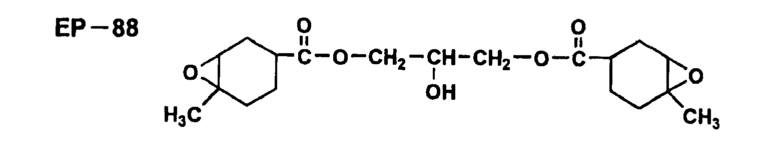

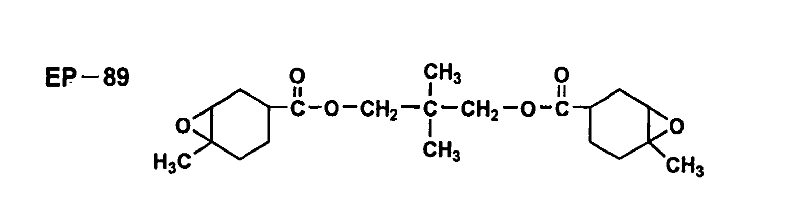

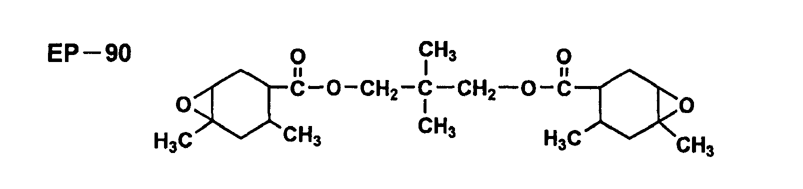

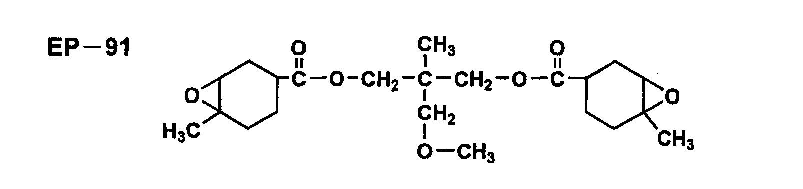

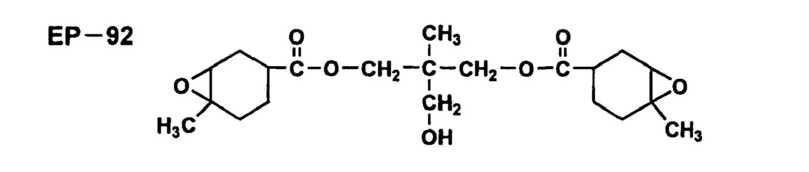

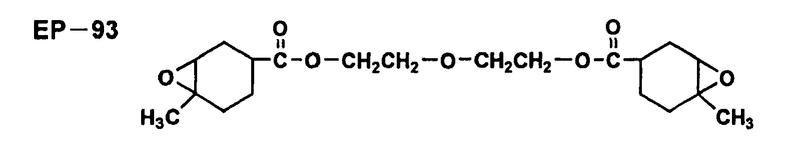

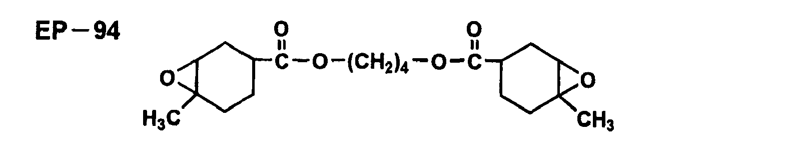

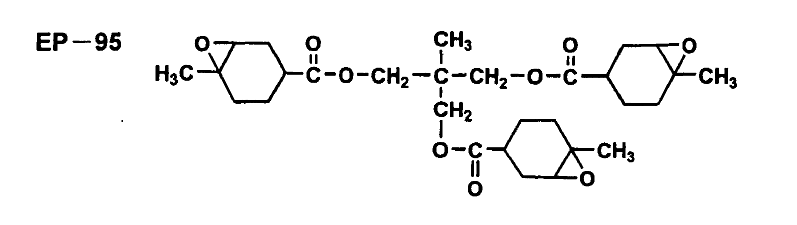

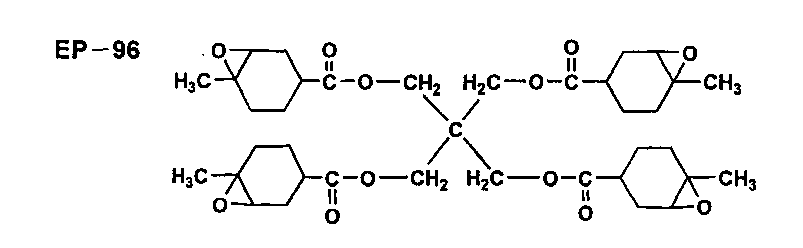

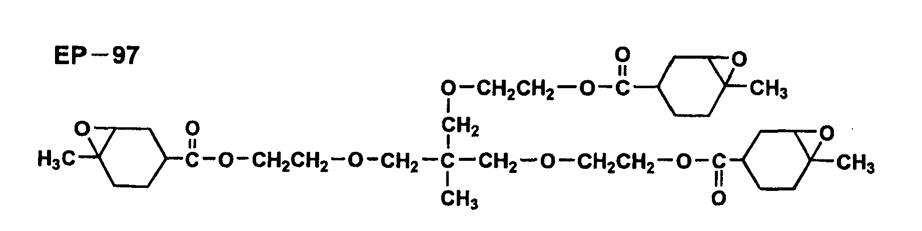

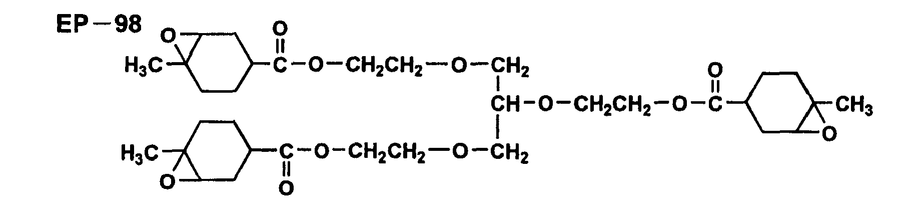

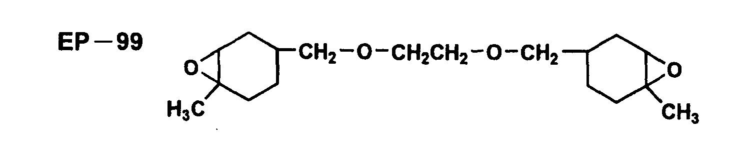

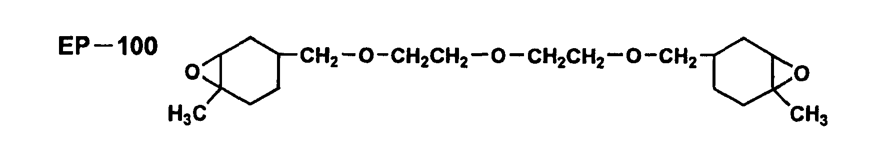

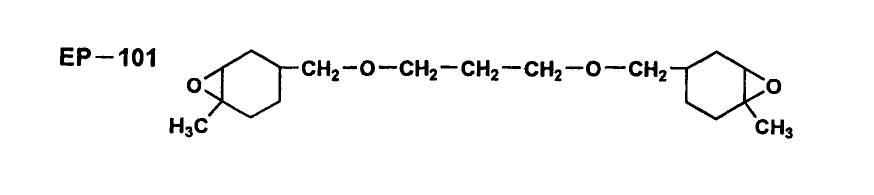

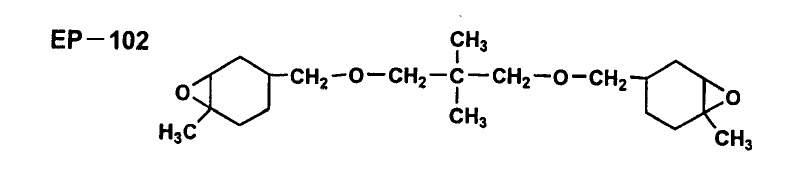

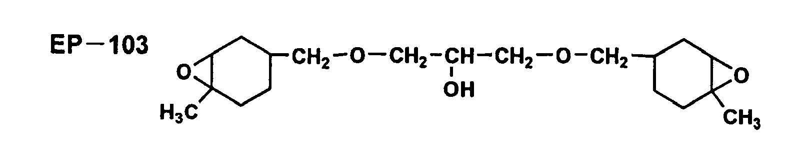

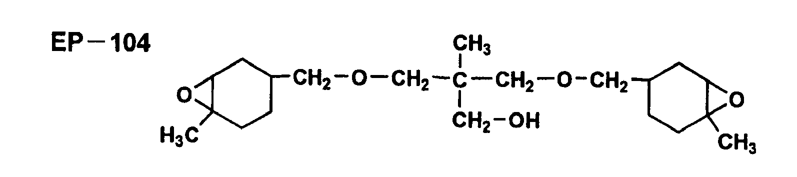

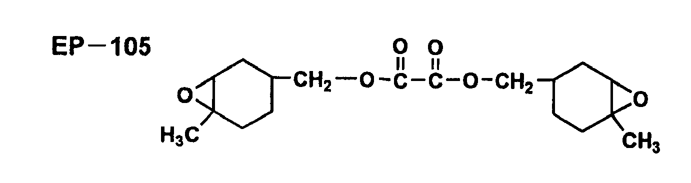

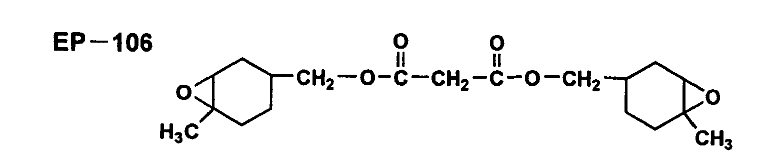

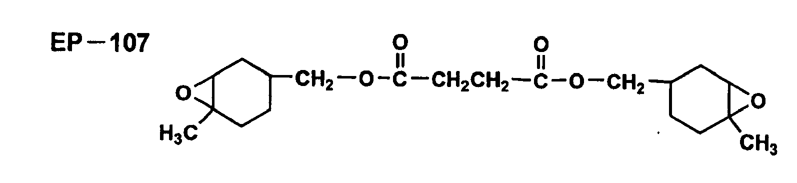

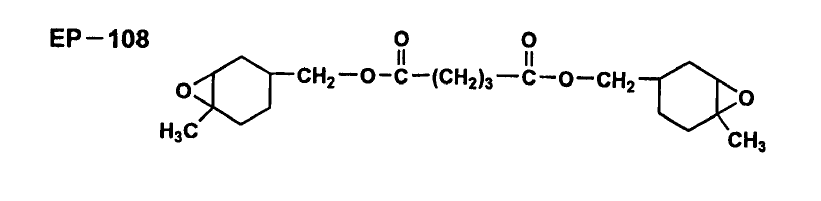

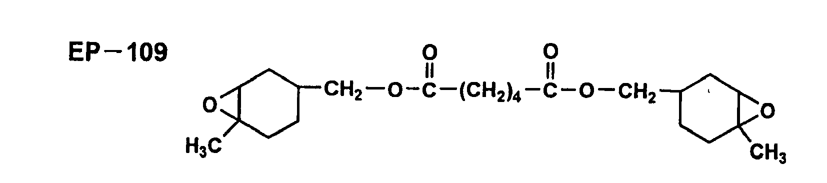

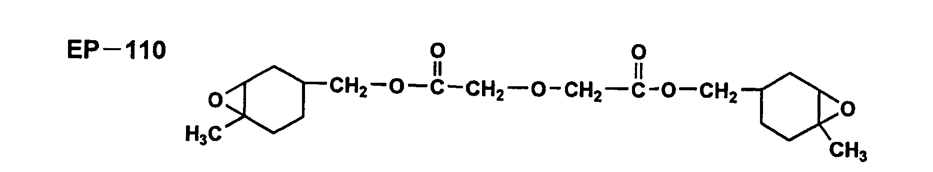

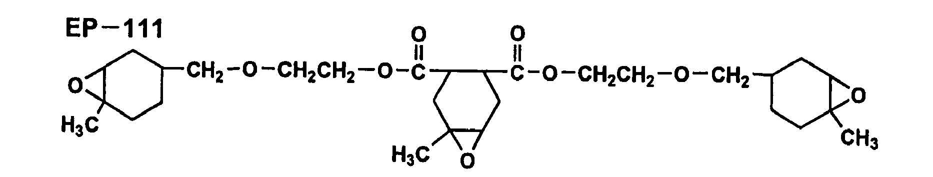

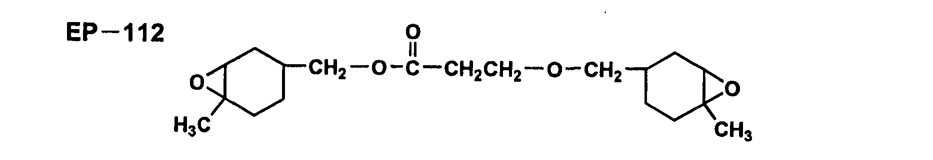

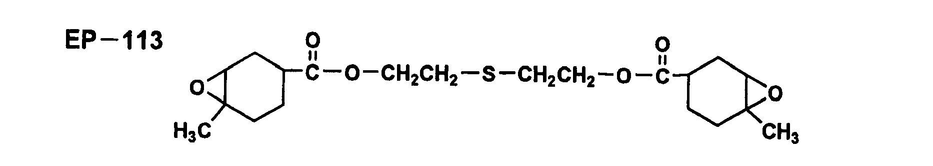

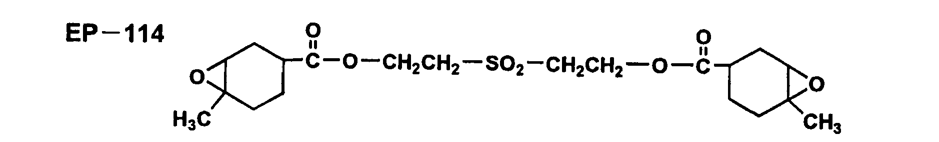

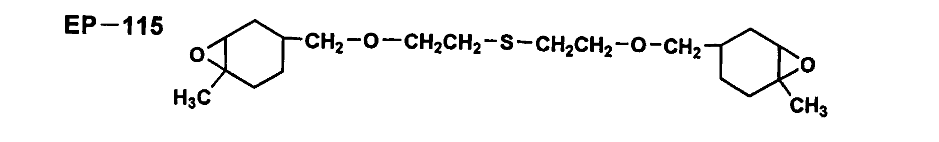

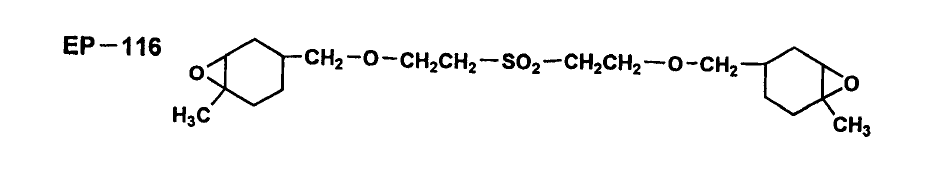

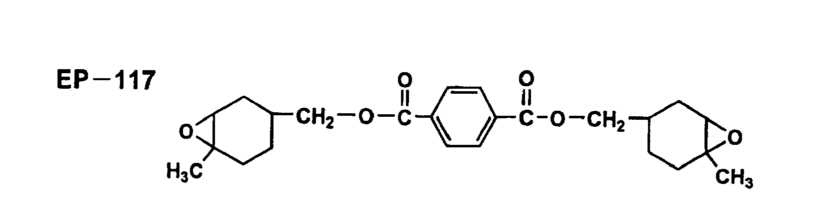

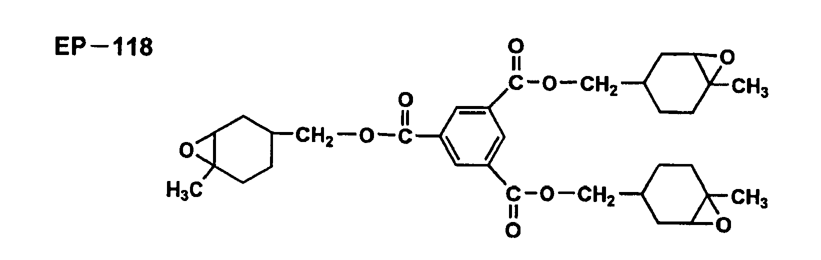

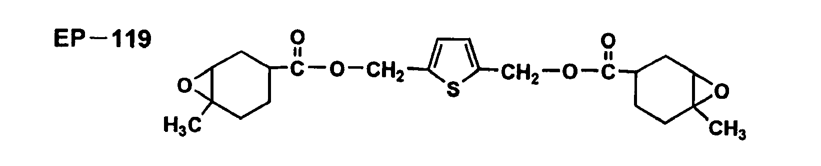

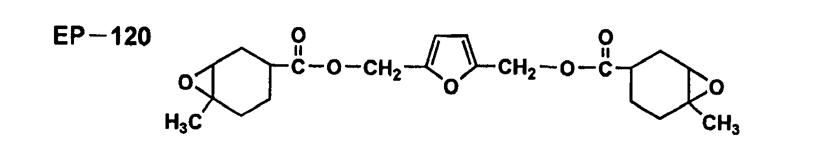

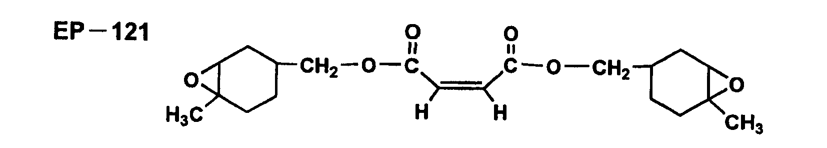

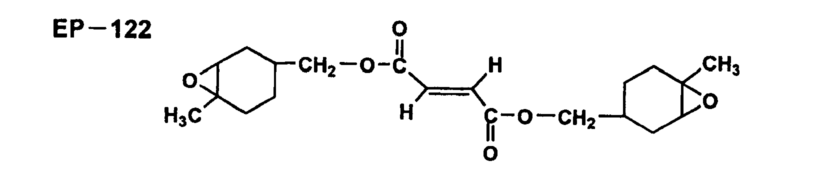

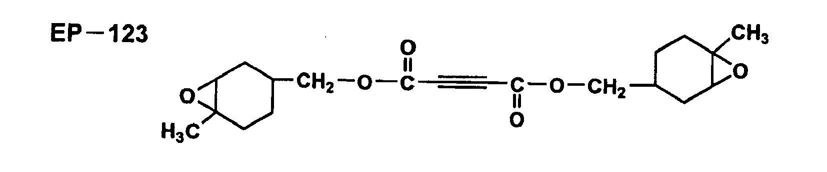

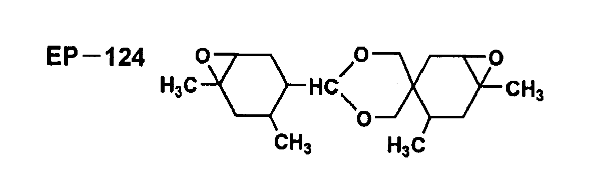

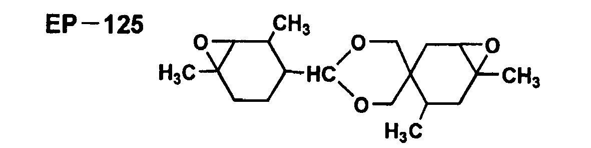

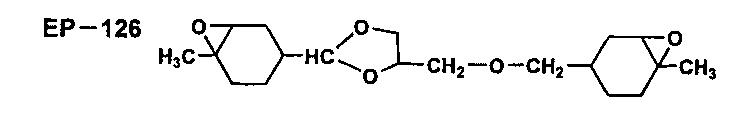

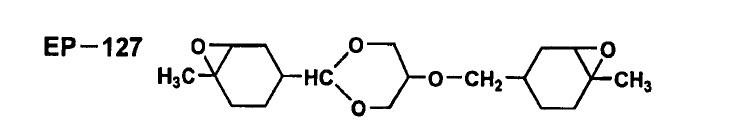

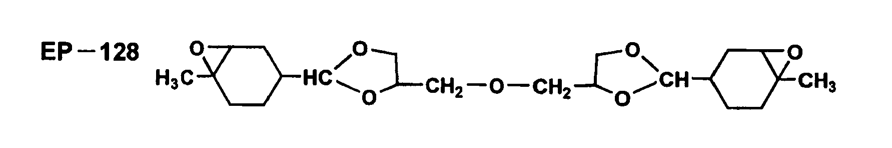

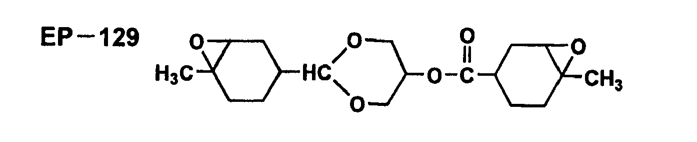

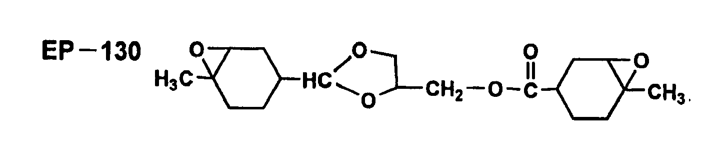

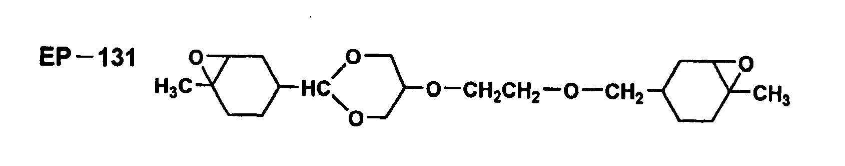

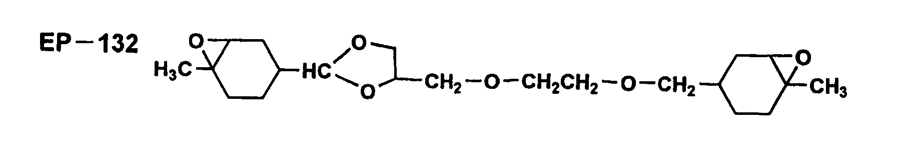

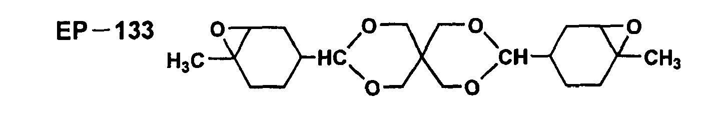

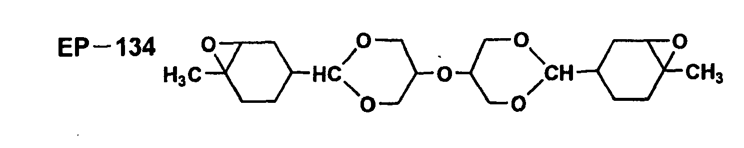

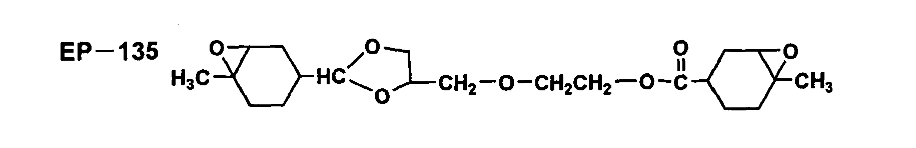

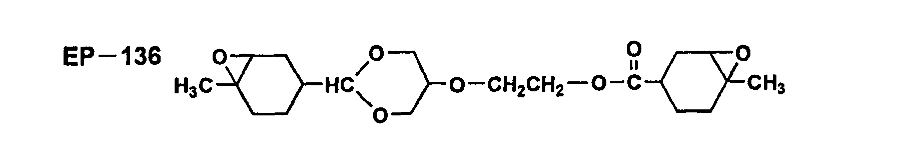

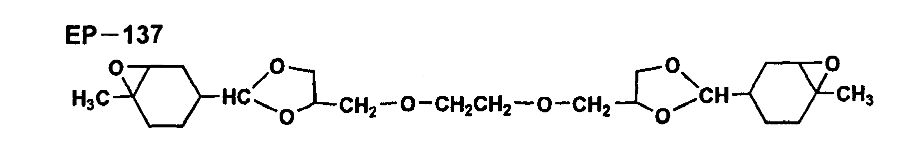

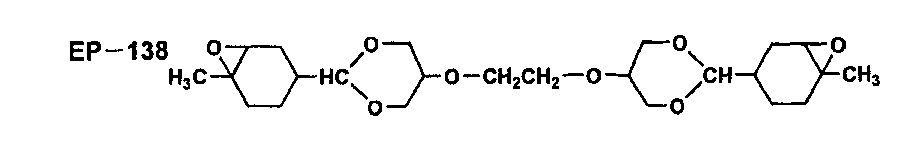

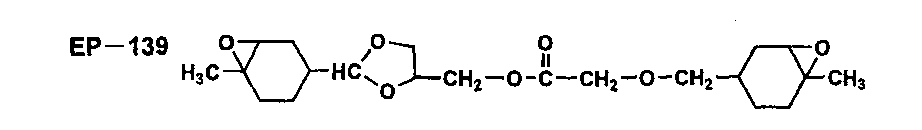

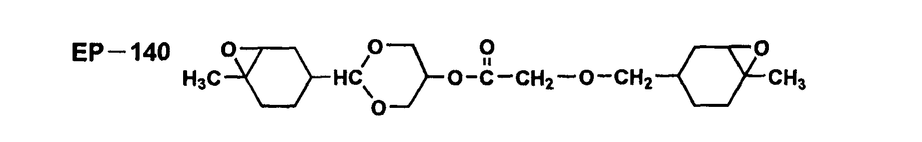

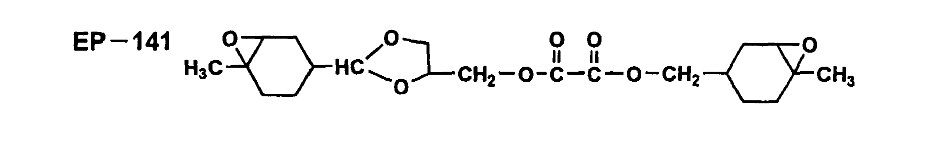

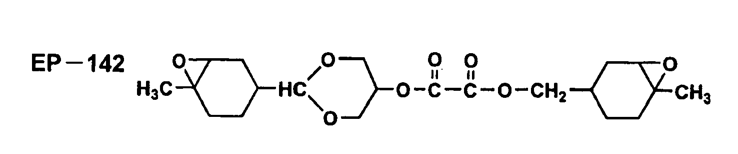

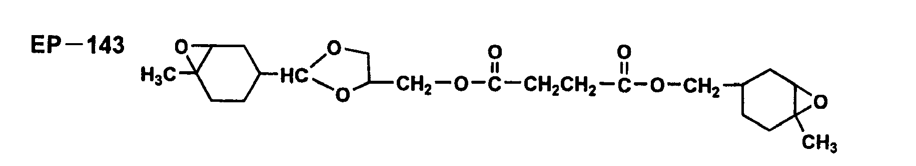

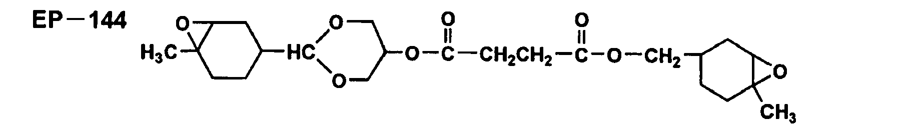

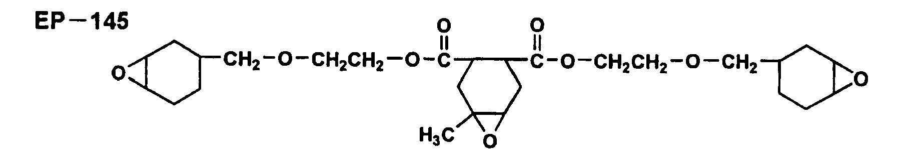

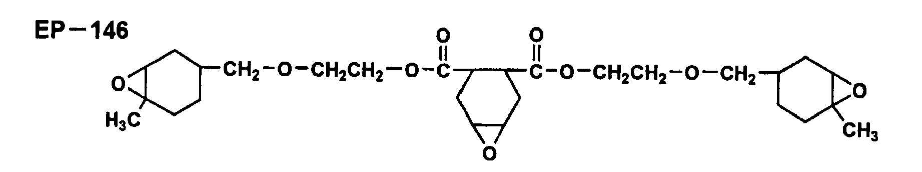

An addition amount of an epoxy comound is preferably 10 - 40 weight%. When it is less than 10 weight%, curing capability may be changed depending on curing environment (temperature, humidity) to make the compound unusable. When it is over 50 weight%, film physical properties are weak after curing to make the compound unusable. It is more preferable to incorporate the compound at 20 - 40 weight%. In this invention, one type of a poly-functional epoxy compound may be utilized alone; however, not less than two types may be also utilized in appropriate combination.

These alicyclic epoxy compounds, although the manufacturing method is not specifically limited, can be synthesized referring to, for example, the forth edition, Experimental Chemistry Course 20, Organic Synthesis II, p. 213 -, published by Maruzen KK Shuppan (1992); "The Chemistry of Heterocyclic Compounds-Small Ring Heterocycles part 3, Oxiranes", edited by Alfred Hasfner, John & Willey and Sons, An Interscience Publication, New York (1985); Yoshimura, Adhesion vol. 29, No. 12, 32 (1985), Yoshimura, Adhesion vol. 30, No. 5, 42 (1986), Yoshimura, Adhesion vol. 30, No. 7, 42 (1986), JP-A Nos. 11-100378, 4-36263 and 4-69360.

As a photo-induced acid generator utilized in cationic polymerizing ink employing an actinic ray curable composition of this invention, a compound utilized in chemical amplification type photoresist and photo-cationic polymerization is employed (refer to "Organic Materials for Imaging", pp. 187 - 192, Bunshin Shuppan (1993)). Compounds preferably utilized in this invention are listed bellow.

Firstly, listed are salt of B(C₆F₅)₄⁻, PF₆⁻, AsF₆⁻ and SbF₆⁻; and sulfonate such as p-CH₃C₆H₄SO₃⁻ and p-CF₃SO₃⁻; of aromatic onium compounds such as diazonium, ammonium, iodonium, sulfonium and phosphonium. Counter anions are preferably those having a borate compound and PF₆⁻ salt because of a high generation capability of acid. Specific examples of an onium compound will be shown below.

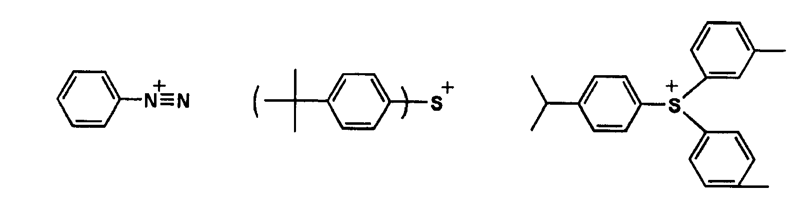

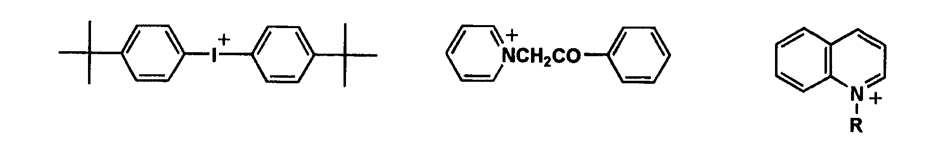

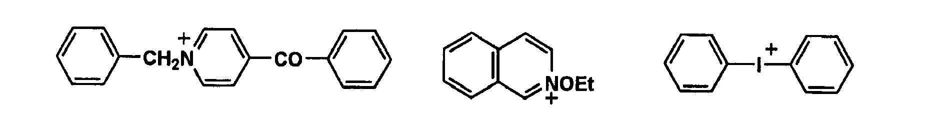

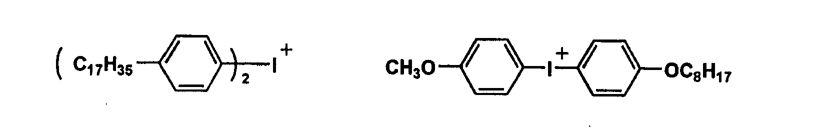

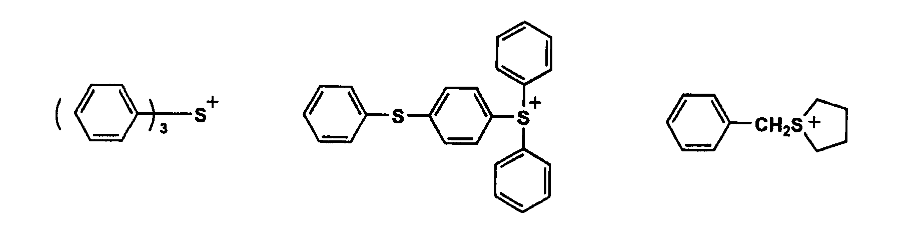

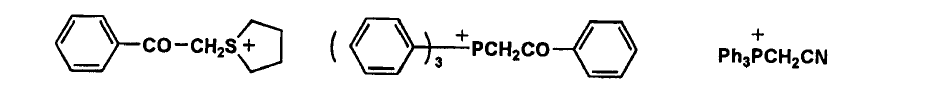

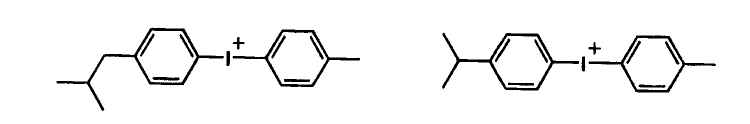

Secondly, listed are sulfonated compounds which generate sulfonic acid. Specific compounds will be exemplified below.

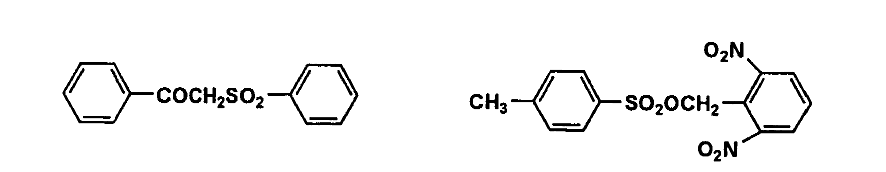

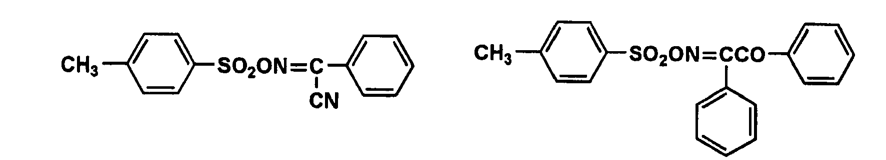

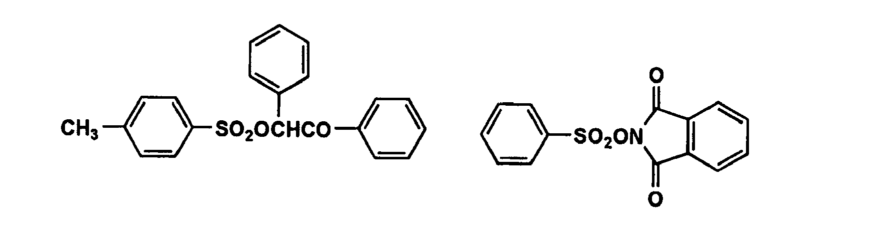

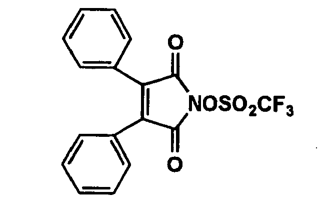

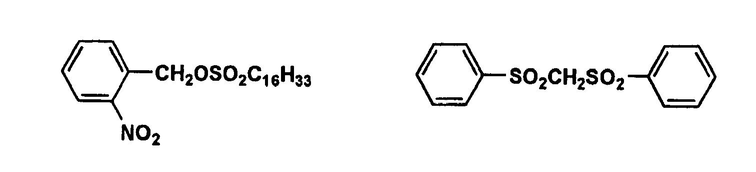

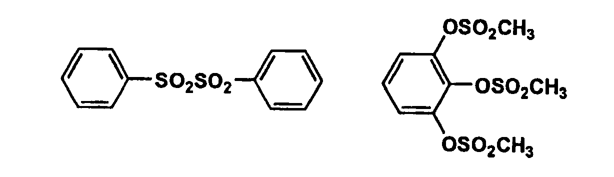

Thirdly, halogenides which generate hydrogen halogenide can be also utilized. Specific compounds will be exemplified below.

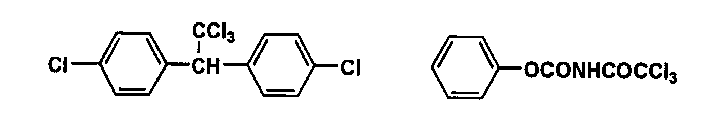

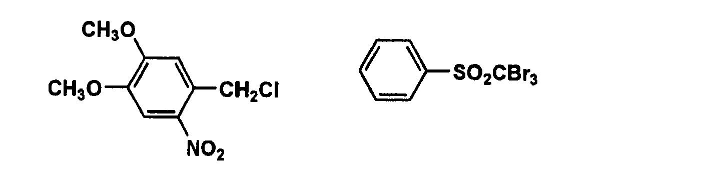

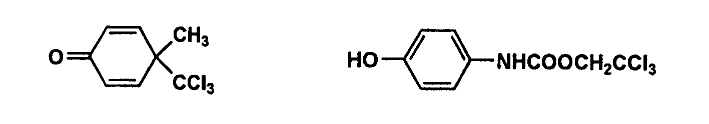

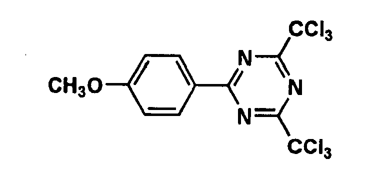

Fourthly, listed are iron allene complexes.

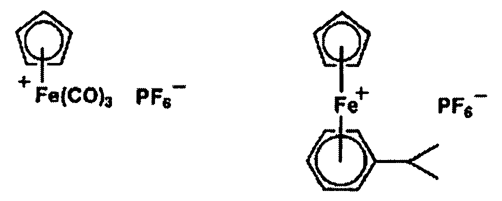

A cationic photo-polymerization initiator utilized in this invention includes a photo-induced acid generator such as aryl sulfonium salt derivatives (such as Silacure UVI-6990 and Silacure UVI-6974 manufactured by Union Carbide Corp.; Adekaoptomer SP-150, Adekaoptomer SP-152, Adekaoptomer SP-170 and Adekaoptomer SP-172 manufactured by Asahi Denka Co., Ltd.), allyl iodonium salt derivatives (such as RP-2074, manufactured by Rohdia Corp.), allene-ion complex derivatives (such as Irgacure 261, manufactured by Ciba Geigy Corp.), diazonium salt derivatives, triazine type initiators and other halogenides. A cationic photo-polymerization initiator is preferably incorporated at a ratio of 0.2 - 20 weight parts against 100 weight parts of a compound having cationic polymerizing capability. It is difficult to prepare a cured product when the content of photo-polymerization initiator is less than 0.2 weight parts, while there is no further improving effect of curing when the amount is over 20 weight parts. These photo-cationic polymerization initiators may be utilized by selecting one type or not less than two types.

A photo-induced acid generator utilized in this invention is preferably onium salt such as sulfonium salt, iodonium salt, ammonium salt and phosphonium salt, and among them preferable is a sulfonium salt compound. A structure of a more preferable sulfonium salt compound includes sulfonium compounds represented by aforesaid Formula (I-1), (I-2) or (I-3) .

In Formula (I-1) , R₁₁, R₁₂ and R₁₃ are a substituent. Examples of the substituent include a halogen atom (such as a chlorine atom, a bromine atom and a fluorine atom), an alkyl group having a carbon number of 1 - 6 (such as a methyl group, an ethyl group, a propyl group, an isopropyl group and a butyl group), a cycloalkyl group having a carbon number of 3 - 6 (such as a cyclopropyl group, a cyclobutyl group, a cyclopentyl group and a cyclohexyl group), an alkenyl group having a carbon number of 1 - 6 (such as a vinyl group, a 1-propenyl group, a 2-propenyl group and a 2-butenyl group), an alkynyl group having a carbon number of 1 - 6 (such as an acetylenyl group, a 1-propinyl group, a 2-propinyl group and a 2-butynyl group), an alkoxy group having a carbon number of 1 - 6 (such as a methoxy group, an ethoxy group, a n-propoxy group, an iso-propoxy group, an n-butoxy group and a tert-butoxy group), an alkylthio group having a carbon number of 1 - 6 (such as a methylthio group, an ethylthio group, an n-propylthio group, an iso-propylthio group, an n-butylthio group and a tert-butylthio group), an aryl group having a carbon number of 6 - 14 (such as a phenyl group, a naphthyl group and an anthracenyl group), an aryloxy group having a carbon number of 6 - 10 (such as an phenoxy group and a naphthoxy group), an arylthio group having a carbon number of 6 - 10 (such as a phenylthio group and a naphthylthio group), an acyl group (such as an acetyl group, a propionyl group, a trifloroacetyl group and a benzoyl group), an acyloxy group (such as an acetoxy group, a propionyloxy group, a trifluoroacetoxy group and a benzoyloxy group), an alkoxycarbonyl group (such as a methoxycarbonyl group, an ethoxycarbonyl group and a tert-butoxycarbonyl group), a hetero atom containing aromatic ring group having a carbon number 4 - 8 (such as a furyl group and a thienyl group), a nitro group and a cyano group. Preferable substituents are a halogen atom, an alkyl group, an alkoxyl group, an aryl group, an aryloxy group, an arylthio group and an acyl group. Among these substituents, those which can be substituted may be further substituted.

m, n and p are an integer of 0 - 2 and are each preferably not less than 1.

X₁₁⁻ is a counter ion. A counter ion includes a complex ion such as BF₄⁻, B (C₆F₆)₄⁻, PF₆⁻, AsF₆⁻ and SbF₆⁻; and a sulfonate ion such as p-CH₃C₆H₄SO₃⁻ and CF₃SO₃⁻. As a counter ion, a borate ion and PF₆⁻ are preferable with respect to high acid generation capability.

In Formula (I -2), R₁₄ is identical to R₁₁, R₁₂ and R₁₃· q is an integer of 0 - 2, preferably not less than 1, and more preferably 2.

Further, R₁₅ and R₁₆ are a substituted or unsubstituted alkyl group, a substituted or unsubstituted alkenyl group, a substituted or unsubstituted alkynyl group or a substituted or unsubstituted aryl group. Examples of a substituent are a halogen atom (such as a chlorine atom, a bromine atom and a fluorine atom), an alkyl group having a carbon number of 1 - 6 (such as a methyl group, an ethyl group, a propyl group, an isopropyl group and a butyl group), a cycloalkyl group having a carbon number of 3 - 6 (such as a cyclopropyl group, a cyclobutyl group, a cyclopentyl group and a cyclohexyl group), an alkenyl group having a carbon number of 1 - 6 (such as a vinyl group, a 1-propenyl group, a 2-propenyl group and a 2-butenyl group), an alkynyl group having a carbon number of 1 - 6 (such as an acetylenyl group, a 1-propinyl group, a 2-propinyl group and a 2-butynyl group), an alkoxy group having a carbon number of 1 - 6 (such as a methoxy group, an ethoxy group, a n-propoxy group, an iso-propoxy group, an n-butoxy group and a tert-butoxy group), an alkylthio group having a carbon number of 1 - 6 (such as a methylthio group, an ethylthio group, an n-propylthio group, an iso-propylthio group, an n-butylthio group and a tert-butylthio group), an aryl group having a carbon number of 6 - 14 (such as a phenyl group, a naphthyl group and an anthracenyl group), an aryloxy group having a carbon number of 6 - 10 (such as an phenoxy group and a naphthoxy group), an arylthio group having a carbon number of 6 - 10 (such as a phenylthio group and a naphthylthio group), an acyl group (such as an acetyl group, a propionyl group, a trifloroacetyl group and a benzoyl group), an acyloxy group (such as an acetoxy group, a propionyloxy group, a trifluoroacetoxy group and a benzoyloxy group), an alkoxycarbonyl group (such as a methoxycarbonyl group, an ethoxycarbonyl group and a tert-butoxycarbonyl group), a hetero atom containing aromatic ring group having a carbon number 4 - 8 (such as a furyl group and a thienyl group), a nitro group and a cyano group. Preferable substituents are a halogen atom, an alkyl group, an alkoxy group, an aryloxy group and an acyl group.

X₁₂⁻ is identical to X₁₁⁻.

In Formula (I-3) , R₁₇ is identical with R₁₁, R₁₂ and R₁₃. r is an integer of 0 - 3, preferably not less than 1 and more preferably 2. R₁₈ is a hydrogen atom or a substituted or unsubstituted alkyl group; R₁₉ and R₂₀ are identical with R₁₅ and R₁₆.

X₁₃ is identical to X₁₁⁻·

In the following, specific examples of a sulfonium salt compound represented by Formula (I-1), (I-2) or (I-3) will be shown; however, this invention is not limited thereto.

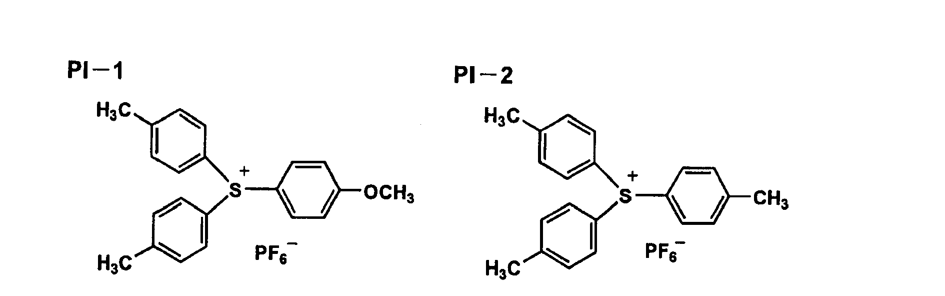

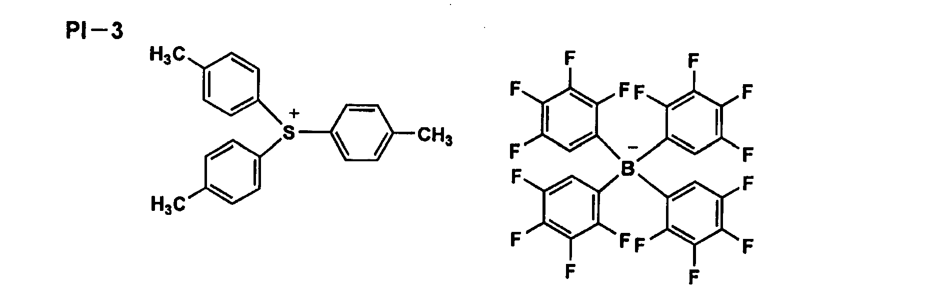

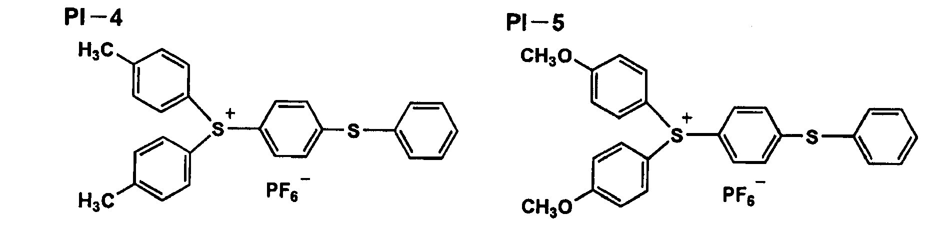

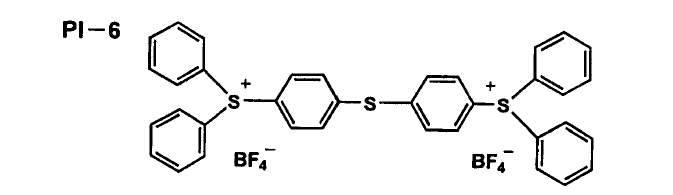

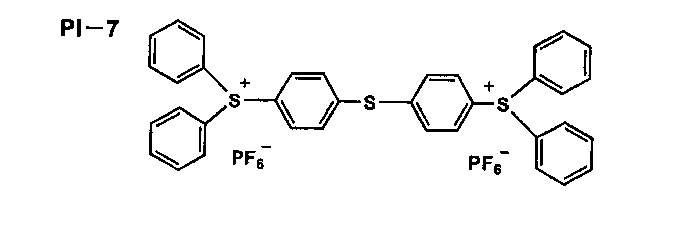

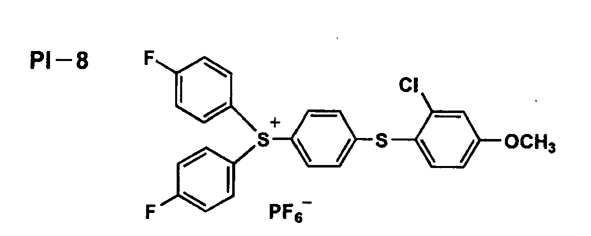

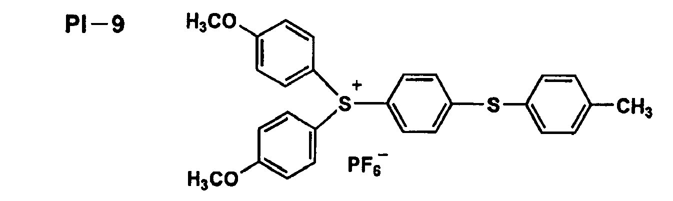

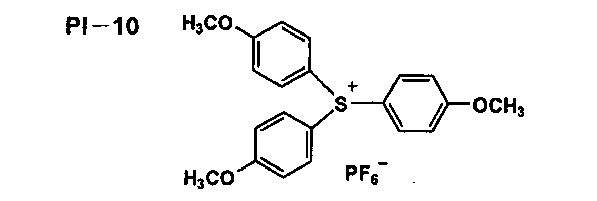

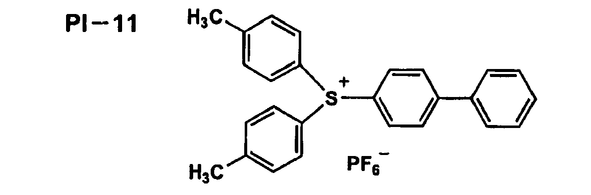

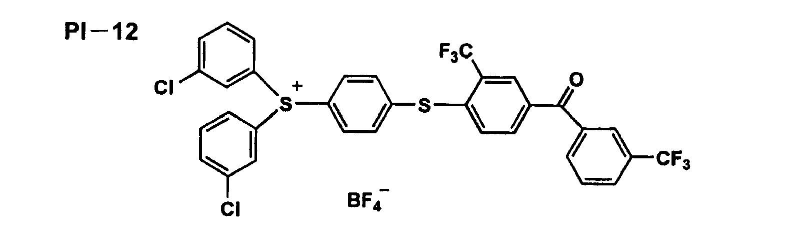

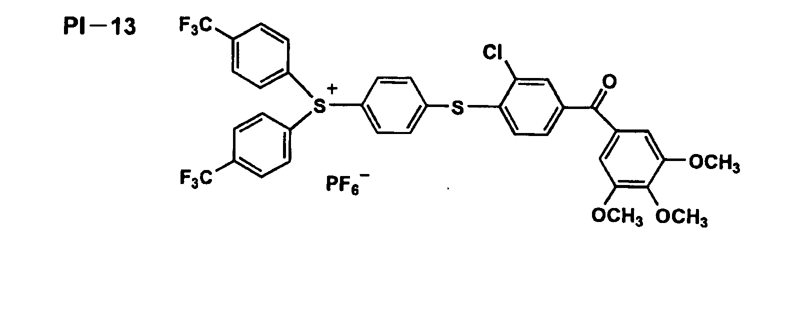

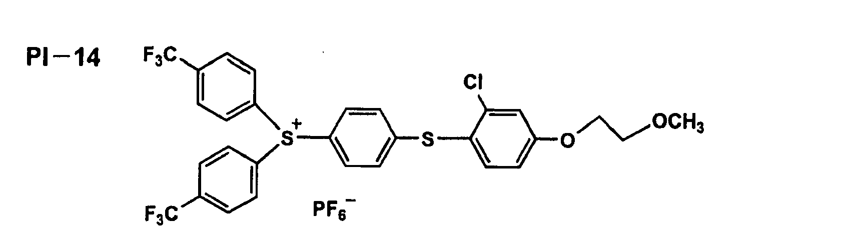

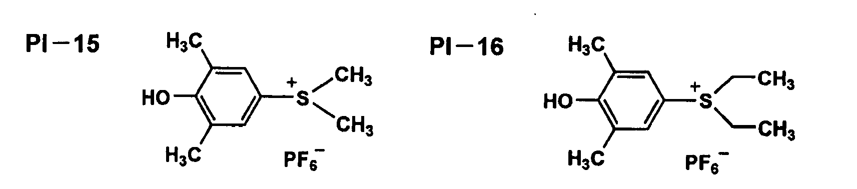

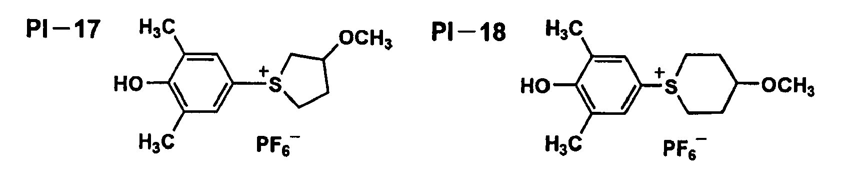

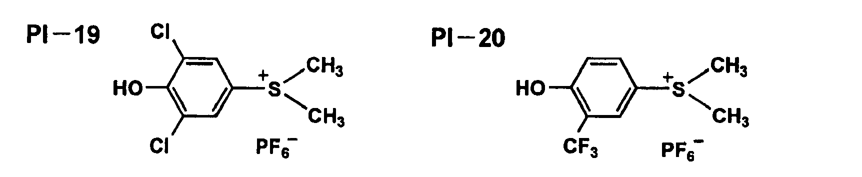

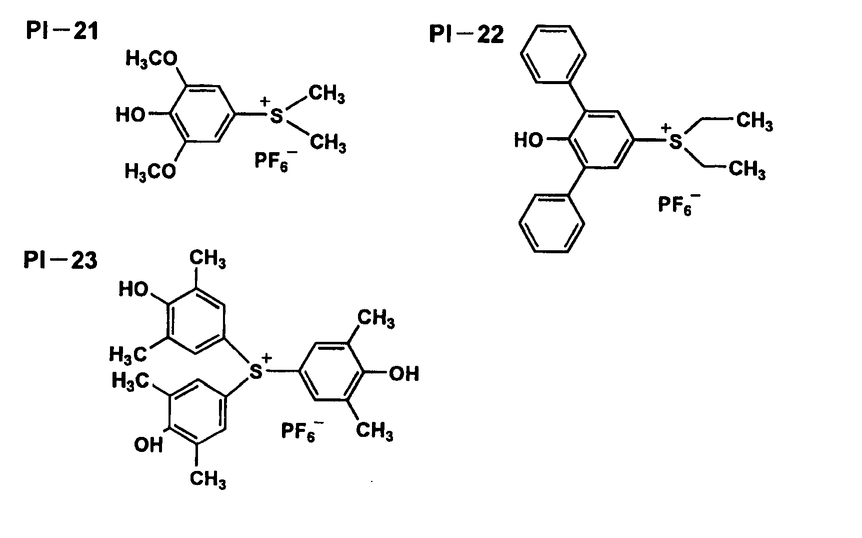

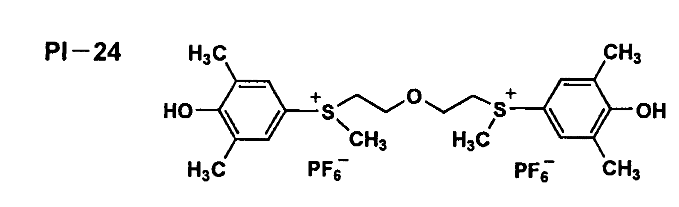

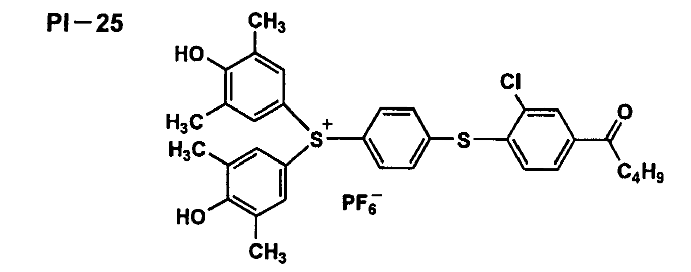

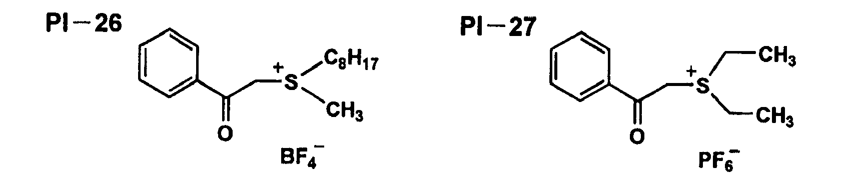

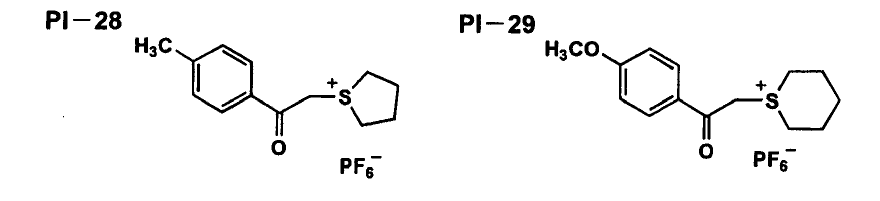

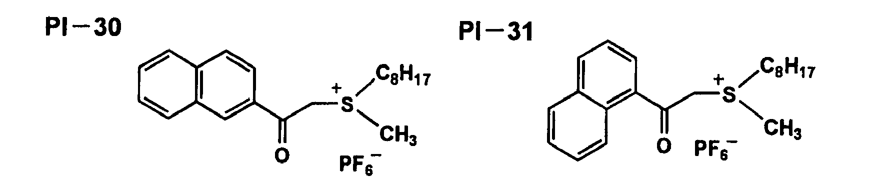

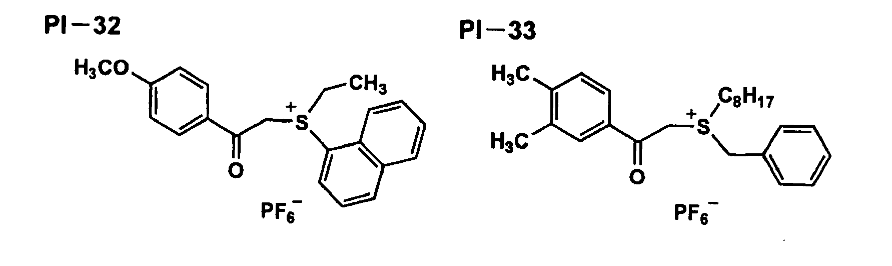

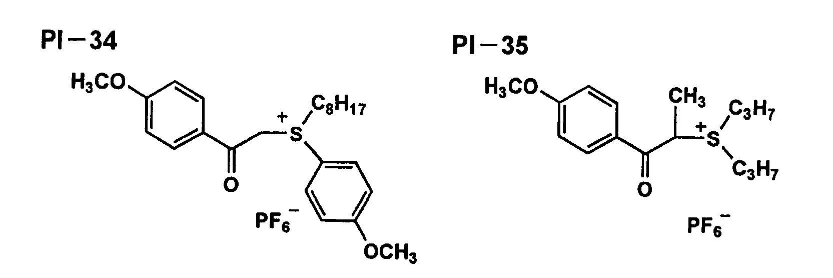

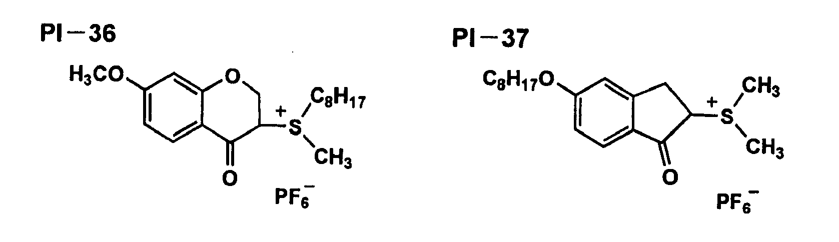

A photo-polymerization accelerator includes anthracene, anthracene derivatives (such as Adekaoptomer SP-100, manufactured by Asahi Denka Kogyo K.K), phenothiazine (10H-phenothiazine) and phenothiazine derivatives (such as 10-methylphenothiazine, 10-ethylphenothiazine, 10-decylphenothiazine, 10-acetylphenothiazine, 10-decylphenothiazine-5-oxide, 10-decylphenothiazine-5, 5-dioxide and 10-acetylphenothiazine-5,5-dioxide). One type or combination of plural types, of these photo-polymerization accelerators, can be utilized.

In an actinic ray curable composition of this invention, various types of additives other than constituent elements explained above can be utilized.

As a colorant utilized in actinic ray curable ink employing an actinic ray curable composition of this invention and in ink-jet ink (hereinafter, also described as "ink-jet ink of this invention") employing actinic ray curable ink of this invention, a colorant capable of being dissolved or dispersed in the primary component of a photo polymerizable compound can be utilized, however, pigment is preferred with respect to weather-proofing.

Preferable pigment utilized in this invention will be listed below.

### C. I. Pigment Yellow-1, 2, 3, ---

### C. I. Pigment Black-7

For the purpose of dispersion of the above pigment, such as a ball mill, a sand mill, an atliter, a roll mill, an agitator, a Henschel mixer, a colloidal mill, an ultrasonic homogenizer, a pearl mill, a wet jet mill and a paint shaker can be utilized. Further, it is also possible to incorporate a dispersant at the time of dispersing pigment. As a dispersant, preferably utilized is a polymer dispersant which includes Solsperse series by Avecia Corp. Further, a synergist corresponding to various types of pigment as a dispersion aid can be also utilized. These dispersant and dispersion aid are preferably added at 1 - 50 weight parts against 100 weight parts of pigment. A solvent or a polymerizing compound is utilized as a dispersion medium, however, in an actinic ray curable ink utilized in this invention, solvent-less is preferred since reaction and curing are performed immediately after ink landing. When a solvent remains in a cured image, there caused problems of deterioration of solvent resistance and VOC problem of a residual solvent. Therefore, a dispersion medium is preferably not a solvent but a photo polymerizable compound, and monomer having the lowest viscosity among them is more preferably selected with respect to dispersion adaptability.

For dispersion of pigment, it is preferable to make the particle size of pigment particles of 0.08 - 0.5 µm, and selection of pigment, a dispersant and a dispersion medium; dispersion condition, and filtration condition are appropriately set so as to make the maximum particle size of 0.3 - 10 µm and preferably of 0.3 - 3 µm. By this particle size control, clogging of a head nozzle is depressed and storage stability, transparency and curing sensitivity of ink can be maintained.

In ink-jet ink of this invention, pigment concentration is preferably 1 - 10 weigh% against the whole ink.

In this invention, a heat-induced base generator can be also utilized to improve ejection stability and storage stability.

As heat-induced base generator, for example, salt of organic acid, which decomposes by decarboxylation with heat, and base; a compound which release amines by decomposition via a reaction such as intramolecular neucleophilic susbstitution, Lossen rearrangement and Beckman's rearrangement, and those releasing base by causing some reaction with heat can be preferably utilized. Specifically, listed are salt of trichloroacetic acid described in British Patent No. 998,949, salt of α-sulfonylacetic acid described in USP No. 4,060,420, aldoxime carbamate derivatives and 2-carboxycarboxyamide derivatives described in JP-A 59-157637, salt utilizing alkali metal or alkaline earth metal other than organic base as a base component with thermal decomposing acid described in JP-A 59-168440, hydroxame carbamates utilizing Lossen rearrangement described in JP-A 59-180537, and aldoxime carbamates which generate nitrile with heat described in JP-A 59-195237. In addition to these, useful are heat-induced base generators described in British Patent No. 998,945, USP 3,220,846, British Patent No. 279,480, JP-A Nos. 50-22625, 61-32844, 61-51139, 61-52638, 61-51140, 61-53634, 61-53640, 61-55644 and 61-55645.

Further specific examples include guanidine trichloroacetate guanidine, methylguanidine trichloroacetate, potassium trichloroacetate, guanidine phenylsulfonylacetate, guanidine p-chlorophenylsulfonylacetate, guanidine p-methanesulfonylphenylsulfonylacetate, potassium phenylpropiolate, guanidine phenylpropiolate, cesium phenylpropiolate, guanidine p-chlorophenylpropiolate, guanidine p-phenylene-bisphenylpropiolate, tetramethylammonium phenylsulfonylacetate and tetramethylammonium phenylpropiolate. The above-described heat-induced base generators can be utilized in a wide range.

Ink-jet ink of this invention can also contain an acid multiplier, which newly generates acid by acid generated by actinic ray irradiation, well known in the art according to such as JP-A Nos. 8-248561 and 9-34106.

Ink-jet ink of this invention is manufactured by sufficiently dispersing pigment together with an actinic ray curable composition and a pigment dispersant by use of a homogenizer such as a sand mill. It is preferable to dilute a concentrated solution of pigment having a high concentration, which has been prepared in advance, with an actinic ray curable compound. Sufficient dispersion is possible by dispersion employing an ordinary homogenizer, and therefore, since no excess dispersion energy is applied nor plenty of dispersion time is required, alternation of ink component at the time of dispersion is hardly caused and ink having superior stability is prepared. Ink is preferably filtered through a filter having a pore diameter of not more than 3 µm and more preferably of not more than 1 µm.

An actinic ray curable composition of this invention is preferably adjusted to have a viscosity at 25 °C of 1 - 500 mPa·s. It is more preferably adjusted to 1 - 300 mPa·s and furthermore preferably adjusted to 1 - 150 mPa·s, with respect to utilizing various characteristics of an actinic ray curable composition having a low viscosity.

To adjust viscosity at 25 °C to less than 1 mPa·s, it is possible to dilute utilizing appropriate quantity of an organic solvent having a low viscosity, however, this case is not preferable because of fear for decrease of curing capability due to decrease of density of a polymerizing group in a curing composition in addition to problems will be caused with respect to odor and generation of a volatile compound. Further, in the case of a viscosity of not less than 500 mPa·s, it is not preferable because fear for limitation of the addition amount of a solid component such as filler, inorganic micro-particles and pigment.

Further, ink-jet ink of this invention is preferably prepared as ink which has a conductivity of not higher than 10 µS/cm and does not cause electrical corrosion at the interior of a head. Further, in a continuous type, the conductivity is necessarily adjusted by electrolyte, and in this case, the conductivity should be adjusted to not lower than 0.5 mS/cm.

In this invention, surface tension of ink at 25 °C is preferably in a range of 25 - 40 mN/m. In the case of surface tension of ink being less than 25 mN/m, stable ejection is hardly realized, while in the case of over 40 mN/m, an aimed dot diameter can not be achieved. Out of a range of 25 - 40 mN/m, it becomes difficult to obtain a uniform dot diameter against various supports even with light irradiation while controlling viscosity and water content of ink as described in this invention.

A surfactant may be appropriately incorporated to adjust the surface tension. A surfactant preferably utilized in ink-jet ink of this invention includes anionic surfactants such as dialkylsulfosuccinates, alkylnaphthalenesulfonates and fatty acid salts; nonionic surfactants such as polyoxyethylene alkyl ethers, polyoxyethylene alkylallyl ethers, acetylene glycols and polyoxyethylene-polyoxypropylene block copolymers; cationic surfactants such as alkylamines and quaternary ammonium salts; and surface active compounds having a polymerizing group. Among them, specifically preferred are surface active compounds having a polymerizing group such as an unsaturated bond and an oxirane or oxetane ring, such as silicone modified acrylate, fluorine modified acrylate, silicone modified epoxy, fluorine modified epoxy, silicone modified oxetane and fluorine modified oxetane.

In ink-jet ink of this invention, various additives other than those explained above can be utilized. For example, added can be a leveling agent, a matting agent; polyester type resin, polyurethane type resin, vinyl type resin, acrylic type resin, rubber type resin and waxes to adjust film physical properties. Addition of a slight amount of an organic solvent is also effective to improve adhesion with a recording medium. In this case, addition in a range of not causing problems of solvent resistance and VOC is effective, and the using amount is in a range of 0.1 - 5% and preferably of 0.1 - 3%. Further, by combining with radical polymerizing monomer and an initiator, it is also possible to make radical·cation hybrid type curable ink.

In an image forming method of this invention, an ink composition is ejected and drawn on a recording material by an ink-jet recording method and successively the ink is cured by irradiation of actinic rays such as ultraviolet rays.

In an image forming method of this invention, it is preferable to heat ink together with an ink-jet nozzle to make ink liquid be provided with a low viscosity. The heating temperature is 35 - 100 °C, preferably 35 - 80 °C and more preferably 35 - 60 °C.

In this invention, the total ink layer thickness after ink has landed and has been cured by irradiation of actinic rays is preferably 2 - 20 µm. In actinic ray curable ink-jet recording in a screen printing field, it is the present stat that the total ink layer thickness is over 20 µm, however, in a soft package printing field, in which a recording material is often a thin plastic material, this thickness is unusable because of problems that stiffness and quality of sensation will be changed in addition to curl and wrinkles of a recording material described before. Further, in this invention, liquid drop quantity ejected from each nozzle is preferably 2 - 15 pl.

In this invention, to form an image of a high precision, it is the better that the irradiation timing is the faster; however, it is preferable, in this invention, to start light irradiation at the timing that the viscosity or water content of ink becomes a preferable state.

In detail, as an irradiation condition of actinic rays, to start actinic ray irradiation within 0.001 - 1.0 second after ink landing is preferable and more preferably within 0.001 - 0.4 seconds. Further, it is preferable to finish irradiation after light irradiation of an extent to lose ink fluidity, in 0.1 - 3 seconds and preferably 0.2 - 1 second. By setting the above condition, it is possible to prevent enlargement of a dot diameter and bleeding between dots.

As an irradiation method of actinic rays, the basic method has been disclosed in JP-A 60-132767. According to this, light sources are provided on the both sides of a recording head and the recording head and the light sources are scanned in a shuttle mode. Irradiation is performed leaving a certain time after ink landing. Further, curing is completed by a separate light source without drive. In USP 6,145,979, disclose are, a method utilizing an optical fiber, and a method in which a collimated light source is incident into a mirror arranged on the side of a recording head unit and UV light is irradiated on a recorded portion. In an image forming method of this invention, any one of these irradiation methods can be utilized.

Further, a preferable embodiment is a method in which irradiation of actinic rays is divided into two steps to firstly irradiate actinic rays according to the aforesaid method within 0.001 - 2.0 seconds after ink landing and actinic rays are further irradiated after finishing the whole print. By dividing irradiation of actinic rays into two steps, it is possible to restrain shrinkage of a recording material which is caused at the time of ink curing.

Examples of a light source utilized for actinic ray irradiation include a mercury arc lamp, a xenon arc lamp, a fluorescent arc lamp, a carbon arc lamp, a tungsten-halogen copying lamp, a high pressure mercury lamp, a metal halide lamp, a non-electrode UV lamp, a low pressure mercury lamp, a UV laser, a xenon flush lamp, an insect catching lamp, a black light, a sterilizing lamp, a cold cathode tube and a LED, however, are not limited thereto; and among them preferable is a fluorescent lamp because of a low energy and a low cost. Wavelength of a light source is preferably in a range of 250 - 370 nm and more preferably of 270 - 320 nm based on the peak of an emission wavelength with respect to sensitivity. The illuminance is 1 - 3,000 mW/cm² and preferably 1 - 200 mW/cm². In the case of curing with electron rays, generally electron rays having energy of not higher than 300 eV are employed, however, it is also possible to employ an irradiation amount of 1 - 5 Mrad to perform instant curing.

Image printing on a recoding medium (also referred to as a substrate) is performed utilizing ink-jet ink of this invention, and as a recording medium, all the wide range of synthetic resin, which is conventionally utilized in various applications, can be employed, including such as polyester, polyvinyl chloride, polyethylene, polyurethane, polypropylene, acrylic resin, polycarbonate, polystyrene, acrylonitrile-butadiene-styrene copolymer, polyethyleneterephthalate; the thickness and form of these resin substrates are not limited at all.

As a substrate utilizable in this invention, a non-absorptive support in addition to such as ordinary non-coated paper and coated paper can be employed, however, a non-absorptive support among them is preferably utilized.

In this invention, as a non-absorptive support, various types of plastic and film thereof can be utilized, and various plastic film includes such as PET film, OPS film, OPP film, ONy film, PVC film, PE film and TAC film. In addition to these, such as polycarbonate, acrylic resin, ABS, polyacetal, PVA and rubbers can be utilized. Further, metals and glasses are also applicable. Among these recording materials, a constitution of this invention is effective in the case of forming an image on PET film, OPS film, OPP film, ONy film and PVC film, which are capable of shrinkage by heat. In these substrates, curl and deformation of film are easily caused by such as curing shrinkage of ink and heat at the time of a curing reaction, and ink film is difficult to follow shrinkage of a substrate.

The surface energy of these various types of plastic film significantly differs and there has been a problem heretofore that the dot diameter will change after ink landing depending on a recording material. A constitution of this invention includes from OPP film and OPS film having a small surface energy till PET film having a relatively large surface energy, however, a substrate is preferably provided with a wet index of 40 - 60 mN/m.

In this invention, a long length (a web) recording material is advantageously utilized, with respect to a cost of a recording material such as packaging expense and a manufacturing cost, preparation efficiency of print and adaptability to various sizes of print.

Next, ink-jet recording apparatus of this invention will be explained.

In the following, an ink-jet recording apparatus of this invention will be explained appropriately referring to drawings. Herein, a recording apparatus of the drawings is only one embodiment of an ink-jet recording apparatus of this invention and an ink-jet recording apparatus of this invention is not limited to the drawings.

Fig. 1 is a front view to show a constitution of the primary portion of a recording apparatus of this invention. Recording apparatus 1 is constituted of such as head carriage 2, recording head 3, irradiation means 4 and platen portion 5. In this recording apparatus 1, platen portion 5 is arranged under recording material P. Platen portion 5 is provided with a function to absorb ultraviolet rays and absorbs excess ultraviolet rays having passed through recording material P. As a result, an image having a high precision can be very stably reproduced.

Recording material P is guided by guide member 6 to be transferred from this side to the interior of Fig. 1 by operation of a transfer means (not shown in the drawing). A head scanning means (not shown in the drawing) scans recording head 3 held by head carriage 2 by shifting head carriage 2 back and forth along the Y direction in Fig. 1.

Head carriage 2 is arranged over recording material P to store a plural number of recording head 3, which will be described later, corresponding to the number of colors utilized for image printing on recording material P while arranging the ejection outlet downward. Head carriage 2 is arranged in a form of freely shiftable back and forth against the main body of recording apparatus 1, and is shifted back and forth along the Y direction of Fig. 1 by drive of a head scanning means.

Herein, in Fig. 1, head carriage 2 is drawn so as to store recording head 3 of yellow (Y), magenta (M), cyan (C) black (K) and white (W), however, in practical use, a number of colors of recording head 3 to be stored in head carriage 3 can be appropriately determined.

Recording head 3 ejects actinic ray curable ink-jet ink (for example, UV curable ink), which has been supplied from an ink supply means (not shown in the drawing) toward recording material P through an ejection outlet by operation of an ejection means (not shown in the drawing), a plural number of which are stored in the recording head. UV ink ejected from recording head 3 is comprised of such as a colorant, polymerizing monomer and an initiator, and provided with a capability of curing by cross-linking or polymerization of the monomer accompanied with catalytic action of the initiator by receiving irradiation of ultraviolet rays.

Recording head 3 ejects ultraviolet curable ink as ink drops on a predetermined region of recording material (a region capable of being landed) to land ink drops on said region capable of being landed as ink drops during a scan of being shifted from one end of recording material P to the other end of recording material P along the Y direction in Fig. 1 by drive of a head scanning means.

After the above-described scan is performed appropriate times to eject ultraviolet curable ink toward one of a region capable of being landed, recording material P is shifted from this side to the interior direction of Fig. 1 by a shifting means and ultraviolet ink is ejected toward the next region capable of being landed adjacent along the interior direction of Fig. 1 against the above-described region capable of being landed by recording head 3 while performing a scan by a shifting a head scanning means again.

By repeating the above-described operations to eject ultraviolet curable ink from recording head 3 synchronous with a head scanning means and a transfer means, an image comprising aggregate of ultraviolet curable ink drops is formed on recording material P.

Irradiation means 4 is constituted of an ultraviolet lamp, which emits ultraviolet rays of a specific wavelength region at stable exposure energy, and a filter, which transmits ultraviolet rays of a specific wavelength region. Herein, as an ultraviolet lamp, such as a mercury lamp, a metal halide lamp, an excimer laser, an ultraviolet laser, a cold cathode tube, a black light and a LED (being a Light Emitting Diode) are applicable, and a metal halide lamp, a cold cathode tube, a mercury lamp or a black light, of a band form, is preferable. Specifically, a cold cathode tube and a black light, which emit ultraviolet rays of a wavelength of 365 nm, are preferred because prevention of bleeding and control of a dot diameter can be efficiently performed as well as wrinkles at curing can be decreased. By employing a black light as a radiation source of irradiation means 4, irradiation means 4 to cure ultraviolet curable ink can be prepared at a low cost.

Irradiation means 4 is provided with a form approximately same as the maximum one of the region which can be set by recording apparatus 1, or larger than a region capable of being landed, among a region capable of being landed by ultraviolet curable ink ejected by recording head 3 in one time scan by drive of a head scanning means.

Irradiation means 4 is arranged by being fixed in nearly parallel to recording material P on the both sides of head carriage 2.

As described before, as a means to adjust illuminance at an ink ejection portion, the whole of recording head 3 is naturally light shielded; in addition to this, it is effective to set distance h2 between ink ejection portion 31 of recording head 3 and recording material P larger than distance h1 between irradiation means 4 and recording material P (h1 < h2), or to make distance d between recording head and irradiation means 4 remote (make d large). Further, it is more preferable to provide bellows structure 7 between recording head 3 and irradiation means 4.

Herein, the wavelength of ultraviolet rays irradiated by irradiation means 4 can be appropriately varied by changing an ultraviolet lamp or a filter which is arranged in irradiation means 4.

Fig. 2 is a drawing to show another example of a constitution of the primary portion of an ink-jet recording apparatus.

An ink-jet recording apparatus shown in Fig. 2 is called a line head type, a plural number of ink-jet recording head 3 of each color are fixing arranged so as to cover the whole width of recording material P. Head carriage 2 is light shielded.

On the other hand, on the down stream of head carriage 2, irradiation means 4 is arranged similarly so as to cover the whole width of recording material P and to cover the whole ink printing region. As an ultraviolet lamp utilized for irradiation means 4, similar one to those described in Fig. 1 can be employed.

In this line head type, head carriage 2 and irradiation means 4 are fixed, and only recording material P is transferred to form an image by ink ejection and curing.

### EXAMPLES

In the following, this invention will be detailed referring to examples, however, is not limited thereto.

### Example 1

### <Preparation of Actinic Ray Curable Composition>

Actinic ray curable compositions were prepared by addition and dissolution of a photo polymerizable compound, a photo-induced acid generator and a basic compound as shown in Tables 1 and 2.

**Table 1**

| Sample No. | Epoxy compound of formula (X) | | Oxetane compound | | Other epoxy compound | | Photo-induced acid generator | | Basic compound | | Remarks |
|---|---|---|---|---|---|---|---|---|---|---|---|
| | Type | *1 | Type | *1 | Type | *1 | Type | *1 | Type | *1 | |
| 1 | X-1 | 50 | OXT-221 | 50 | | | UVI-6992 | 5 | A | 0.5 | Invention |
| 2 | X-3 | 50 | OXT-221 | 50 | | | PI-1 | 5 | A | 0.5 | Invention |
| 3 | X-4 | 50 | OXT-221 | 50 | | | PI-1 | 5 | A | 0.5 | Invention |
| 4 | X-7 | 50 | OXT-221 | 50 | | | PI-1 | 5 | A | 0.5 | Invention |
| 5 | X-8 | 50 | OXT-221 | 50 | | | PI-1 | 5 | A | 0.5 | Invention |
| 6 | X-11 | 50 | OXT-221 | 50 | | | PI-1 | 5 | A | 0.5 | Invention |
| 7 | X-12 | 50 | OXT-221 | 50 | | | PI-2 | 5 | B | 0.5 | Invention |
| 8 | X-3 | 25 | OXT-221 | 50 | X-4 | 25 | SP-152 | 5 | B | 0.5 | Invention |
| 9 | X-7 | 25 | OXT-221 | 50 | X-8 | 25 | PI-1 | 5 | A | 0.5 | Invention |
| 10 | X-7 | 25 | OXT-221 | 50 | X-11 | 25 | PI-1 | 5 | A | 0.5 | Invention |
| 11 | X-7 | 25 | OXT-221 | 50 | X-12 | 25 | PI-1 | 5 | A | 0.5 | Invention |
| 12 | X-8 | 25 | OXT-221 | 50 | X-11 | 25 | PI-1 | 5 | A | 0.5 | Invention |
| 13 | X-11 | 25 | OXT-221 | 50 | X-12 | 25 | PI-2 | 5 | A | 0.5 | Invention |
| 14 | X-1 | 30 | OXT-221 | 60 | Celloxide 2021P | 10 | UVI-6992 | 5 | A | 0.5 | Invention |
| 15 | X-3 | 30 | OXT-221 | 60 | EP-89 | 10 | PI-1 | 5 | B | 0.5 | Invention |
| 16 | X-3 | 50 | OXT-221 | 30 | SEP-155 | 20 | PI-1 | 5 | B | 0.5 | Invention |
| 17 | X-4 | 50 | OXT-212 | 25 | SEP-136 | 25 | PI-1 | 5 | B | 0.5 | Invention |
| 18 | X-7 | 50 | OXT-212 | 25 | SEP-143 | 25 | PI-1 | 5 | B | 0.5 | Invention |
| 19 | X-3 | 50 | OXT-212 | 25 | SEP-150 | 25 | PI-1 | 5 | B | 0.5 | Invention |
| 20 | X-11 | 50 | OXT-212 | 25 | SEP-155 | 25 | PI-1 | 5 | B | 0.5 | Invention |
| 21 | X-12 | 30 | OXT-221 | 40 | SEP-134 | 20 | PI-1 | 5 | A | 0.5 | Invention |
| 22 | X-1 | 30 | OXT-221 | 40 | SEP-142 | 20 | PI-1 | 5 | A | 0.5 | Invention |
| 23 | X-3 | 30 | OXT-221 | 40 | SEP-159 | 20 | PI-1 | 5 | A | 0.5 | Invention |
| 24 | X-4 | 30 | OXT-221 | 40 | SEP-162 | 20 | PI-1 | 5 | B | 0.5 | Invention |
| 25 | X-7 | 30 | OXT-221 | 40 | SEP-167 | 20 | PI-1 | 5 | B | 0.5 | Invention |
| 26 | X-1 | 20 | OXT-221 | 60 | SEP-12 | 20 | UVI-6992 | 5 | A | 0.5 | Invention |
| 27 | X-3 | 20 | OXT-221 | 60 | SEP-36 | 20 | SP-152 | 5 | A | 0.5 | Invention |
| 28 | X-4 | 20 | OXT-221 | 60 | SEP-56 | 20 | PI-1 | 5 | B | 0.5 | Invention |
| 29 | X-7 | 20 | OXT-221 | 60 | SEP-72 | 20 | PI-1 | 5 | B | 0.5 | Invention |
| 30 | X-12 | 20 | OXT-221 | 60 | SEP-89 | 20 | SP-152 | 5 | B | 0.5 | Invention |

| | | | | | | | | | | | |
|---|---|---|---|---|---|---|---|---|---|---|---|
| *1: Addition amount (weight parts) | | | | | | | | | | | |

**Table 2**

| Sample No. | Epoxy compound of formula (X) | | Oxetane compound | | Other epoxy compound | | Photo-induced acid generator | | Basic compound | | Remarks |
|---|---|---|---|---|---|---|---|---|---|---|---|
| | Type | *1 | Type | *1 | Type | *1 | Type | | Type | *1 | |
| 31 | X-21 | 40 | OXT-221 | 60 | | | PI-1 | 5 | B | 0.5 | Inv. |
| 32 | X-22 | 40 | OXT-221 | 60 | | | PI-1 | 5 | A | 0.5 | Inv. |
| 33 | X-24 | 40 | OXT-221 | 60 | | | PI-1 | 5 | A | 0.5 | Inv. |
| 34 | X-26 | 40 | OXT-221 | 60 | | | PI-1 | 5 | B | 0.5 | Inv. |
| 35 | X-27 | 40 | OXT-221 | 60 | | | PI-1 | 5 | B | 0.5 | Inv. |
| 36 | X-50 | 40 | OXT-221 | 60 | | | PI-1 | 5 | B | 0.5 | Inv. |
| 37 | X-21 | 40 | OXT-221/OXT-101 | 35/5 | SEP-150 | 20 | PI-1 | 5 | A | 0.5 | Inv. |
| 38 | X-22 | 40 | OXT-221/OXT-101 | 35/5 | SEP-150 | 20 | PI-1 | 5 | A | 0.5 | Inv. |
| 39 | X-24 | 40 | OXT-221/OXT-101 | 35/5 | SEP-150 | 20 | PI-1 | 5 | B | 0.5 | Inv. |
| 40 | X-26 | 40 | OXT-221/OXT-101 | 35/5 | SEP-152 | 20 | PI-1 | 5 | B | 0.5 | Inv. |
| 41 | X-27 | 40 | OXT-221/OXT-101 | 35/5 | SEP-152 | 20 | PI-1 | 5 | B | 0.5 | Inv. |
| 42 | X-22 | 40 | OXT-221/OXT-101 | 35/5 | SEP-154 | 20 | PI-1 | 5 | B | 0.5 | Inv. |
| 43 | X-21 | 60 | OXT-221/OXT-101 | 10/30 | | | UVI-6992 | 5 | B | 0.5 | Inv. |
| 44 | X-24 | 50 | OXT-221/OXT-101 | 15/35 | | | PI-1 | 5 | A | 0.5 | Inv. |
| 45 | X-21 | 60 | OXT-221/OXT-101 | 20/10 | Epolead GT301 | 10 | UVI-6992 | 5 | A | 0.5 | Inv. |
| 46 | X-24 | 60 | OXT-221/OXT-101 | 20/10 | Epolead GT301 | 10 | SP-152 | 5 | B | 0.5 | Inv. |
| Comp.1 | | | OXT-221 | 70 | Celloxide 2021P | 30 | UVI-6992 | 5 | A | 0.5 | Comp. |
| Comp.2 | | | OXT-221 | 80 | Celloxide 2021P | 20 | UVI-6992 | 5 | A | 0.5 | Comp. |
| Comp.3 | | | OXT-221 | 80 | EP-89 | 20 | UVI-6992 | 5 | A | 0.5 | Comp. |
| Comp.4 | | | OXT-221 | 70 | Vf7010 | 30 | UVI-6992 | 5 | A | 0.5 | Comp. |
| Comp.5 | | | OXT-221 | 50 | Celloxide 3000 | 50 | UVI-6992 | 5 | A | 0.5 | Comp. |
| Comp.6 | | | OXT-221 | 20 | Celloxide 3000 | 80 | UVI-6992 | 5 | A | 0.5 | Comp. |
| Comp.7 | X-1 | 50 | OXT-221/OXT-101 | 5/20 | Epolead GT301 | 25 | UVI-6992 | 5 | A | 0.5 | Comp. |

| | | | | | | | | | | | |
|---|---|---|---|---|---|---|---|---|---|---|---|
| ^{*}1: Addition amount (weight parts), Inv.: Invention, Comp.: Comparison | | | | | | | | | | | |

Compounds utilized are shown below.

### [Photo polymerizable compound]

### <Alicyclic Epoxy Compound>

Celloxide 2021P: produced by Daicel Chemical Industries Ltd.

Celloxide 3000: produced by Daicel Chemical Industries Ltd.

Epolead GT301: produced by Daicel Chemical Industries Ltd.

### <Epoxidated Soybean Oil>

Vf70710: Vikoflex 7010 (produced by Atofina S. A.)

### <Oxetane Compound>

OXT-101: produced by Toagosei Co., Ltd.
OXT-221: produced by Toagosei Co., Ltd.
OXT-212: produced by Toagosei Co., Ltd.

### [Photo-induced Acid Generator]

SP-152: Adekaoptomer SP-152, produced by Asahi Denka Co., Ltd.

UV16992: produced by The Daw Chemicals Co., being a propione carbonate 50% solution

### [Surfactant]

F178k: Megafax F178k, acryl oligomer containing a perfluoroalkyl group (produced by Dainippon Ink & Chemicals, Inc.)
F1405: Megafax 1405, ethyleneoxide adduct containing a perfluoroalkyl group (produced by Dainippon Ink & Chemicals, Inc.)

### [Compatibilizer]

R100: Haritax R100 (Rosin modified maleic acid resin, produced by Harima Chemical Co., Ltd.)
145P: Haritax 145 (Rosin modified maleic acid resin, produced by Harima Chemical Co., Ltd.)

### [Basic Compound]

Basic compound A: N-ethyldiethanol amine
Basic compound B: Triisopropanol amine
Evaluation of Viscosity

Viscosity at 25 °C and at the time of a share rate of 1,000 (1/s) was measured with respect to actinic ray curable compositions before being cured. A composition having a viscosity at 25 °C of not more than 150 mPa·s was ranked A; a composition having a viscosity at 25 °C of 150 - 300 mPa·s was ranked B; a composition having a viscosity at 25 °C of 300 - 500 mPa·s was ranked C; and a composition having a viscosity at 25 °C of not less than 500 mPa·s was ranked D.

### Curing of Actinic Ray Curable Composition

Coated film, after having been produced via the following method, was cured. A prepared actinic ray curable composition, after having been coated onto synthetic paper to a layer thickness of 3 µm, was irradiated by ultraviolet rays at 800 mJ/cm² within 1 second to prepare a cured product.

### Evaluation Method of Cured Product

Physical properties of the prepared cured product were evaluated by the testing methods described below.
1) Pencil Scratch Test: Hardness of each cured product was measured based on JIS K5400.
2) Residual Adhesion Ratio of Cross Cut Method (being Tape Peel-off Test): Adhesive tape, after having been adhered onto a cured product sample prepared by the cross cut of JIS K5400, and pressed twice at right angles by a 2 Kg roller, was peeled off quickly, whereby the remaining adhered samples were examined.
3) Bending Resistance Evaluation: An actinic ray curable composition, after having been coated on synthetic paper (Synthetic Paper Upo FGS, produced by Upo Corp.) to make a layer thickness of 3 µm, was irradiated with ultraviolet rays at 800 mJ/cm² by use of a metal halide lamp within 1 second, whereby a cured product was prepared. A cured product prepared was evaluated based on the method of bending resistance evaluation of JIS K5600.
4) Storage Stability Evaluation of Cured Film: A cured composition prepared above was kept under a room fluorescent light for 1 year at room temperature, and tinting of the cured composition was visually evaluated. One with little tinting was ranked A; one with slight tinting was ranked B; and one with tinting was ranked C. A is a level having no problem in practical use.

The results will be shown in Table 3.

**Table 3**

| Sample No. | Pencil scratch value Pencil hardness | Adhesion remaining tance remaining ratio (%) | Bending resistance φ: mm | Storage stability of cured film | Viscosity | Remarks |
|---|---|---|---|---|---|---|
| 1 | 3H | 85 | 3 mm φ | A | A | Inv. |
| 2 | 3H | 85 | *1 | A | A | Inv. |
| 3 | 3H | 85 | *1 | A | A | Inv. |
| 4 | 2H | 80 | *1 | A | A | Inv. |
| 5 | 2H | 80 | *1 | A | A | Inv. |
| 6 | 2H | 80 | *1 | A | A | Inv. |
| 7 | 2H | 80 | *1 | A | A | Inv. |
| 8 | 3H | 85 | *1 | A | A | Inv. |
| 9 | 2H | 80 | *1 | A | A | Inv. |
| 10 | 2H | 80 | *1 | A | A | Inv. |
| 11 | 2H | 80 | *1 | A | A | Inv. |
| 12 | 2H | 80 | *1 | A | A | Inv. |
| 13 | 2H | 80 | *1 | A | A | Inv. |
| 14 | 2H | 75 | 2 mm φ | A | A | Inv. |
| 15 | 3H | 85 | *1 | A | A | Inv. |
| 16 | 3H | 85 | *1 | A | A | Inv. |
| 17 | 2H | 80 | *1 | A | A | Inv. |
| 18 | 2H | 80 | *1 | A | A | Inv. |
| 19 | 2H | 80 | *1 | A | A | Inv. |
| 20 | 3H | 85 | *1 | A | A | Inv. |
| 21 | 3H | 80 | *1 | A | A | Inv. |
| 22 | 3H | 80 | *1 | A | A | Inv. |
| 23 | 2H | 80 | *1 | A | A | Inv. |
| 24 | 3H | 85 | *1 | A | A | Inv. |
| 25 | 3H | 85 | *1 | A | A | Inv. |
| 26 | 2H | 75 | *1 | A | A | Inv. |
| 27 | 2H | 75 | *1 | A | A | Inv. |
| 28 | 3H | 80 | *1 | A | A | Inv. |
| 29 | 3H | 80 | *1 | A | A | Inv. |
| 30 | 2H | 80 | *1 | A | A | Inv. |
| 31 | 3H | 85 | *1 | A | A | Inv. |
| 32 | 3H | 85 | *1 | A | A | Inv. |
| 33 | 3H | 85 | *1 | A | A | Inv. |
| 34 | 3H | 85 | *1 | A | A | Inv. |
| 35 | 3H | 85 | *1 | A | A | Inv. |
| 36 | 3H | 85 | *1 | A | A | Inv. |
| 37 | 3H | 90 | *1 | A | A | Inv. |
| 38 | 3H | 90 | *1 | A | A | Inv. |
| 39 | 3H | 90 | *1 | A | A | Inv. |
| 40 | 3H | 90 | *1 | A | A | Inv. |
| 41 | 3H | 90 | *1 | A | A | Inv. |
| 42 | 3H | 90 | *1 | A | A | Inv. |
| 43 | 3H | 80 | *1 | A | B | Inv. |
| 44 | 3H | 85 | *1 | A | B | Inv. |
| 45 | 3H | 75 | *1 | A | C | Inv. |
| 46 | 3H | 80 | *1 | A | C | Inv. |
| Comp.1 | 2H | 85 | *2 | C | A | Comp. |
| Comp.2 | 3H | 85 | *2 | C | A | Comp. |
| Comp.3 | 3H | 85 | *2 | B | A | Comp. |
| Comp.4 | HB | 40 | 3 mm φ | C | A | Comp. |
| Comp.5 | 3H | 80 | *2 | C | A | Comp. |
| Comp.6 | 2H | 80 | *2 | C | A | Comp. |
| Comp.7 | H | 75 | 8 mm φ | A | D | Comp. |

| | | | | | | |
|---|---|---|---|---|---|---|
| *1: No cracking generated even at 1 mm φ, Inv.: Invention *2: Cracking generated even at 10 mm φ, Comp.: Comparison | | | | | | |

It is clear from Table 3 that samples of this invention exhibit little deterioration of film hardness, improved adhesion and flexibility, and excellent storage stability.

### Example 2

### <Preparation of Actinic Ray Curable Ink>

A dispersant (PB822, manufactured by Ajinomoto Finetechno Co., Ltd.) of 5 weight parts and each photo polymerizable compound described in Tables 4 and 5 were charged in a stainless beaker, the mixture being dissolved by stirring and mixing while being heated on a hot plate of 65 °C, and successively, the resulting solution, after having been added with each types of pigment of 3 weight parts, was pored into a polyethylene bottle together with zirconia beads having a diameter of 1 mm to be sealed, followed by being subjected to a dispersion treatment for 2 hours by use of a paint shaker. Next, zirconia beads were eliminated, each of various additives such as a photo-induced acid generator, a basic compound and a surfactant being added as a combination described in Tables 4 and 5, and the resulting mixture was filtered by a 0.8 µm membrane filter to prevent printer clogging, whereby ink composition sets were prepared.

**Table 4**

| Sample No. | Epoxy compound of formula (X) | | Oxetane compound | | Other epoxy compound | | Photo-induced acid generator | | Basic compound Addition amount of 0.5 weight parts | Surfactant Addition amount of 0.5 weight parts | Compatibilizer Addition amount of 0.5 weight parts | Remarks |
|---|---|---|---|---|---|---|---|---|---|---|---|---|
| | Type | *1 | Type | *1 | Type | *1 | Type | *1 | Type | Type | Type | |
| 1 | X-1 | 50 | OXT-221 | 50 | | | UVI-6992 | 5 | A | F1405 | 145P | Inv. |
| 2 | X-3 | 50 | OXT-221 | 50 | | | PI-1 | 5 | A | F1405 | 145P | Inv. |
| 3 | X-4 | 50 | OXT-221 | 50 | | | PI-1 | 5 | A | F1405 | 145P | Inv. |
| 4 | X-7 | 50 | OXT-221 | 50 | | | PI-1 | 5 | A | F1405 | 145P | Inv. |
| 5 | X-8 | 50 | OXT-221 | 50 | | | PI-1 | 5 | A | F1405 | 145P | Inv. |
| 6 | X-11 | 50 | OXT-221 | 50 | | | PI-1 | 5 | A | F1405 | 145P | Inv. |
| 7 | X-12 | 50 | OXT-221 | 50 | | | PI-2 | 5 | B | F1405 | 145P | Inv. |
| 8 | X-3 | 25 | OXT-221 | 50 | X-4 | 25 | SP-152 | 5 | B | F1405 | 145P | Inv. |
| 9 | X-7 | 25 | OXT-221 | 50 | X-8 | 25 | PI-1 | 5 | A | F1405 | 145P | Inv. |
| 10 | X-7 | 25 | OXT-221 | 50 | X-11 | 25 | PI-1 | 5 | A | F1405 | 145P | Inv. |
| 11 | X-7 | 25 | OXT-221 | 50 | X-12 | 25 | PI-1 | 5 | A | F1405 | 145P | Inv. |
| 12 | X-8 | 25 | OXT-221 | 50 | X-11 | 25 | PI-1 | 5 | A | F1405 | 145P | Inv. |
| 13 | X-11 | 25 | OXT-221 | 50 | X-12 | 25 | PI-2 | 5 | A | F178k | R100 | Inv. |
| 14 | X-1 | 30 | OXT-221 | 60 | Celloxide 2021P | 10 | UVI-6992 | 5 | A | F178k | R100 | Inv. |
| 15 | X-3 | 30 | OXT-221 | 60 | EP-89 | 10 | PI-1 | 5 | B | F178k | R100 | Inv. |
| 16 | X-3 | 50 | OXT-221 | 30 | SEP-155 | 20 | PI-1 | 5 | B | F178k | R100 | Inv. |
| 17 | X-4 | 50 | OXT-212 | 25 | SEP-136 | 25 | PI-1 | 5 | B | F178k | R100 | Inv. |
| 18 | X-7 | 50 | OXT-212 | 25 | SEP-143 | 25 | PI-1 | 5 | B | F178k | R100 | Inv. |
| 19 | X-3 | 50 | OXT-212 | 25 | SEP-150 | 25 | PI-1 | 5 | B | F178k | R100 | Inv. |
| 20 | X-11 | 50 | OXT-212 | 25 | SEP-155 | 25 | PI-1 | 5 | B | F178k | R100 | Inv. |
| 21 | X-12 | 30 | OXT-221 | 40 | SEP-134 | 20 | PI-1 | 5 | A | F1405 | 145P | Inv. |
| 22 | X-1 | 30 | OXT-221 | 40 | SEP-142 | 20 | PI-1 | 5 | A | F1405 | 145P | Inv. |
| 23 | X-3 | 30 | OXT-221 | 40 | SEP-159 | 20 | PI-1 | 5 | A | F1405 | 145P | Inv. |
| 24 | X-4 | 30 | OXT-221 | 40 | SEP-162 | 20 | PI-1 | 5 | B | F1405 | 145P | Inv. |
| 25 | X-7 | 30 | OXT-221 | 40 | SEP-167 | 20 | PI-1 | 5 | B | F1405 | 145P | Inv. |
| 26 | X-1 | 20 | OXT-221 | 60 | SEP-12 | 20 | UVI-6992 | 5 | A | F178k | R100 | Inv. |
| 27 | X-3 | 20 | OXT-221 | 60 | SEP-36 | 20 | SP-152 | 5 | A | F178k | R100 | Inv. |

| | | | | | | | | | | | | |
|---|---|---|---|---|---|---|---|---|---|---|---|---|
| *1: Addition amount (weight parts), Inv.: Invention | | | | | | | | | | | | |

**Table 5**

| Sample No. | Epoxy compound of formula (X) | | Oxetane compound | | Other epoxy compound | | Photo-induced acid generator | | Basic compound Addition amount of 0.5 weight parts | Surfactant Addition amount of 0.5 weight parts | Compatibilizer Addition amount of 0.5 weight parts | Remarks |
|---|---|---|---|---|---|---|---|---|---|---|---|---|
| | Type | *1 | Type | *1 | Type | *1 | Type | *1 | Type | Type | Type | |
| 28 | X-4 | 20 | OXT-221 | 60 | SEP-56 | 20 | PI-1 | 5 | B | F178k | R100 | Inv. |
| 29 | X-7 | 20 | OXT-221 | 60 | SEP-72 | 20 | PI-1 | 5 | B | F178k | R100 | Inv. |
| 30 | X-12 | 20 | OXT-221 | 60 | SEP-89 | 20 | SP-152 | 5 | B | F178k | R100 | Inv. |
| 31 | X-21 | 40 | OXT-221 | 60 | | | PI-1 | 5 | B | F178k | R100 | Inv. |
| 32 | X-22 | 40 | OXT-221 | 60 | | | PI-1 | 5 | A | F178k | R100 | Inv. |
| 33 | X-24 | 40 | OXT-221 | 60 | | | PI-1 | 5 | A | F178k | R100 | Inv. |
| 34 | X-26 | 40 | OXT-221 | 60 | | | PI-1 | 5 | B | F178k | R100 | Inv. |
| 35 | X-27 | 40 | OXT-221 | 60 | | | PI-1 | 5 | B | F178k | R100 | Inv. |
| 36 | X-50 | 40 | OXT-221 | 60 | | | PI-1 | 5 | B | F178k | R100 | Inv. |
| 37 | X-21 | 40 | OXT-221/OXT-101 | 35/5 | SEP-150 | 20 | PI-1 | 5 | A | F178k | R100 | Inv. |
| 38 | X-22 | 40 | OXT-221/OXT-101 | 35/5 | SEP-150 | 20 | PI-1 | 5 | A | F178k | R100 | Inv. |
| 39 | X-24 | 40 | OXT-221/OXT-101 | 35/5 | SEP-150 | 20 | PI-1 | 5 | B | F178k | R100 | Inv. |
| 40 | X-26 | 40 | OXT-221/OXT-101 | 35/5 | SEP-152 | 20 | PI-1 | 5 | B | F178k | R100 | Inv. |
| 41 | X-27 | 40 | OXT-221/OXT-101 | 35/5 | SEP-152 | 20 | PI-1 | 5 | B | F178k | R100 | Inv. |
| 42 | X-22 | 40 | OXT-221/OXT-101 | 35/5 | SEP-154 | 20 | PI-1 | 5 | B | F178k | R100 | Inv. |
| Comp.1 | | | OXT-221 | 70 | Celloxide 2021P | 30 | UVI-6992 | 5 | A | F178k | R100 | Comp. |
| Comp.2 | | | OXT-221 | 80 | Celloxide 2021P | 20 | UVI-6992 | 5 | A | F178k | R100 | Comp. |
| Comp.3 | | | OXT-221 | 80 | EP-89 | 20 | UVI-6992 | 5 | A | F178k | R100 | Comp. |
| Comp.4 | | | OXT-221 | 70 | Vf7010 | 30 | UVI-6992 | 5 | A | F178k | R100 | Comp. |
| Comp.5 | | | OXT-221 | 50 | Celloxide 3000 | 50 | UVI-6992 | 5 | A | F178k | R100 | Comp. |
| Comp.6 | | | OXT-221 | 20 | Celloxide 3000 | 80 | UVI-6992 | 5 | A | F178k | R100 | Comp. |
| Comp.7 | X-1 | 50 | OXT-221/OXT-101 | 20/5 | Epolead GT301 | 25 | UVI-6992 | 5 | A | F178k | R100 | Comp. |

| | | | | | | | | | | | | |
|---|---|---|---|---|---|---|---|---|---|---|---|---|
| *1: Addition amount (weight parts), Inv.: Invention, Comp.: Comparison | | | | | | | | | | | | |

Utilized pigment is as follows.
K: C. I. Pigment Black 7
C: C. I. Pigment Blue 15:3
M: C. I. Pigment Red 57:1
Y: C. I. Pigment Yellow 13
W: Titanium oxide (anatase type: particle size of 0.2 µm)
Lk: C. I. Pigment Black 7
Lc: C. I. Pigment Blue 15:3
Lm: C. I. Pigment Red 57:1
Ly: C. I. Pigment Yellow 13

Utilized compounds will be shown below.

### [Surfactant]

F178k: Megafax F178k, acryl oligomer containing a perfluoroalkyl group (manufactured by Dainippon Ink & Chemicals, Inc.)
F1405: Megafax 1405, ethyleneoxide adduct containing a perfluoroalkyl group (manufactured by Dainippon Ink & Chemicals, Inc.)

### [Compatibilizer]

R100: Haritax R100 (Rosin modified maleic acid resin, manufactured by Harima Chemical Co., Ltd.)
145P: Haritax 145 (Rosin modified maleic acid resin, manufactured by Harima Chemical Co., Ltd.)

### <Ink-jet Image Forming Method>

On an ink-jet recording apparatus having a constitution described in fig. 1 which is equipped with a piezo type ink-jet nozzle, each actinic ray curable ink prepared above was mounted and the following image recording was continuously performed on each long length recording material having a width of 600 mm and a length of 20 m. The ink supply system was constituted of an ink tank, a supply tube, a pre-room ink tank immediately before the head, a piping with a filter and a piezo head, and a portion from the pre-room tank to the head was heat-insulated and heated at 50 °C. Herein, the head portion was heated corresponding to a viscosity of each actinic curable ink and driven so as to eject a multi-size dot of a liquid drop quantity of 2 - 15 pl at a resolution of 720 x 720 dpi (dpi is a dot number per 1 inch, that is, per 2.54 cm), whereby the above-described actinic ray curable ink was continuously ejected. Further, a recording material was heated at 50 °C by a plane heater. Ink-jet ink, after having landed, was cured in a moment (less than 0.5 seconds after landing) by irradiation light source A: high pressure mercury lamp VZero 085 (produced by Integration Technology Corp.) or irradiation light source B: metal halide lamp MAL400NL (produced by Nippon Denchi Co., Ltd., electric power = 3 kW·hr, 120 W/cm) on the both sides of a carriage. The total ink layer thickness after image formation was measured to be in a range of 2.3 - 13 µm. Herein, in ink-jet image formation, printing was performed according to the above-described method under environments of 30 °C, 80% RH and 25 °C, 20% RH.

Herein, illuminance of each irradiation light source was measured by use of UVPF-A1 manufactured by Iwasaki Electric Co., Ltd. as accumulated illuminance at 254 nm.

Further, the detail of an abbreviation of each recording material described above was as follows.

Synthetic Paper: synthetic paper UPO FGS, produced by Upo Corp.
PVC: polyvinyl chloride
Evaluation of Viscosity
Ink having a viscosity at 25 °C in a range of 7 - 40 mPa·s was ranked A, and ink having a viscosity beyond this range was ranked C.

### Evaluation of Ejection Behavior

After ink had been continuously ejected for 30 minutes, the state of ink non-ejection was visually evaluated.
A: No missed ejection was observed, which was noted as a good level.
B: Slight missed ejection was observed, which was within acceptable levels.
C: Some missed ejection was observed, which was at a minimally acceptable level of image quality.
D: Numerous missed ejection was observed, which was at an unviable commercial level.

### Evaluation of Ink-jet Recorded Image

With respect to each image recorded by the above described image forming method, the following evaluations were performed. MS-Mincho style 6 point characters were printed at a specific density, and roughness of the printed character was evaluated at magnification with a loupe, whereby character quality was evaluated based on the following criteria.
A: No character edge roughness was noted.
B: Slight character edge roughness was apparent.
C: Some edge roughness was observed, however, printed characters could be easily recognized, which was rated a barely usable level.
D: Pronounced edge roughness was observed and characters appeared scratchy, which was rated at an unviable level.

Further, printing was performed so that individual dots of colors Y, M, C and K were adjacent to each other, and each color dot was observed through a loupe to visually observe any state of bleeding and wrinkles, whereby color mixing (being bleeding) was evaluated based on the following criteria.
A: The shape of adjacent dots each other showed a true circle of no bleeding.
B: The shape of adjacent dots exhibited an almost true circle, showing minimal bleeding.
C: Adjacent dots showed slight bleeding and the shape was slightly deformed, which was rated at a barely viable level.
D: Adjacent dots showed obvious bleeding and were mixed with each other, and wrinkles were generated at the superposed portion, which was rated at an unviable level.

### Evaluations of Hardness and Bending Resistance of Cured Film

Physical properties of cured film which was prepared by printing via the above ink-jet image forming method, by use of cyan ink in which cyan pigment had been dispersed, were evaluated according to the following test methods.
1) Pencil scratch test: Hardness of each cured film sample was measured based on JIS K5400.
2) Evaluation of bending resistance: After a curable composition was ejection coated on synthetic paper (synthetic paper Upo FGS, produced by Upo Corp.) to make a layer thickness of 30 µm, the coated film was irradiated with ultraviolet rays of 800 mJ/cm² by a metal halide lamp within 1 second to prepare a cured film. The prepared cured film was evaluated based on a method of bending resistance evaluation of JIS K5600.

### Evaluation of Ink Storage Stability

After prepared ink was sealed in a vessel to be stored in the dark at room temperature for 1 month, image formation and tests similar to the above methods were performed to judge ink storage stability. Samples with no significant variation of the test results, even after 1 month of aging, were ranked as A; samples showing significant variation in any one of the tests were ranked as B; and samples in which image formation was not possible due to viscosity increase were ranked as C. Ink rated at "A" is a level of storage stability exhibiting no problems in practical use.

The results are shown in following Tables 6 and 7. Herein, comparative sample 7 of Table 5 could not be evaluated because of ejection failure.

**Table 6**

| Sample No. * | 30 °C-80%RH | | | | 25 °C-20%RH | | | | Cured film hardness | Bending resistance φ: mm | Ink ejection behavior | Ink storage stability | Ink viscosity | Remarks |
|---|---|---|---|---|---|---|---|---|---|---|---|---|---|---|
| | PVC | | Upo FGS | | PVC | | Upo FGS | | | | | | | |
| | * | Bleed-ing | * | Bleed-ing | * | Bleeding | * | Bleeding | | | | | | |
| 1 | A | B | A | B | A | B | A | B | 3H | 3 mm φ | A | A | A | Inv. |
| 2 | A | B | A | B | A | B | A | B | 2H | 2 mm φ | A | A | A | Inv. |
| 3 | A | B | A | B | A | B | A | B | 2H | 1 mm φ | A | A | A | Inv. |
| 4 | A | B | B | B | A | B | A | B | 2H | *1 | A | A | A | Inv. |
| 5 | A | B | B | B | A | B | A | B | 2H | *1 | A | A | A | Inv. |
| 6 | A | B | B | B | A | B | A | B | 2H | *1 | A | A | A | Inv. |
| 7 | A | B | A | B | A | B | A | B | 2H | 3 mm φ | A | A | A | Inv. |
| 8 | A | B | A | B | A | B | A | B | 2H | 2 mm φ | A | A | A | Inv. |
| 9 | A | B | B | B | A | B | A | B | 2H | 1 mm φ | A | A | A | Inv. |
| 10 | A | B | B | B | A | B | A | B | 2H | *1 | A | A | A | Inv. |
| 11 | A | B | B | B | A | B | A | B | 2H | *1 | A | A | A | Inv. |
| 12 | A | B | B | B | A | B | A | B | 2H | *1 | A | A | A | Inv. |
| 13 | A | A | A | B | A | A | A | A | 3H | 1 mm φ | B | A | A | Inv. |
| 14 | A | A | A | B | A | A | A | A | 3H | 1 mm φ | B | A | A | Inv. |
| 19 | A | A | A | B | A | A | A | A | 3H | 1 mm φ | B | A | A | Inv. |
| 16 | A | A | A | B | A | A | A | A | 3H | 1 mm φ | B | A | A | Inv. |
| 17 | B | B | B | B | A | B | A | B | 2H | *1 | A | A | A | Inv. |
| 18 | B | B | B | B | A | B | A | B | 2H | *1 | A | A | A | Inv. |
| 19 | B | B | B | B | A | B | A | B | 2H | *1 | A | A | A | Inv. |
| 20 | B | B | B | B | A | B | A | B | 2H | *1 | A | A | A | Inv. |
| 21 | A | B | B | B | A | B | A | B | 2H | *1 | A | A | A | Inv. |
| 22 | A | B | B | B | A | B | A | B | 2H | *1 | A | A | A | Inv. |
| 23 | A | B | B | B | A | B | A | B | 2H | *1 | A | A | A | Inv. |
| 24 | A | B | B | B | A | B | A | B | 2H | *1 | A | A | A | Inv. |
| 25 | A | A | A | B | A | A | A | B | 2H | *1 | A | A | A | Inv. |
| 26 | A | A | A | B | A | A | A | A | 3H | *1 | A | A | A | Inv. |
| 27 | A | A | A | B | A | A | A | A | 3H | *1 | B | A | A | Inv. |
| 28 | A | A | A | B | A | A | A | A | 3H | *1 | B | A | A | Inv. |
| 29 | A | A | A | B | A | A | A | A | 3H | *1 | B | A | A | Inv. |
| 30 | A | A | A | A | A | A | A | A | 3H | *1 | B | A | A | Inv. |

| | | | | | | | | | | | | | | |
|---|---|---|---|---|---|---|---|---|---|---|---|---|---|---|
| Inv.: Invention, *1: No cracking generation even at 1 mm φ, *: Character | | | | | | | | | | | | | | |

**Table 7**

| Sample No. | 30 °C·80%RH | | | | 25 °C·20%RH | | | | Cured film hardness | Bending resistance φ:mm | Ink behavior | Ink storage stability | Ink Viscosity | Remarks |
|---|---|---|---|---|---|---|---|---|---|---|---|---|---|---|
| | PVC | | Upo FGS | | PVC | | Upo FGS | | | | | | | |
| | * | Bleeding | * | Bleeding | * | Bleed- * ing | * | Bleeding | | | | | | |
| 31 | A | A | A | A | A | A | A | A | 3H | *1 | A | A | A | Inv. |
| 32 | A | A | A | A | A | A | A | A | 3H | *1 | A | A | A | Inv. |
| 33 | A | A | A | A | A | A | A | A | 3H | *1 | A | A | A | Inv. |
| 34 | A | A | A | A | A | A | A | A | 3H | *1 | A | A | A | Inv. |
| 35 | A | A | A | A | A | A | A | A | 3H | *1 | A | A | A | Inv. |
| 36 | A | A | A | A | A | A | A | A | 3H | *1 | A | A | A | Inv. |
| 37 | A | A | A | A | A | A | A | A | 3H | *1 | A | A | A | Inv. |
| 38 | A | A | A | A | A | A | A | A | 3H | *1 | A | A | A | Inv. |
| 39 | A | A | A | A | A | A | A | A | 3H | *1 | A | A | A | Inv. |
| 40 | A | A | A | A | A | A | A | A | 3H | *1 | A | A | A | Inv. |
| 41 | A | A | A | A | A | A | A | A | 3H | *1 | A | A | A | Inv. |
| 42 | A | A | A | A | A | A | A | A | 3H | *1 | A | A | A | Inv. |
| Comp.1 | A | B | A | B | A | B | A | B | 3H | *2 | B | B | A | Comp. |
| Comp.2 | A | B | A | B | A | B | A | B | 2H | 8 mm φ | B | B | A | Comp. |
| Comp.3 | A | B | A | B | A | B | A | B | 2H | *2 | C | B | A | Comp. |
| Comp.4 | D | D | D | D | C | C | C | C | HB | 3 mm φ | B | B | A | Comp. |
| Comp.5 | B | B | B | B | A | B | A | B | 3H | 8 mm φ | A | B | A | Comp. |
| Comp.6 | B | B | B | B | A | B | A | B | 2H | *2 | A | B | A | Comp. |

| | | | | | | | | | | | | | | |
|---|---|---|---|---|---|---|---|---|---|---|---|---|---|---|
| Inv.: Invention, *1: No cracking generation even at 1 mm φ, *: Character Comp.: Comparison, *2: Cracking generation even at 10 mmφ | | | | | | | | | | | | | | |

It is clear from the results of Tables 6 and 7 that samples of this invention exhibit excellent ejection behavior, improved image quality and excellent storage stability. It is clear that samples of this invention exhibit superior curing capability regardless of environmental variation.

## Claims

1. An actinic ray curable composition comprising at least one type of epoxy compound represented Formula (X) and at least one type of oxetane compound as a photo polymerizable compound, and the actinic ray curable composition having a viscosity at 25 °C of 1 - 500 mPa·s: 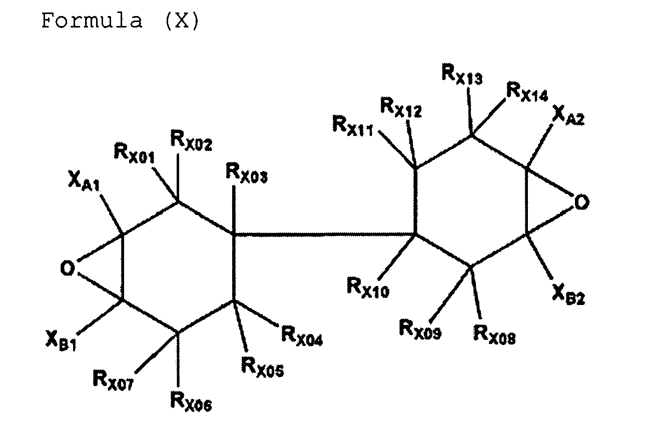 wherein X_{A1}, X_{A2}, X_{B1} and X_{B2} are each a hydrogen atom or an alkyl group, and R_{X01} - R_{X14} are each a hydrogen atom or a substituent.

2. The actinic ray curable composition described in claim 1,
wherein, in the epoxy compound represented by Formula (X), at least one of X_{A1} or X_{A2} is an alkyl group and at least one of X_{B1} or X_{B2} is an alkyl group.

3. The actinic ray curable composition described in claim 1 or 2,
wherein the oxetane compound has no substituent at the 2-position of an oxetane ring.

4. The actinic ray curable composition described in any one of claims 1 - 3,
wherein at least one type of epoxy compound represented by Formula (A) is further incorporated as a photo polymerizable compound: 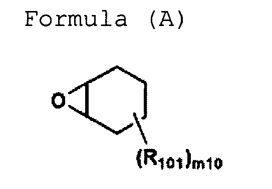 wherein R₁₀₁ is a substituent containing no reactive functional group which is cationic polymerizable or radical polymerizable, and m10 is 1, 2, 3 or 4.

5. The actinic ray curable composition described in claim 4,
wherein the epoxy compound represented by Formula (A) is a compound represented by Formula (A-I): 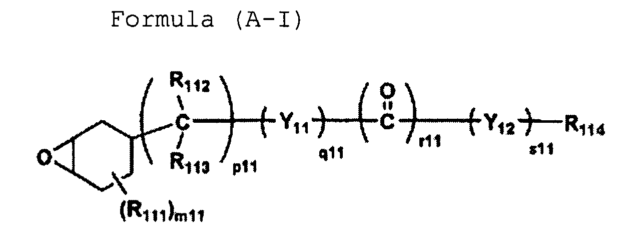 wherein R₁₁₁ is a substituent; m11 is 0, 1, 2 or 3; R₁₁₂, R₁₁₃ and R₁₁₄ are each independently a hydrogen atom or a substituted or unsubstituted alkyl group; Y₁₁ and Y₁₂ are each independently O or S; p11 is 0, 1 or 2; q11 is 0 or 1; r11 is 0 or 1; and s11 is 0 or 1.

6. The actinic ray curable composition described in claim 4,
wherein the epoxy compound represented by Formula (A) is a compound represented by Formula (A-II): 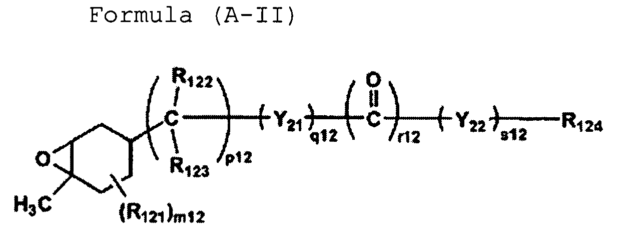 wherein R₁₂₁ is a substituent; m12 is 0, 1 or 2; R₁₂₂, R₁₂₃ and R₁₂₄ are each independently a hydrogen atom or a substituted or unsubstituted alkyl group; Y₂₁ and Y₂₂ are each independently O or S; p12 is 0, 1 or 2; q12 is 0 or 1; r12 is 0 or 1; and s12 is 0 or 1.

7. The actinic ray curable composition described in claim 4,
wherein the epoxy compound represented by Formula (A) is a compound represented by Formula (A-III), (A-IV) or (A-V) : 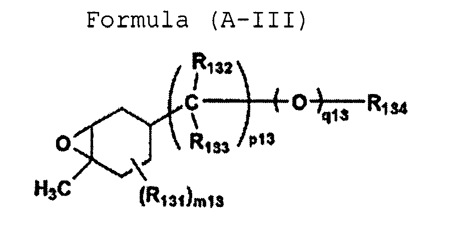 wherein R₁₃₁ is a substituent; m13 is 0, 1 or 2; R₁₃₂, R₁₃₃ and R₁₃₄ are each independently a hydrogen atom or a substituted or unsubstituted alkyl group; p13 is 0, 1 or 2; and q13 is 0 or 1. 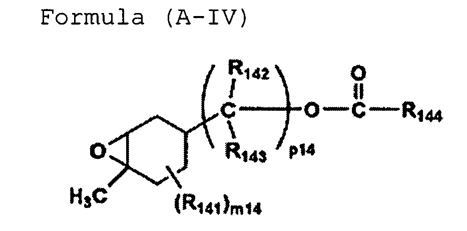 wherein R₁₄₁ is a substituent; m14 is 0, 1 or 2; R₁₄₂, R₁₄₃ and R₁₄₄ are each independently a hydrogen atom or a substituted or unsubstituted alkyl group; and p14 is 0, 1 or 2. 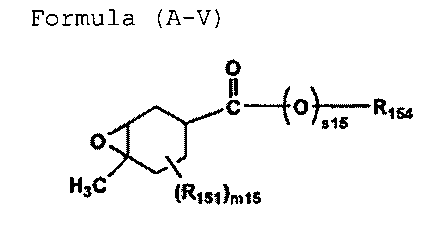 wherein R₁₅₁ is a substituent; m15 is 0, 1 or 2; R₁₅₄ is a hydrogen atom or a substituted or unsubstituted alkyl group; and s15 is 0 or 1.

8. The actinic ray curable composition described in claim 4,
wherein the epoxy compound represented by Formula (A) is a compound represented by Formula (A-VI). 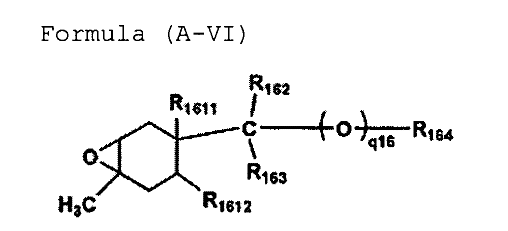 wherein R₁₆₁₁ and R₁₆₁₂ are each independently a hydrogen atom or an alkyl group having a carbon number of 1 - 6, R₁₆₂, R₁₅₃ and R₁₆₄ are each independently a hydrogen atom or a substituted or unsubstituted alkyl group, and q16 is 0 or 1.

9. The actinic ray curable composition described in any one of claims 3 - 8,
wherein the oxetane compound having no substituent at the 2-position of an oxetane ring is a poly-functional oxetane compound having at least two oxetane rings.

10. The actinic ray curable composition described in any one of claims 1 - 9,
wherein the actinic ray curable composition contains a compound which generates acid by actinic ray irradiation.

11. The actinic ray curable composition described in claim 10,
wherein the compound which generates acid by actinic ray irradiation is an onium compound.

12. The actinic ray curable composition described in claim 11,
wherein the onium compound is a sulfonium compound.

13. The actinic ray curable composition described in claim 12,
wherein the onium compound is a compound represented by Formula (I-1), (1-2) or (1-3): 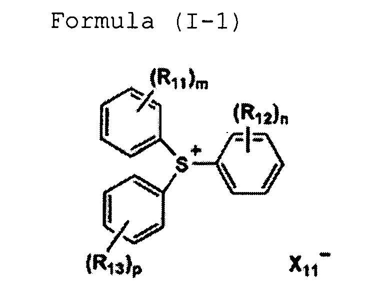 wherein R₁₁, R₁₂ and R₁₃ are a substituent; m, n and p are an integer of 0 - 2; and X₁₁⁻ is a counter ion. 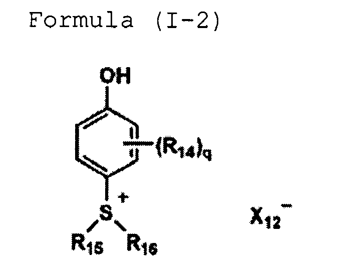 wherein R₁₄ is a substituent; q is an integer of 0 - 2; R₁₅ and R₁₆ are a substituted or unsubstituted alkyl group, a substituted or unsubstituted alkenyl group, a substituted or unsubstituted alkynyl group, or a substituted or unsubstituted aryl group; and X₁₂⁻ is a counter ion. 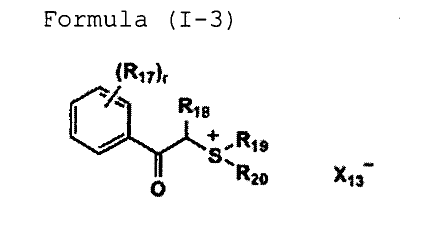 wherein R₁₇ is a substituent, r is an integer of 0 - 3, R₁₉ and R₂₀ are a substituted or unsubstituted alkyl group, a substituted or unsubstituted alkenyl group, a substituted or unsubstituted alkynyl group, or a substituted or unsubstituted aryl group; and X₁₃- is a counter ion.

14. An actinic ray curable ink containing an actinic ray curable composition described in any one of claims 1 - 13.

15. The actinic ray curable ink described in claim 14 exhibits viscosity at 25 °C of 7 - 40 mPa·s.

16. The actinic ray curable ink described in claim 14 or 15 contains a pigment.

17. An image forming method comprising the steps of:
(a) ejecting the actinic ray curable ink described in any one of claims 14 - 16 from an ink-jet recording head onto a recording material, and
(b) curing the ink to form an image,
wherein the actinic ray curable ink is cured by irradiation of actinic rays within 0.001 - 1.0 second after ink landing.

18. The image forming method described in claim 17,
wherein a minimum ink liquid quantity ejected from each nozzle of the ink-jet recording head is 2 - 15 pl.

19. An ink-jet recording apparatus which is utilized for the image forming method described in claim 17 or 18,
wherein ink ejection is performed after an actinic ray curable ink and a recording head are heated to 35 - 100 °C.

20. An ink-jet recording apparatus which is utilized for the image forming method described in claim 17 or 18,
wherein ink ejection is performed onto a recording material being heated at 35 - 60 °C.

21. An epoxy compound being represented by following Formula (X-a): 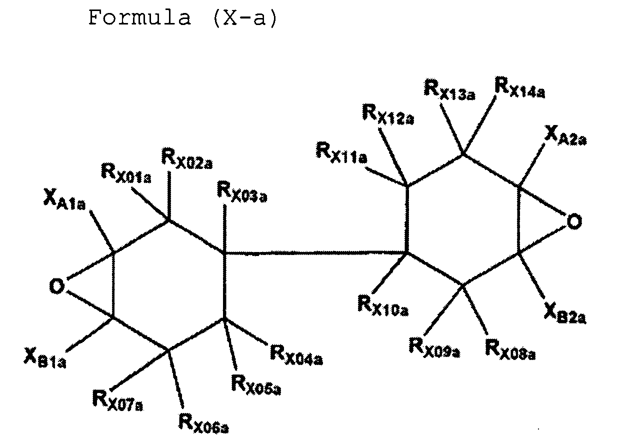 wherein X_{A1a}, X_{A2a}, X_{B1a}, and X_{B2a} are each a hydrogen atom or an alkyl group; R_{X01a}- R_{X14a} are each a hydrogen atom or a substituent; at least one of X_{A1a} or X_{B1a} is an alkyl group; and at least one of X_{A2a} and X_{B2a} is an alkyl group.

22. An epoxy compound being represented by Formula (X-b): 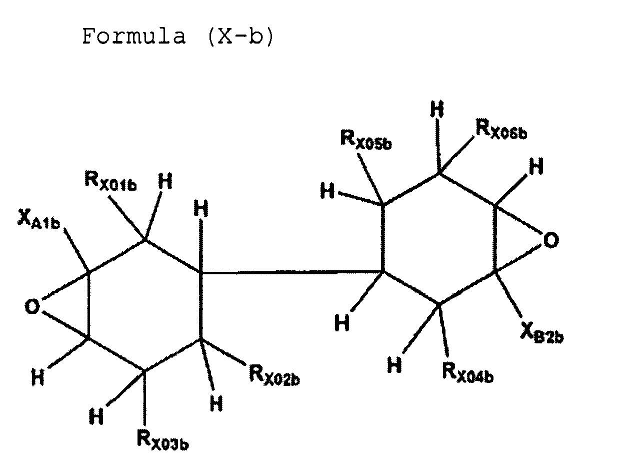 wherein X_{A1b} and X_{B2b} are each an alkyl group, and R_{X01b} - R_{x06b} are each a hydrogen atom or an alkyl group.
